# EUROPEAN PATENT APPLICATION

(11) **EP 3 357 924 A1**
(43) Date of publication of application: **08.08.2018**
(21) Application number: 17305129.3
(22) Date of filing: 06.02.2017
(51) Int. Cl.: C07D 471/08, A61P 31/04, A61K 31/551, A61K 31/529

(54) **NOVEL HETEROCYCLIC COMPOUNDS AND THEIR USE IN PREVENTING OR TREATING BACTERIAL INFECTIONS**

(71) Applicant: Mutabilis, 75007 Paris (FR)
(72) Inventor: BONNARD, Damien, 75019 PARIS (FR); BRIAS, Julie, 75013 PARIS (FR); BARBION, Julien, 95110 SANNOIS (FR); CARAVANO, Audrey, 95880 ENGHIEN LES BAINS (FR); CHASSET, Sophie, 77176 NANDY (FR); CHEVREUIL, Francis, 60500 CHANTILLY (FR); LECOINTE, Nicolas, 75018 PARIS (FR); LEDOUSSAL, Benoît, 22450 POMMERIT JAUDY (FR); LE ROUZIC, Erwann, 75011 PARIS (FR); LE STRAT, Frédéric, 77380 COMBS LA VILLE (FR); MOREAU, François, 91400 ORSAY (FR); QUERNIN, Marie-Hélène, 59700 MARCQ EN BAROEUL (FR); WAECKEL, Ludovic, 95240 CORMEILLES EN PARISIS (FR)
(74) Representative: Lavoix

(57) **Abstract**

The present invention relates to compound of formula (I) and their use for treating bacterial infections

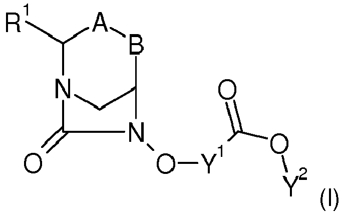

## Description

The present invention relates to heterocyclic compounds especially as prodrug compounds, their process of preparation, the pharmaceutical compositions comprising these compounds and use thereof, optionally in combination with other antibacterial agents and/or beta-lactams, for the prevention or treatment of bacterial infections. The present invention also relates to the use of these compounds as beta-lactamase inhibitors and/or antibacterial agent.

It has been described that there is a continuous evolution of antibacterial resistance which could lead to bacterial strains against which known antibacterial compounds are inefficient. There is thus a need to provide novel compounds and composition that can overcome bacterial antibiotic resistance.

There is also a need to provide antibacterial agents and/or beta-lactamase inhibitors with oral bioavailability. The medical community urgently needs effective oral drugs for the treatment of uncomplicated UTIs.

The objective of the present invention is to provide new heterocyclic compounds, and especially new prodrugs, that can be used as antibacterial agent and/or beta-lactamase inhibitor.
An objective of the present invention is also to provide new heterocyclic compounds, and especially new prodrugs, that can be used for the prevention or treatment of bacterial infections.
Another objective of the present invention is to provide such new compounds which can overcome bacterial antibiotic resistance.
An objective of the invention is also to provide composition comprising these new heterocyclic compounds, optionally in combination with one or more other antibacterial agent, for the prevention or treatment of bacterial infections and which can overcome bacterial antibiotic resistance.
Other objectives will appear throughout the following description of the invention.

The present invention relates to compounds of formula (I) 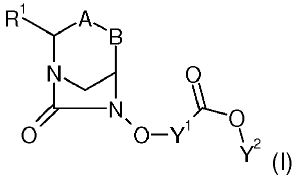 wherein
Y¹ represents CHF or CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C5-C10)-heteroaryl comprising from 1 to 4 heteroatom chosen among N, O or S, (C6-C10)-aryl, (C7-C16)-aralkyl, (C7-C16)-heteroaralkyl comprising from 1 to 4 heteroatom chosen among N, O or S, a (C1-C6)alkyl-heterocycle wherein the heterocycle comprises from 4 to 5 carbon atoms and 1 to 2 heteroatoms chosen among N, O or S, preferably N and O, a polyethylene glycol group (PEG), a cetal group or an acetal group, wherein the alkyl, cycloalkyl, cycloalkenyl, heterocycloalkyl, heteroaryl, aryl, aralkyl, heterocycle and heteroaralkyl is optionally substituted ;
R¹ represents H, CN, CH₂OQ¹, C(=O)OQ¹, C(=O)NQ¹Q², C(=O)NQ¹OQ², C(=O)NQ¹NQ¹Q² or C(=O)ONQ¹Q² ;
Q¹ and Q², identical or different, represents H, linear or branched (C1-C6)-alkyl, (C3-C6)-cycloalkyl, (4-,5-,6-membered)-heterocycle aromatic, saturated or partially unsaturated with at least 1 N and optionally 1 or 2 other heteroatom chosen among N, O or S, linear or branched C(=O)(C1-C6)-alkyl, C(=O)(C1-C6)-cycloalkyl, C(=O)(4-,5-,6-membered)-heterocycle aromatic, saturated or partially unsaturated with at least 1 N and optionally 1 or 2 other heteroatom chosen among N, O or S or Q¹ and Q² form together a saturated or partially unsaturated (4-,5-,6-membered)-heterocycle comprising 1 to 4 heteroatoms chosen among N, O or S; the alkyl, cycloalkyl and heterocycle is optionally substituted;
A-B represents CH₂-C(=NOR²), C(R³)=C(R⁴);
R² represents H, linear or branched (C1-C6)-alkyl, (C1-C6)alkyl-C(=O)NH₂, (C3-C6)-cycloalkyl, (4-,5-,6-membered)-heterocycle aromatic, saturated or partially unsaturated with at least 1 N, the alkyl, cycloalkyl and heterocycle is optionally substituted;
R³ and R⁴, different, represents H, (4 to 10-membered)-heterocycle, aromatic, saturated or partially or totally unsaturated, optionally substituted, or R³ and R⁴ form together with the carbon atoms to which they are linked a non-aromatic cycle of formula (II) 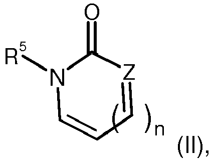
n represents 0 or 1 and Z represents S, N(R⁶) or C(R⁶) with the condition that if Z is S then n=0;
R⁵ represents a linear or branched (C1-C6)-alkyl, a linear or branched (C1-C6)-alkyl-OH, a linear or branched (C1-C6)-alkyl-NH₂, optionally substituted or a (C3-C6)-cycloalkyl optionally substituted; R⁶ represents H, a linear or branched (C1-C6)-alkyl optionally substituted or a (C3-C6)-cycloalkyl optionally substituted;
   - any carbon atom present within a group selected from alkyl, cycloalkyl, cycloalkenyl, heterocycle can be oxidized to form a C(O) group;
   - any sulphur atom present within an heterocycle can be oxidized to form a S(O) group or a S(O)₂ group ;
   - any nitrogen atom present within a group wherein it is trisubstituted (thus forming a tertiary amine) or within an heterocycle can be further quaternized by a methyl group;
with the exception that one of R³ and R⁴ is H and at most one of R³ and R⁴ is H;
and a pharmaceutically acceptable salt, a zwitterion, an optical isomer, a racemate, a diastereoisomer, an enantiomer, a geometric isomer or a tautomer thereof.

The presence of at least one fluorine atom on the molecule, and specifically at the position 2 of the ester function, renders this molecule highly hydrolysable and it is thus very difficult to provide a prodrug sufficiently stable for the targeted effect.

Preferably, in the compounds according to the invention:
- the alkyl, cycloalkyl, cycloalkenyl, heterocycloalkyl, heteroaryl, aryl, aralkyl, heterocycle and heteroaralkyl representing Y² is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴, and
- Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S, the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;

- the alkyl, cycloalkyl and heterocycle representing Q¹, Q² and R² is optionally substituted by one or more T¹ chosen among F, =O, CN, OT³, OC(=O)NT³T⁴, NT³C(=O)T⁴, NT³S(=O)₂T⁴, NT³S(=O)₂NT³T⁴, NT³C(=O)OT⁴, NT³C(=O)NT³T⁴, NT³T⁴, NT³C(=NT³)NT³T⁴, NT³CH(=NT⁴), C(=O)NT³T⁴, C(=O)NT³OT⁴, C(=O)NT³NT³T⁴, C(=NT³)NT³T⁴, linear or branched (C1-C6)-alkyl, (C3-C6)-cycloalkyl, S(=O)NT³T⁴, S(=O)₂NT³T⁴, (4-,5-,6-membered)-heterocycle aromatic, saturated or partially unsaturated with at least 1 N; the alkyl, cycloalkyl, and Heterocycle is optionally substituted by one or more T²; and
- the heterocycle representing R³ and/or R⁴ is optionally substituted by one or more T¹;
- the alkyl, cycloalkyl and heterocycle representing T¹ is optionally substituted by one or more T²;
- T², identical or different, is chosen among F, CN, NT³T⁴, NT³C(=NT³)NT³T⁴, NT³CH(=NT⁴), OT³, NT³C(=O)T⁴ and C(=O)NT³T⁴,
- T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂, and
- the alkyl or cycloalkyl representing R⁵ and R⁶ is optionally substituted by one or more T².

Preferably, in the compounds of formula (I):
Y¹ represents CHF or CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, (C1-C6)alkyl-heterocycle wherein the heterocycle comprises from 4 to 5 carbon atoms and 1 to 2 heteroatoms chosen among N, O or S,
preferably N and O, a polyethylene glycol group (PEG), or a group of formula 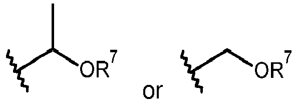
wherein R⁷ represents a linear or branched (C1-C6)-alkyl or C(=O)(C1-C6)-alkyl, wherein the alkyl, cycloalkyl, cycloalkenyl, heterocycloalkyl, aralkyl, heterocycle and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CH₂OQ¹, C(=O)OQ¹, C(=O)NQ¹Q², C(=O)NQ¹OQ² or C(=O)NQ¹NQ¹Q²;
Q¹ and Q², identical or different represents H, linear or branched (C1-C6)-alkyl, (5-,6-membered)-heterocycle aromatic, saturated or partially unsaturated with at least 1 N and optionally 1 or 2 other heteroatom chosen among N, O or S, C(=O)(4-,5-,6-membered)-heterocycle aromatic, saturated or partially unsaturated with at least 1 N and optionally 1 or 2 other heteroatom chosen among N, O or S; the alkyl and heterocycle is optionally substituted by one or more T¹;
A-B represents CH₂-C(=NOR²), C(R³)=C(R⁴);
R² represents H, linear or branched (C1-C6)-alkyl, (C1-C6)alkyl-C(=O)NH₂, the alkyl is optionally substituted by one or more T¹;
R³ and R⁴, different, represents H, (5-,6-membered)-heterocycle aromatic optionally substituted by one or more T¹, or R³ and R⁴ form together with the carbon atoms to which the following cycle: 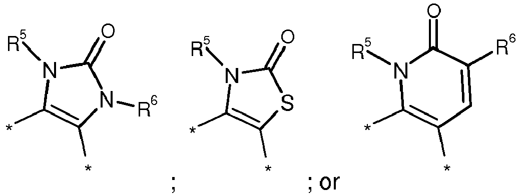
R⁵ different represents a linear or branched (C1-C6)-alkyl optionally substituted by one or more T², a linear or branched (C1-C6)-alkyl-OH, a linear or branched (C1-C6)-alkyl-NH₂, or a (C3-C6)-cycloalkyl optionally substituted by one or more T²;
R⁶ represents H, a linear or branched (C1-C6)-alkyl optionally substituted by one or more T² or a (C3-C6)-cycloalkyl optionally substituted by one or more T²;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents F, =O, CN, OT³, OC(=O)NT³T⁴, NT³C(=O)T⁴, NT³S(=O)₂T⁴, NT³S(=O)₂NT³T⁴, NT³C(=O)OT⁴, NT³C(=O)NT³T⁴, NT³T⁴, NT³C(=NT³)NT³T⁴, NT³CH(=NT⁴), C(=O)NT³T⁴, C(=O)NT³OT⁴, C(=O)NT³NT³T⁴, C(=NT³)NT³T⁴, linear or branched (C1-C6)-alkyl, (C3-C6)-cycloalkyl, S(=O)NT³T⁴, S(=O)₂NT³T⁴, (4-,5-,6-membered)-heterocycle aromatic, saturated or partially unsaturated with at least 1 N; the alkyl, cycloalkyl, and Heterocycle is optionally substituted by one or more T²; and
T², identical or different, is chosen among CN, NT³T⁴, OT³ and C(=O)NT³T⁴,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂;
   - any carbon atom present within a group selected from alkyl, cycloalkyl, cycloalkenyl, heterocycle can be oxidized to form a C(O) group;
   - any sulphur atom present within an heterocycle can be oxidized to form a S(O) group or a S(O)₂ group ;
   - any nitrogen atom present within a group wherein it is trisubstituted (thus forming a tertiary amine) or within an heterocycle can be further quaternized by a methyl group;
with the exception that one of R³ and R⁴ is H and at most one of R³ and R⁴ is H;
and a pharmaceutically acceptable salt, a zwitterion, an optical isomer, a racemate, a diastereoisomer, an enantiomer, a geometric isomer or a tautomer thereof.

Preferably, in the compounds of formula (I) Y² represents linear or branched (C3-C16)-alkyl, (C3-C11)-cycloalkyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, a polyethylene glycol group (PEG), (C1-C6)alkyl-heterocycle wherein the heterocycle comprises from 4 to 5 carbon atoms and 1 to 2 heteroatoms chosen among N, O or S, preferably N and O, (C5-C11)-cycloalkenyl, or a group of formula 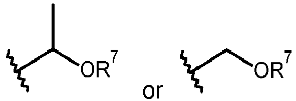 wherein R⁷ represents a linear or branched (C1-C6)-alkyl or C(=O)(C1-C6)-alkyl, the alkyl, cycloalkyl, cycloalkenyl, heterocycloalkyl, aralkyl, heterocycle, and heteroaralkyl is optionally substituted by one or more group chosen among =O, linear or branched (C1-C6)-alkyl.

Preferably, in the compounds of formula (I) R¹ is H or C(=O)NH₂.

Preferably, in the compounds of formula (I):
Y² represents linear or branched (C3-C16)-alkyl, (C3-C11)-cycloalkyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, a polyethylene glycol group (PEG), (C1-C6)alkyl-heterocycle wherein the heterocycle comprises from 4 to 5 carbon atoms and 1 to 2 heteroatoms chosen among N, O or S, preferably N and O, (C5-C11)-cycloalkenyl, or a group of formula 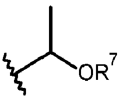 or 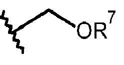
wherein R⁷ represents a linear or branched (C1-C6)-alkyl or C(=O)(C1-C6)-alkyl, the alkyl, cycloalkenyl, cycloalkyl, heterocycloalkyl, heterocycle, aralkyl and heteroaralkyl is optionally substituted by one or more group chosen among =O, linear or branched (C1-C6)alkyl; and
R¹ is H or C(=O)NH₂.

Preferably, the compounds of formula (I) are compounds of formula (IA): 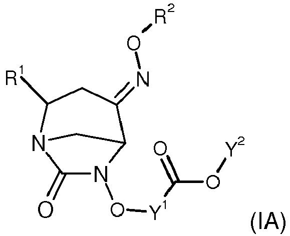 wherein
Y¹ represents CHF or CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, (C1-C6)alkyl-heterocycle wherein the heterocycle comprises from 4 to 5 carbon atoms and 1 to 2 heteroatoms chosen among N, O or S, preferably N and O, a polyethylene glycol group (PEG) or a group of formula 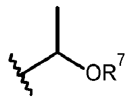 or 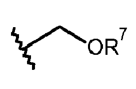
wherein R⁷ represents a linear or branched (C1-C6)-alkyl or C(=O)(C1-C6)-alkyl, wherein the alkyl, cycloalkyl, cycloalkenyl, heterocycloalkyl, aralkyl, heterocycle and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CH₂OQ¹, C(=O)OQ¹, C(=O)NQ¹Q², C(=O)NQ¹OQ² or C(=O)NQ¹NQ¹Q²;
Q¹ and Q², identical or different represents H, linear or branched (C1-C6)-alkyl, (5-,6-membered)-heterocycle aromatic, saturated or partially unsaturated with at least 1 N and optionally 1 or 2 other heteroatom chosen among N, O or S, C(=O)(4-,5-,6-membered)-heterocycle aromatic, saturated or partially unsaturated with at least 1 N and optionally 1 or 2 other heteroatom chosen among N, O or S; the alkyl and heterocycle is optionally substituted by one or more T¹;
R² represents H, linear or branched (C1-C6)-alkyl, (C1-C6)alkyl-C(=O)NH₂, the alkyl is optionally substituted by one or more T¹;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S, the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents OT³, NT³T⁴, C(=O)NT³T⁴, linear or branched (C1-C6)-alkyl, (5-,6-membered)-heterocycle aromatic, saturated or partially unsaturated with at least 1 N; the alkyl and Heterocycle is optionally substituted by one or more T²; and
T², identical or different, is chosen among CN, NT³T⁴, OT³ and C(=O)NT³T⁴,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

Preferably, in the compounds of formula (IA):
Y¹ represents CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, (C1-C6)alkyl-heterocycle wherein the heterocycle comprises from 4 to 5 carbon atoms and 1 to 2 heteroatoms chosen among N, O or S, preferably N and O, a polyethylene glycol group (PEG), or a group of formula 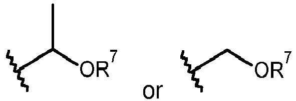
wherein R⁷ represents a linear or branched (C1-C6)-alkyl or C(=O)(C1-C6)-alkyl, wherein the alkyl, cycloalkyl, cycloalkenyl, heterocycloalkyl, aralkyl, heterocycle and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, C(=O)NH₂, CH₂OH, CH₂OMe, or group of formula 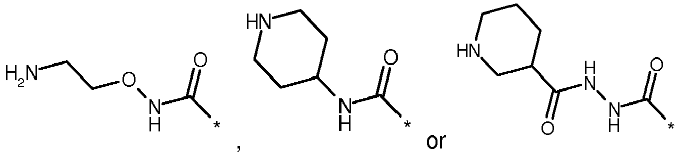
R² represents H, linear or branched (C1-C6)-alkyl, (C1-C6)alkyl-C(=O)NH₂, the alkyl is optionally substituted by one or more T¹;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents OH, OMe, NH₂, CN, C(=O)NH₂, linear or branched (C1-C6)-alkyl; the alkyl is optionally substituted by one or more T²; and
T², identical or different, is chosen among OH, OMe, NH₂, CN, C(=O)NH₂.

Preferably, in compounds of formula (IA) R¹ is C(=O)NH₂.

Preferably, in compounds of formula (IA) R² is (C1-C6)alkyl-C(=O)NH₂.

Preferably, in compounds of formula (IA) R¹ is C(=O)NH₂ and R² is (C1-C6)alkyl-C(=O)NH₂

Preferably, the compounds of formula (I) are compounds of formula (IB): 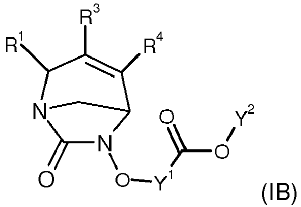 wherein
Y¹ represents CHF or CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, (C1-C6)alkyl-heterocycle wherein the heterocycle comprises from 4 to 5 carbon atoms and 1 to 2 heteroatoms chosen among N, O or S, preferably N and O, a polyethylene glycol group (PEG), or a group of formula 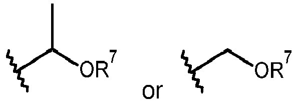
wherein R⁷ represents a linear or branched (C1-C6)alkyl or C(=O)(C1-C6)alkyl, wherein the alkyl, cycloalkyl, cycloalkenyl, heterocycloalkyl, aralkyl, heterocycle and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CH₂OQ¹, C(=O)OQ¹, C(=O)NQ¹Q², C(=O)NQ¹OQ² or C(=O)NQ¹NQ¹Q²;
Q¹ and Q², identical or different represents H, linear or branched (C1-C6)-alkyl, (5-,6-membered)-heterocycle aromatic, saturated or partially unsaturated with at least 1 N and optionally 1 or 2 other heteroatom chosen among N, O or S, C(=O)(4-,5-,6-membered)-heterocycle aromatic, saturated or partially unsaturated with at least 1 N and optionally 1 or 2 other heteroatom chosen among N, O or S; the alkyl and heterocycle is optionally substituted by one or more T¹;
R³ and R⁴, different, represents H, (5-,6-membered)-heterocycle aromatic optionally substituted by one or more T¹, or R³ and R⁴ form together with the carbon atoms to which the following cycle: 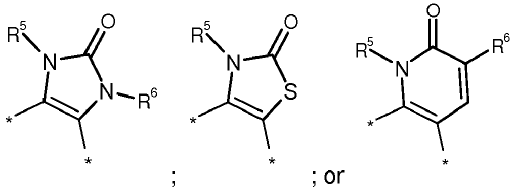
R⁵ different represents a linear or branched (C1-C6)-alkyl optionally substituted by one or more T², a linear or branched (C1-C6)-alkyl-OH, a linear or branched (C1-C6)-alkyl-NH₂, or a (C3-C6)-cycloalkyl optionally substituted by one or more T²;
R⁶ represents H, a linear or branched (C1-C6)-alkyl optionally substituted by one or more T² or a (C3-C6)-cycloalkyl optionally substituted by one or more T²;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents F, OT³, NT³C(=O)T⁴, NT³T⁴, CN, C(=O)NT³T⁴, C(=O)NT³OT⁴, C(=O)NT³NT³T⁴, linear or branched (C1-C6)-alkyl, (5-,6-membered)-heterocycle aromatic, saturated or partially unsaturated with at least 1 N; the alkyl, and Heterocycle is optionally substituted by one or more T²; and
T², identical or different, is chosen among CN, NT³T⁴, OT³ and C(=O)NT³T⁴,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

Preferably, in the compounds of formula (IB):
Y¹ represents CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, (C1-C6)alkyl-heterocycle wherein the heterocycle comprises from 4 to 5 carbon atoms and 1 to 2 heteroatoms chosen among N, O or S, preferably N and O, a polyethylene glycol group (PEG), or a group of formula 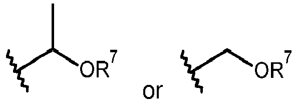
wherein R⁷ represents a linear or branched (C1-C6)-alkyl or C(=O)(C1-C6)-alkyl, wherein the alkyl, cycloalkyl, cycloalkenyl, heterocycloalkyl, aralkyl, heterocycle and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CONH₂, CH₂OH, CH₂OMe, or group of formula 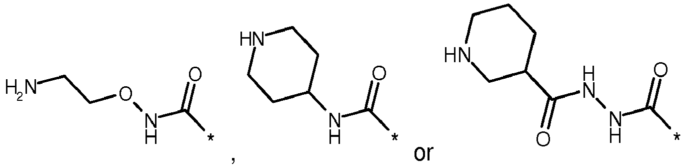
R³ and R⁴, different, represents H, (5-,6-membered)-heterocycle aromatic optionally substituted by one or more T¹, or R³ and R⁴ form together with the carbon atoms to which the following cycle: 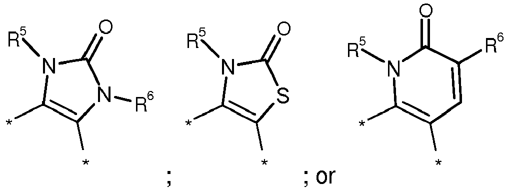
R⁵ different represents a linear or branched (C1-C6)-alkyl optionally substituted by one or more T², a linear or branched (C1-C6)-alkyl-OH, a linear or branched (C1-C6)-alkyl-NH₂, or a (C3-C6)-cycloalkyl optionally substituted by one or more T²;
R⁶ represents H, a linear or branched (C1-C6)-alkyl optionally substituted by one or more T² or a (C3-C6)-cycloalkyl optionally substituted by one or more T²;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents F, OT³, NT³C(=O)T⁴, NT³T⁴, CN, C(=O)NT³T⁴, C(=O)NT³OT⁴, C(=O)NT³NT³T⁴, linear or branched (C1-C6)-alkyl, (5-,6-membered)-heterocycle aromatic, saturated or partially unsaturated with at least 1 N; the alkyl, and Heterocycle is optionally substituted by one or more T²; and
T², identical or different, is chosen among CN, NH₂, OH, OMe, and C(=O)NH₂,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

Preferably, the compounds of formula (IB) are compounds of formula (IB1): 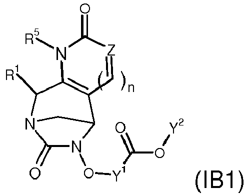 wherein
Y¹ represents CHF or CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, (C1-C6)alkyl-heterocycle wherein the heterocycle comprises from 4 to 5 carbon atoms and 1 to 2 heteroatoms chosen among N, O or S, preferably N and O, or a group of formula 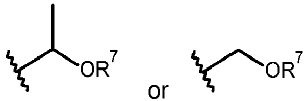
wherein R⁷ represents a linear or branched (C1-C6)alkyl or C(=O)(C1-C6)alkyl, a polyethylene glycol group (PEG), wherein the alkyl, cycloalkyl, cycloalkenyl, heterocycloalkyl, aralkyl, heterocycle and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CH₂OQ¹, C(=O)OQ¹, C(=O)NQ¹Q², C(=O)NQ¹OQ² or C(=O)NQ¹NQ¹Q²;
Q¹ and Q², identical or different represents H, linear or branched (C1-C6)-alkyl, (5-,6-membered)-heterocycle aromatic, saturated or partially unsaturated with at least 1 N and optionally 1 or 2 other heteroatom chosen among N, O or S, C(=O)(4-,5-,6-membered)-heterocycle aromatic, saturated or partially unsaturated with at least 1 N and optionally 1 or 2 other heteroatom chosen among N, O or S; the alkyl and heterocycle is optionally substituted by one or more T¹;
n is 0 or 1;
Z is S, NR⁶ or CR⁶
R⁵ different represents a linear or branched (C1-C6)-alkyl optionally substituted by one or more T², a linear or branched (C1-C6)-alkyl-OH, a linear or branched (C1-C6)-alkyl-NH₂, or a (C3-C6)-cycloalkyl optionally substituted by one or more T²;
R⁶ represents H, a linear or branched (C1-C6)-alkyl optionally substituted by one or more T² or a (C3-C6)-cycloalkyl optionally substituted by one or more T²;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents F, OT³, NT³C(=O)T⁴, NT³T⁴, CN, C(=O)NT³T⁴, C(=O)NT³OT⁴, C(=O)NT³NT³T⁴, linear or branched (C1-C6)-alkyl, (5-,6-membered)-heterocycle aromatic, saturated or partially unsaturated with at least 1 N; the alkyl, and Heterocycle is optionally substituted by one or more T²; and
T², identical or different, is chosen among CN, NT³T⁴, OT³ and C(=O)NT³T⁴,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

Preferably, in the compounds of formula (IB1):
Y¹ represents CF₂;
Y² represents linear or branched (C3-C16)-alkyl, (C3-C11)-cycloalkyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, a polyethylene glycol group (PEG), (C1-C6)alkyl-heterocycle wherein the heterocycle comprises from 4 to 5 carbon atoms and 1 to 2 heteroatoms chosen among N, O or S, preferably N and O, (C5-C11)-cycloalkenyl, or a group of formula 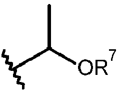 or 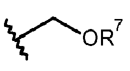
wherein R⁷ represents a linear or branched (C1-C6)-alkyl or C(=O)(C1-C6)-alkyl, wherein the alkyl, cycloalkyl, cycloalkenyl, heterocycle, heterocycloalkyl, aralkyl and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CH₂OH, CH₂OMe, or group of formula 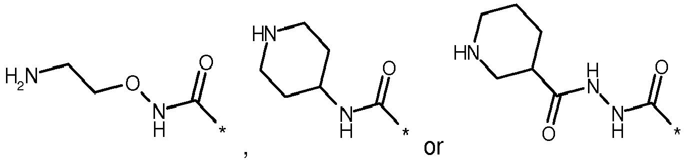 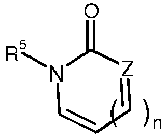 represents 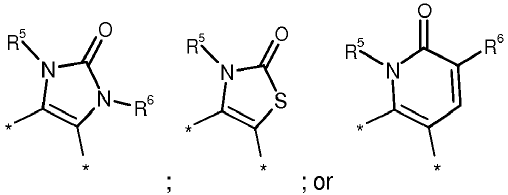
R⁵ different represents a linear or branched (C1-C6)-alkyl optionally substituted by one or more T², a linear or branched (C1-C6)-alkyl-OH, a linear or branched (C1-C6)-alkyl-NH₂, or a (C3-C6)-cycloalkyl optionally substituted by one or more T²;
R⁶ represents H, a linear or branched (C1-C6)-alkyl optionally substituted by one or more T² or a (C3-C6)-cycloalkyl optionally substituted by one or more T²;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents F, OT³, NT³C(=O)T⁴, NT³T⁴, CN, C(=O)NT³T⁴, C(=O)NT³OT⁴, C(=O)NT³NT³T⁴, linear or branched (C1-C6)-alkyl, (5-,6-membered)-heterocycle aromatic, saturated or partially unsaturated with at least 1 N; the alkyl, and Heterocycle is optionally substituted by one or more T²; and
T², identical or different, is chosen among CN, NH₂, OH, OMe, and C(=O)NH₂,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

Preferably, in the compounds of formula (IB) and (IB1): 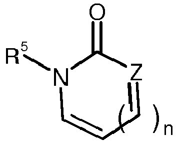 represents 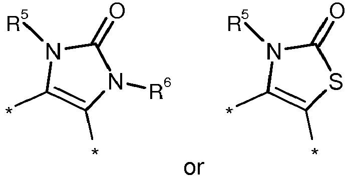 wherein R⁵ and R⁶ are as mentioned above, preferably R⁵ is linear or branched (C1-C6)-alkyl, linear or branched (C1-C6)-alkyl-OH, linear or branched (C1-C6)-alkyl-NH² and R⁶ is H or linear or branched (C1-C6)alkyl.

Preferably, in the compounds of formula (IB) and (IB1) R¹ is H.

Preferably, in the compounds of formula (IB) and (IB1):
R¹ is H, and 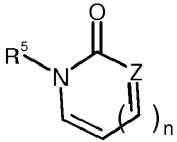 represents 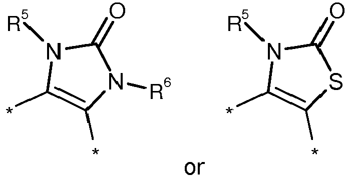
wherein R⁵ and R⁶ are as mentioned above, preferably R⁵ is linear or branched (C1-C6)-alkyl, linear or branched (C1-C6)-alkyl-OH, linear or branched (C1-C6)-alkyl-NH² and R⁶ is H or linear or branched (C1-C6)-alkyl.

Preferably, the compounds of formula (IB) are compounds of formula (IB2): 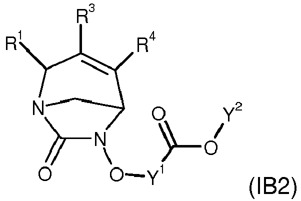 wherein
Y¹ represents CHF or CF₂;
Y² represents linear or branched (C3-C16)-alkyl, (C3-C11)-cycloalkyl, (C1-C6)alkyl-heterocycle wherein the heterocycle comprises from 4 to 5 carbon atoms and 1 to 2 heteroatoms chosen among N, O or S, preferably N and O, (C5-C11)-cycloalkenyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, a polyethylene glycol group (PEG), or a group of formula 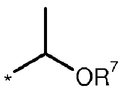 or 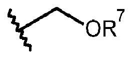
wherein R⁷ represents a linear or branched (C1-C6)alkyl or C(=O)(C1-C6)alkyl, wherein the alkyl, cycloalkyl, cycloalkenyl, heterocycle, heterocycloalkyl, aralkyl and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CH₂OQ¹, C(=O)OQ¹, C(=O)NQ¹Q², C(=O)NQ¹OQ² or C(=O)NQ¹NQ¹Q²;
Q¹ and Q², identical or different represents H, linear or branched (C1-C6)-alkyl, (5-,6-membered)-heterocycle aromatic, saturated or partially unsaturated with at least 1 N and optionally 1 or 2 other heteroatom chosen among N, O or S, C(=O)(4-,5-,6-membered)-heterocycle aromatic, saturated or partially unsaturated with at least 1 N and optionally 1 or 2 other heteroatom chosen among N, O or S; the alkyl and heterocycle is optionally substituted by one or more T¹;
R³ and R⁴, different, represents H, (5-,6-membered)-heterocycle aromatic optionally substituted by one or more T¹,
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents F, OT³, NT³C(=O)T⁴, NT³T⁴, CN, C(=O)NT³T⁴, C(=O)NT³OT⁴, C(=O)NT³NT³T⁴, linear or branched (C1-C6)-alkyl, (5-,6-membered)-heterocycle aromatic, saturated or partially unsaturated with at least 1 N; the alkyl, and Heterocycle is optionally substituted by one or more T²; and
T², identical or different, is chosen among CN, NT³T⁴, OT³ and C(=O)NT³T⁴,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

Preferably, in the compounds of formula (IB2) one of R³ and R⁴ is H and the other is a 5-membered heteroaryl comprising at least one nitrogen atom and another heteroatom chosen among N or O.

Preferably, in the compounds of formula (IB2) Y¹ is CF₂.

Preferably, in the compounds of formula (IB2) Y² represents linear or branched (C3-C16)-alkyl, (C3-C11)-cycloalkyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, or a group of formula 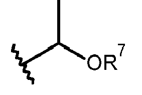 or 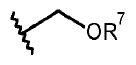 wherein R⁷ represents a linear or branched (C1-C6)alkyl or C(=O)(C1-C6)alkyl, the alkyl, cycloalkyl, heterocycloalkyl, aralkyl and heteroaralkyl is optionally substituted by one or more group chosen among =O, linear or branched (C1-C6)alkyl.

Preferably, in the compounds of formula (IB2), one of R³ and R⁴ is H and the other is chosen from oxazole or pyrazole.

Preferably, in the compounds of formula (IB2) R¹ is H.

Preferably, in the compounds of formula (IB2):
one of R³ and R⁴ is H and the other is a 5-membered heteroaryl comprising at least one nitrogen atom and another heteroatom chosen among N or O, preferably one of R³ and
R⁴ is H and the other is chosen from oxazole or pyrazole;
Y¹ is CF₂;
Y² represents linear or branched (C3-C16)-alkyl, (C3-C11)-cycloalkyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, or a group of formula 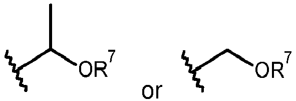
wherein R⁷ represents a linear or branched (C1-C6)alkyl or C(=O)(C1-C6)alkyl, the alkyl, cycloalkyl, heterocycloalkyl, aralkyl and heteroaralkyl is optionally substituted by one or more group chosen among =O, linear or branched (C1-C6)-alkyl; and
R¹ is H.

Preferably, the compounds of formula (I) according to the invention are compounds of formula (I*) 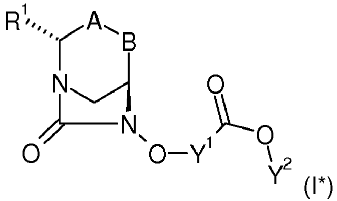 wherein R¹, A, B, Y¹ and Y² are as defined above.

Preferably, the compounds of formula (IA) according to the invention are compounds of formula (IA*) 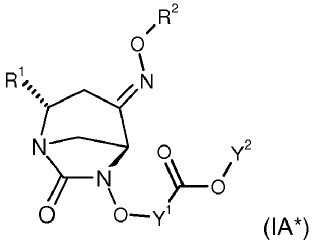 wherein R¹, R², Y¹ and Y² are as defined above.

Preferably, the compounds of formula (IB) according to the invention are compounds of formula (IB*) 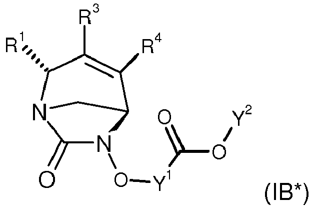 wherein R¹, R³, R⁴, Y¹ and Y² are as defined above.

Preferably, the compounds of formula (IB1) according to the invention are compounds of formula (IB1*) 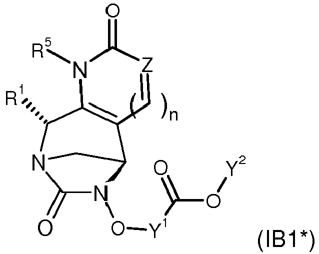 wherein R¹, R⁵, Z, n, Y¹ and Y² are as defined above.

Preferably, the compounds of formula (IB2) according to the invention are compounds of formula (IB2*) 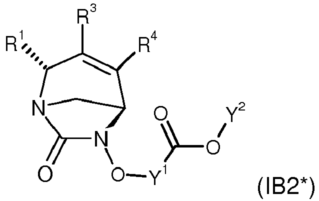 wherein R¹, R³, R⁴, Y¹ and Y² are as defined above.

The compounds of formula (I), (I*), (IA), (IA*), (IB), (IB*), (IB1*), (IB2*) according to the invention with Y² different from H, can be used as a pro-drug of a compound of formula (I'), (I'*), (IA'), (IA'*), (IB'), (IB'*) 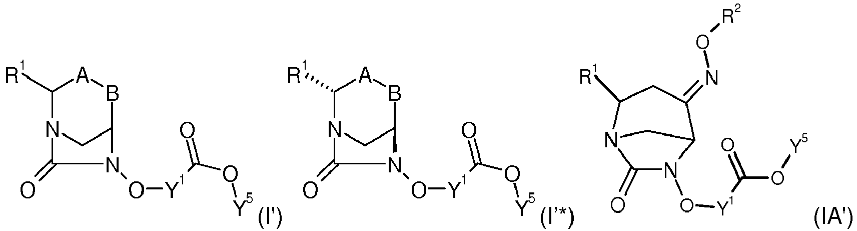 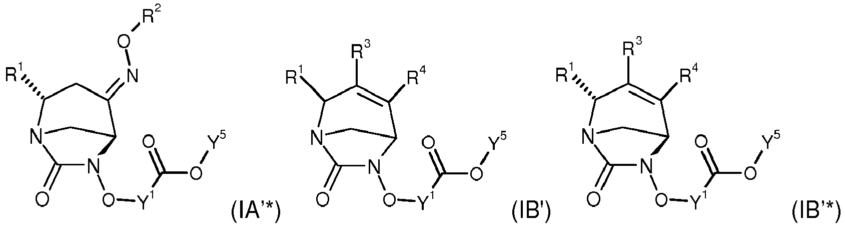 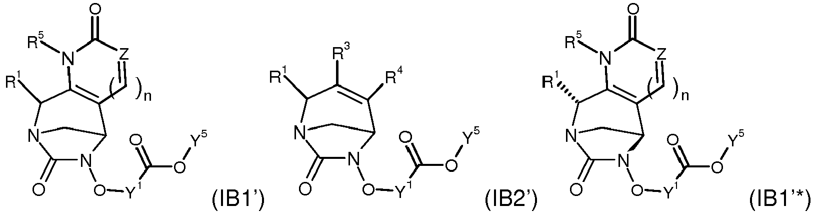 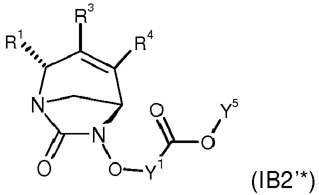 wherein R¹, R², R³, R⁴, A-B, n, R⁵, Z and Y¹ are as defined above and Y⁵ represents H or a base addition salts for example chosen among ammonium salts such as tromethamine, meglumine, epolamine; metal salts such as sodium, lithium, potassium, calcium, zinc, aluminium or magnesium; salts with organic bases such as methylamine, propylamine, trimethylamine, diethylamine, triethylamine, N,N-dimethylethanolamine, tris(hydroymethyl)aminomethane, ethanolamine, pyridine, picoline, dicyclohexylamine, morpholine, benzylamine, procaine, N-methyl-D-glucamine; salts with amino acids such as arginine, lysine, ornithine and so forth; phosphonium salts such as alkylphosphonium, arylphosphonium, alkylarylphosphonium and alkenylarylphosphonium; and salts with quaternary ammonium such as tetra-n-butylammonium. List of suitable salts may be found in Remington's Pharmaceutical Sciences, 17th ed. Mack Publishing Company, Easton, PA, 1985, p 1418, P.H. Stahl, C.G. Wermuth, Hanbook of Pharmaceutical salts - Properties, Selection and Use, Wiley-VCH, 2002 and S.M. Berge et al. "Pharmaceutical Salts" J. Pharm. Sci, 66: p.1-19 (1977).

The term "alkyl", as used herein, refers to an aliphatic-hydrocarbon group which may be linear or branched, having 1 to 16 carbon atoms in the chain unless specified otherwise. Specific examples of alkyl groups, linear or branched, include, but are not limited to, methyl, ethyl, propyl, butyl, pentyl, hexyl, heptyl, octyl, nonyl, decyl, undecyl, dodecyl, tridecyl, tetradecyl, pentadecyl, hexadecyl. Preferably, the alkyl group, straight or branched, is or, propyl, pentyl, heptyl, hexadecyl.

The term "cycloalkyl" refers to a saturated monocyclic, polycyclic or spirocyclic non-aromatic hydrocarbon ring of 3 to 11 carbon atoms. Specific examples of monocyclic, polycyclic or spirocyclic cycloalkyl groups include, but are not limited to, cyclopropyl, cyclobutyl, cyclopentyl, cyclohexyl, cycloheptyl, cyclooctyl, cyclononyl, cyclodecyl, cycloundecyl, decalyl, norbornyl, isopinocamphyl, norpinanyl, adamantyl, spirohexane, spiroheptane, spirooctane, spirononane, spirodecane, spiroundecane. Preferably, the cycloalkyl group is cyclohexyl, adamantyl.

The term "cycloalkenyl" refers to a saturated monocyclic or bicyclic non-aromatic hydrocarbon ring of 5 to 11 carbon atoms and comprising at least one unsaturation. Specific examples of cycloalkenyl groups include, but are not limited to cyclopentenyl, cyclohexenyl, cycloheptenyl, cyclooctenyl. Preferably, the cycloalkenyl group is cyclohexenyl.

The term "heterocycle" or "heterocycloalkyl", as used herein and without contrary definition specifically mentioned, either alone or in combination with another radical, refers to a monocyclic, bicyclic or spirocyclic saturated or partially unsaturated hydrocarbon radical, preferably 4 to 10-membered, comprising one or two heteroatom, such as N, O, S, and linked to the structure of the compounds by a carbon atom of the heterocycloalkyl. Suitable heterocycloalkyl are also disclosed in the Handbook of Chemistry and Physics, 76th Edition, CRC Press, Inc., 1995-1996, pages 2 25 to 2-26. Specific examples of heterocycloalkyl groups include, but are not limited to, azetidinyl, oxetanyl, oxazolidinyl, pyrrolidinyl, tetrahydropyridinyl, piperidinyl, morpholinyl, thiomorpholinyl, dioxanyl, pyrrolidinyl, imidazolidinyl, pyranyl, tetrahydrofuranyl, dioxolanyl, tetrahydropyranyl, tetrahydroquinolinyl, dihydrobenzoxazinyl, oxepanyl, azaspirooctanyl, azaspirodecanyl, oxaspirooctanyl, oxaspirodecanyl, thiaspirooctanyl, thiaspirodecanyl. Preferably, the heterocycloalkyl group is piperidinyl, pyranyl, oxepanyl, morpholinyl, thiomorpholinyl.

The term "heteroaryl", as used herein and without contrary definition specifically mentioned, either alone or in combination with another radical, refers to a monocyclic or bicyclic aromatic hydrocarbon radical, preferably 5 to 10-membered, comprising one, two, three or four heteroatom, such as N, O, S. Suitable heteroaryl are also disclosed in the Handbook of Chemistry and Physics, 76th Edition, CRC Press, Inc., 1995-1996, pages 2-25 to 2-26. Specific examples of heteroaryl groups include, but are not limited to, oxazolyl, oxadiazolyl, pyrrolyl, pyridyl, pyrazolyl, pyrimidinyl, pyrazinyl, tetrazolyl, triazolyl, thienyl, thiazolyl, furanyl, thiadiazolyl, isothiazolyl, isoxazolyl. Preferably, the heteroaryl group is pyridinyl, furanyl, thiazolyl, thienyl, imidazolyl.

The term "aryl", as used herein and without contrary definition specifically mentioned, either alone or in combination with another radical, refers to a monocyclic or bicyclic aromatic hydrocarbon radical. Specific examples of aryl groups include phenyl, naphtyl.

The term "aralkyl", as used herein and without contrary definition specifically mentioned, refers to an alkyl substituted by an aryl, the alkyl and aryl being as defined above. By (C7-C16)-aralkyl it should be understand that the aralkyl group comprises in total from 7 to 16 carbon atoms. Specific examples of aralkyl groups include, but are not limited to benzyl, phenylethyl, phenylpropyl, phenylbutyl, phenylpentyl, phenylhexyl, phenylheptyl, phenyloctyl, phenylnonyln phenyldecyl, naphtylethyl, naphtylpropyl, naphtylbutyl, naphtylpentyl, naphtylhexyl.

The term "heteroaralkyl", as used herein and without contrary definition specifically mentioned, refers to an alkyl substituted by an heteroaryl, the alkyl and heteroaryl being as defined above. By (C7-C16)-heteroaralkyl it should be understand that the heteroaralkyl group comprises in total from 7 to 16 carbon atoms.

The term "cetal", as used herein and without contrary definition specifically mentioned, refers to a group consisting of Y² of formula 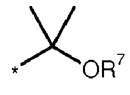 and the oxygen atom to which Y² is linked, wherein R⁷ represents a linear or branched (C1-C6)alkyl or C(=O)(C1-C6)alkyl.

The term "acetal", as used herein and without contrary definition specifically mentioned, refers to a group consisting of Y² of formula 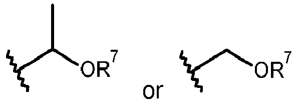 and the oxygen atom to which Y² is linked, wherein R⁷ represents a linear or branched (C1-C6)-alkyl or C(=O)(C1-C6)-alkyl.

The term "PEG" or "polyethylene glycol", as used herein and without contrary definition specifically mentioned, refers to a group Y² of formula 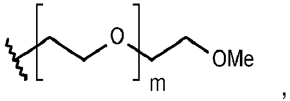 wherein m is an integer from 1 to 10.

Moreover some compounds according to this invention may contain a basic amino group and thus may form an inner zwitterionic salt (or zwitterion) with the acidic group - OCHFCO₂H or -OCF₂CO₂H where Y² is H and such inner zwitterionic salts are also included in this invention.

The term "optionally substituted" means "non-substituted or substituted".

The term "racemate" is employed herein to refer to an equal amount of two specific enantiomers.

The term "enantiomer" is employed herein to refer to one of the two specific stereoisomers which is a non-superimposable mirror image with one other but is related to one other by reflection.

The compounds of the invention can possess one or more asymmetric carbon atoms and are thus capable of existing in the form of optical isomers as well as in the form of racemic or non-racemic mixtures thereof. The compounds of the invention can be used in the present invention as a single isomer or as a mixture of stereochemical isomeric forms. Diastereoisomers, i.e., nonsuperimposable stereochemical isomers can be separated by conventional means such as chromatography, distillation, crystallization or sublimation. The optical isomers (enantiomers) can be obtained by using optically active starting materials, by resolution of the racemic mixtures according to conventional processes, for example by formation of diastereoisomeric salts by treatment with an optically active acid or base or by using chiral chromatography column.

The expression "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the expression "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention can be synthesized from the parent compound which comprises a basic or an acidic moiety, by conventional chemical methods.

Furthermore, the expression "pharmaceutically acceptable salt" refers to relatively nontoxic, inorganic and organic acid or base addition salts of the compounds of the present invention. These salts can be prepared in situ during the final isolation and purification of the compounds. In particular, the acid addition salts can be prepared by separately reacting the purified compound in its purified form with an organic or inorganic acid and by isolating the salt thus formed. Among the examples of acid addition salts are the hydrobromide, hydrochloride, hydroiodide, sulfamate, sulfate, bisulfate, phosphate, nitrate, acetate, propionate, succinate, oxalate, valerate, oleate, palmitate, stearate, laurate, borate, benzoate, lactate, tosylate, citrate, maleate, fumarate, tartrate, naphthylate, mesylate, glucoheptanate, glucoronate, glutamate, lactobionate, malonate, salicylate, methylenebis-b-hydroxynaphthoate, gentisic acid, isethionate, di-p-toluoyltartrate, ethanesulfonate, benzenesulfonate, cyclohexyl sulfamate, quinateslaurylsulfonate salts, and the like. Examples of base addition salts include ammonium salts such as tromethamine, meglumine, epolamine, etc, metal salts such as sodium, lithium, potassium, calcium, zinc or magnesium salts with organic bases such as dicyclohexylamine salts, N-methyl-D-glucamine. Lists of suitable salts may be found in Remington's Pharmaceutical Sciences, 17th ed., Mack Publishing Company, Easton, PA, 1985, p. 1418, P.H. Stahl, C.G. Wermuth, Handbook of Pharmaceutical salts - Properties, Selection and Use, Wiley-VCH, 2002 and S.M. Berge et al. "Pharmaceutical Salts" J. Pharm. Sci, 66: p.1-19 (1977).

Compounds according to the invention also include isotopically-labeled compounds wherein one or more atoms is replaced by an atom having the same atomic number, but an atomic mass or mass number different from the atomic mass or mass number usually found in nature. Examples of isotopes suitable for inclusion in the compounds described above and are not limited to ²H, ³H, ¹¹C, ¹³C, ¹⁴C, ¹⁹F, ¹⁸F, ¹⁵N, ¹³N, ³³S, ³⁴S, ³⁵S, ³⁶S, ¹⁷O or ¹⁸O. In one embodiment, isotopically-labeled compounds are useful in drug and/or substrate tissue distribution studies. In another embodiment, substitution with heavier isotopes such as deuterium (²H) affords greater metabolic stability (for example increased in vivo half-life or reduced dosage requirements). Isotopically-labeled compounds are prepared by any suitable method or by processes using an appropriate isotopically-labeled reagent in place of the non-labeled reagent otherwise employed.

The present invention also relates to a pharmaceutical composition comprising at least a compound of formula (I), (I*), (IA), (IA*), (IB), (IB*), (IB1), (IB1*), (IB2) or (IB2)* according to the invention.

This pharmaceutical composition can further comprise at least one pharmaceutically acceptable excipient.

The term "pharmaceutically acceptable carrier" or "pharmaceutically acceptable excipient" is employed for any excipient, solvent, dispersion medium, absorption retardant, diluent or adjuvant etc., such as preserving or antioxidant agents, fillers, binders, disintegrating agents, wetting agents, emulsifying agents, suspending agents, solvents, dispersion media, coatings, antibacterial agents, isotonic and absorption delaying agents and the like, that does not produce a secondary reaction, for example an allergic reaction, in humans or animals. Typical, non-limiting examples of excipients include mannitol, lactose, magnesium stearate, sodium saccharide, talcum, cellulose, sodium croscarmellose, glucose, gelatin, starch, lactose, dicalcium phosphate, sucrose, kaolin, magnesium carbonate, wetting agents, emulsifying agents, solubilizing agents, sterile water, saline, pH buffers, non-ionic surfactants, lubricants, stabilizing agents, binding agents and edible oils such as peanut oil, sesame oils and the like. In addition, various excipients commonly used in the art may be included. Pharmaceutically acceptable carriers or excipients are well known to a person skilled in the art, and include those described in Remington's Pharmaceutical Sciences (Mack Publishing Company, Easton, USA, 1985), Merck Index (Merck & Company, Rahway, N.J.), Gilman et al (Eds. The pharmacological basis of therapeutics, 8th Ed., pergamon press., 1990). Except insofar as any conventional media or adjuvant is incompatible with the active ingredient according to the invention, its use in the therapeutic compositions is contemplated.

The pharmaceutical composition according to the invention can further comprise at least one compound selected from an antibacterial compound, preferably a β-lactam compound. Thus, the pharmaceutical composition according to the invention can comprise:
- a single compound of formula (I), (I*), (IA), (IA*), (IB), (IB*), (IB1), (IB1*), (IB2) or (IB2)* according to the invention ; or
- at least one compound of formula (I), (I*), (IA), (IA*), (IB), (IB*), (IB1), (IB1*), (IB2) or (IB2)* according to the invention and one or more antibacterial compound ; or
- at least one compound of formula (I), (I*), (IA), (IA*), (IB), (IB*), (IB1), (IB1*), (IB2) or (IB2)* according to the invention and one or more β-lactam compound ; or
- at least one compound of formula (I), (I*), (IA), (IA*), (IB), (IB*), (IB1), (IB1*), (IB2) or (IB2)* according to the invention, one or more antibacterial compound and one or more β-lactam compound.

The term "beta-lactam" or "β-lactam" refers to antibacterial compounds comprising a β-lactam unit, i.e. a group.

The expression "antibacterial agent" as used herein, refers to any substance, compound or their combination capable of inhibiting, reducing or preventing growth of bacteria, inhibiting or reducing ability of bacteria to produce infection in a subject, or inhibiting or reducing ability of bacteria to multiply or remain infective in the environment, or decreasing infectivity or virulence of bacteria.

The antibacterial agent is selected among the following families: aminoglycosides, beta-lactams, glycylcyclines, tetracyclines, quinolones, fluoroquinolones, glycopeptides, lipopeptides, macrolides, ketolides, lincosamides, streptogramins, oxazolidinones and polymyxins alone or in mixture.

Preferably, the further antibacterial agent is selected among the beta-lactam families, and more preferably among penicillin, cephalosporins, penems, carbapenems and monobactam, alone or in mixture.

Among the penicillin the antibacterial agent is preferably selected in the group consisting of amoxicillin, ampicillin, azlocillin, mezocillin, apalcillin, hetacillin, bacampicillin, carbenicillin, sulbenicillin, temocillin, ticarcillin, piperacillin, mecillinam, pivmecillinam, methicillin, ciclacillin, talampacillin, aspoxicillin, oxacillin, cloxacillin, dicloxacillin, flucloxacillin, nafcillin, and pivampicillin, alone or in mixture.

Among the cephalosporin, the antibacterial agent is preferably selected in the group consisting of cefatriazine, cefazolin, cefoxitin, cephalexin, cephradine, ceftizoxime, cephacetrile, cefbuperazone, cefprozil, ceftobiprole, ceftobiprole medocaril, ceftaroline, ceftaroline fosaminyl, cefalonium, cefminox, ceforanide, cefotetan, ceftibuten, cefcapene pivoxil, cefditoren pivoxil, cefdaloxime cefroxadine, ceftolozane and S-649266, cephalothin, cephaloridine, cefaclor, cefadroxil, cefamandole, cefazolin, cephalexin, cephradine, ceftizoxime, cephacetrile, cefotiam, cefotaxime, cefsulodin, cefoperazone, cefmenoxime, cefmetazole, cephaloglycin, cefonicid, cefodizime, cefpirome, ceftazidime, ceftriaxone, cefpiramide, cefbuperazone, cefozopran, cefepime, cefoselis, cefluprenam, cefuzonam, cefpimizole, cefclidine, cefixime, ceftibuten, cefdinir, cefpodoxime axetil, cefpodoxime proxetil, cefteram pivoxil, cefetamet pivoxil, cefcapene pivoxil, cefditoren pivoxil, cefuroxime, cefuroxime axetil, loracarbef, and latamoxef, alone or in mixture.

Among the carbapenem, the antibacterial agent is preferably selected in the group consisting of imipenem, doripenem, meropenem, biapenem, ertapenem, tebipenem and panipenem, alone or in mixture.

Among the monobactam the antibacterial agent is preferably selected in the group consisting of aztreonam, tigemonam, carumonam, BAL30072 and nocardicin A, alone or in mixture.

Preferably, in the pharmaceutical composition according to the invention:
- the antibacterial compound is selected from aminoglycosides, β-lactams, glycylcyclines, tetracyclines, quinolones, fluoroquinolones, glycopeptides, lipopeptides, macrolides, ketolides, lincosamides, streptogramins, oxazolidinones, polymyxins and mixtures thereof; or
- the β-lactam compound is selected from β-lactams, and mixtures thereof, preferably penicillin, cephalosporins, penems, carbapenems and monobactam.

Preferably, in the pharmaceutical composition according to the invention:
- the antibacterial compound is selected from orally bioavailable aminoglycosides, β-lactams, glycylcyclines, tetracyclines, quinolones, fluoroquinolones, glycopeptides, lipopeptides, macrolides, ketolides, lincosamides, streptogramins, oxazolidinones, polymyxins and mixtures thereof; or
- the β-lactam compound is selected from orally available β-lactams, or prodrugs of β-lactams, and mixtures thereof, preferably penicillin, cephalosporins, penems, carbapenems and monobactam.

Preferably, in the pharmaceutical composition according to the invention the β-lactam is chosen among amoxicillin, amoxicillin-clavulanate, sultamicillin cefuroxime, cefazolin, cefaclor, cefdinir, cefpodoxime, cefprozil, cephalexin, loracarbef, cefetamet, ceftibuten, tebipenem pivoxil, cefixime, preferably cefixime.

The present invention also relates to a kit comprising:
- a pharmaceutical composition according to the invention, and
- at least one other composition comprising one or more antibacterial agent(s), preferably at least one of these antibacterial agent(s) is a beta-lactam, the antibacterial agent being as defined above.

The two composition can be prepared separately each with one specific pharmaceutically acceptable carrier, and can be mix especially extemporaneity.

The present invention also refer to a compound of formula (I), (I*), (IA), (IA*), (IB), (IB*), (IB1), (IB1*), (IB2) or (IB2)* according to the invention for use as a medicine.

The present invention also refer to the use of a compound of formula (I), (I*), (IA), (IA*), (IB), (IB*), (IB1), (IB1*), (IB2) or (IB2)* according to the invention or of a composition according to the invention for the preparation of a medicine.

The present invention also provides the use of the compounds of formula (I), (I*), (IA), (IA*), (IB), (IB*), (IB1), (IB1*), (IB2) or (IB2)* on the control of bacteria. The compound according to the invention is usually used in combination with pharmaceutically acceptable excipient.

The present invention also refer to a compound of formula (I), (I*), (IA), (IA*), (IB), (IB*), (IB1), (IB1*), (IB2) or (IB2)* according to the invention for use as antibacterial agent.

The present invention also refer to a compound of formula (I), (I*), (IA), (IA*), (IB), (IB*), (IB1), (IB1*), (IB2) or (IB2)* according to the invention for use as inhibitor of beta-lactamase.

The present invention also refer to the use of a compound of formula (I), (I*), (IA), (IA*), (IB), (IB*), (IB1), (IB1*), (IB2) or (IB2)* according to the invention or of a composition according to the invention for the preparation of an antibacterial agent medicine.

The present invention also refer to the use of a compound of formula (I), (I*), (IA), (IA*), (IB), (IB*), (IB1), (IB1*), (IB2) or (IB2)* according to the invention or of a composition according to the invention for the preparation of an inhibitor of beta-lactamase medicine.

The present invention also refer to the use of a compound of formula (I), (I*), (IA), (IA*), (IB), (IB*), (IB1), (IB1*), (IB2) or (IB2)* according to the invention or of a composition according to the invention for the preparation of an antibacterial agent and inhibitor of beta-lactamase medicine.

The present invention also refer to a compound of formula (I), (I*), (IA), (IA*), (IB), (IB*), (IB1), (IB1*), (IB2) or (IB2)* or a composition according to the invention or a kit according to the invention for use for the treatment or prevention of bacterial infections.

The present invention also refer to the use of a compound of formula (I), (I*), (IA), (IA*), (IB), (IB*), (IB1), (IB1*), (IB2) or (IB2)* or a composition according to the invention for the preparation of a medicine for the treatment or prevention of bacterial infections.

The terms "prevention", "prevent" and "preventing" as used herein are intended to mean the administration of a compound or composition according to the invention in order to prevent infection by bacteria or to prevent occurrence of related infection and/or diseases. The terms "prevention", "prevent" and "preventing" also encompass the administration of a compound or composition according to the present invention in order preventing at least one bacterial infection, by administration to a patient susceptible to be infected, or otherwise at a risk of infection by this bacteria.

The terms "treatment", "treat" and "treating" as used herein are intended to mean in particular the administration of a treatment comprising a compound or composition according to the present invention to a patient already suffering from an infection. The terms "treatment", "treat" and "treating" as used herein, also refer to administering a compound or composition according to the present invention, optionally with one or more antibacterial agent, in order to:
- reduce or eliminate either a bacterial infection or one or more symptoms associated with bacterial infection, or
- retard the progression of a bacterial infection or of one or more symptoms associated with bacterial infection, or
- reduce the severity of a bacterial infection or of one or more symptoms associated with the bacterial infection, or
- suppress the clinical manifestation of a bacterial infection, or
- suppress the manifestation of adverse symptoms of the bacterial infection.

The expression "infection" or "bacterial infection" as used herein, includes the presence of bacteria, in or on a subject, which, if its growth were inhibited, would result in a benefit to the subject. As such, the term "infection" or "bacterial infection" in addition to referring to the presence of bacteria also refers to normal flora, which is not desirable. The term "infection" includes infection caused by bacteria. Exemplary of such bacterial infection are urinary tract infection (UTI), kidney infections (pyelonephritis), gynecological and obstetrical infections, respiratory tract indection (RTI), acute exacerbation of chronic bronchitis (AECB), Community-acquired pneumonia (CAP), hospital-acquired pneumonia (HAP), ventilator associated pneumonia (VAP), intra-abdominal pneumonia (IAI), acute otitis media, acute sinusitis, sepsis, catheter-related sepsis, chancroid, chlamydia, skin infections, bacteremia.

The term "growth" as used herein, refers to the growth of one or more microorganisms and includes reproduction or population expansion of the microorganism, such as bacteria. The term also includes maintenance of on-going metabolic processes of a microorganism, including processes that keep the microorganism alive.

The bacteria are chosen amongst gram-positive bacteria or gram-negative bacteria, preferably the gram-negative bacteria.

The bacteria can be also chosen among bacteria producing "beta-lactamase" or "β-lactamase". These bacteria are well known by the skilled person.

The term "beta-lactamase" or "β-lactamase" as used herein, refers to any enzyme or protein or any other substance that is able to break down a beta-lactam ring. The term "beta-lactamase" or "β-lactamase" includes enzymes that are produced by bacteria and that have the ability to hydrolyze, either partially or completely, the beta-lactam ring present in a compound such as an antibacterial agent.

Among the gram-positive bacteria, the bacteria according to the invention is preferably chosen among *Staphylococcus, Streptococcus, Staphylococcus species* (including *Staphylococcus aureus, Staphylococcus epidermidis*), *Streptococcus species* (including *Streptococcus pneumonia, Streptococcus agalactiae*), *Enterococcus species* (including *Enterococcus faecalis* and *Enterococcus faecium*).

Among the gram-negative bacteria, the bacteria according to the invention is preferably chosen among *Acinetobacter* species (including *Acinetobacter baumannii*)*, Citrobacter* species, *Escherichia* species (including *Escherichia coli*), *Haemophilus influenza, Morganella morganii*, *Klebsiella* species (including *Klebsiella pneumonia*), *Enterobacter* species (including *Enterobacter cloacae*), *Neisseria gonorrhoeae, Burkholderia* species (including *Burkholderia cepacia*), *Proteus* species (including *Proteus mirabilis*), *Serratia* species (including *Serratia marcescens*), Providencia species, *Pseudomonas aeruginosa.*

The invention thus preferably refers to a compound of formula (I), (I*), (IA), (IA*), (IB), (IB*), (IB1), (IB1*), (IB2) or (IB2)* or a composition according to the invention or a kit according to the invention for use for the treatment or prevention of bacterial infection, preferably caused by bacteria producing one or more beta-lactamase(s). Preferably, the bacteria are chosen amongst gram-positive bacteria or gram-negative bacteria, preferably gram-negative bacteria.

The present invention also refer to the use of a compound of formula (I), (I*), (IA), (IA*), (IB), (IB*), (IB1), (IB1*), (IB2) or (IB2)* or a composition according to the invention for the preparation of a medicine for the treatment or prevention of bacterial infection, preferably caused by bacteria producing one or more beta-lactamase(s). Preferably, the bacteria are chosen amongst gram-positive bacteria or gram-negative bacteria, preferably gram-negative bacteria.

The present invention also refers to the kit as defined above, for a simultaneous, separated or sequential administration to a patient in need thereof for use for the treatment or prevention of bacterial infections, preferably caused by bacteria producing one or more beta-lactamase(s). Preferably, the bacteria are chosen amongst gram-positive bacteria or gram-negative bacteria, preferably gram-negative bacteria.

The present invention also refers to compound of formula (I), (I*), (IA), (IA*), (IB), (IB*), (IB1), (IB1*), (IB2) or (IB2)* for use in combination with one or more further antibacterial agent, preferably at least one of the further antibacterial agent is a beta lactam, for the treatment or prevention of bacterial infections, preferably caused by bacteria producing one or more beta-lactamase(s). Preferably, the bacteria are chosen amongst gram-positive bacteria or gram-negative bacteria, preferably gram-negative bacteria. Wherein the compounds of formula (I) or (I*) and the further antibacterial agent are administered simultaneously, separately or sequentially.

The present invention also refers to the use of a compound of formula (I), (I*), (IA), (IA*), (IB), (IB*), (IB1), (IB1*), (IB2) or (IB2)* or a composition according to the invention or a kit according to the invention for the prevention or treatment of bacterial infections, preferably of bacterial infection, preferably caused by bacteria producing one or more beta-lactamase(s). Preferably, the bacteria are chosen amongst gram-positive bacteria or gram-negative bacteria, preferably gram-negative bacteria.

The present invention also relates to a method for the treatment or prevention of bacterial infections, preferably caused by bacteria producing one or more beta-lactamase(s) comprising the administration of a therapeutically effective amount of compound of formula (I), (I*), (IA), (IA*), (IB), (IB*), (IB1), (IB1*), (IB2) or (IB2)*, a composition according to the invention or a kit according to the invention to a patient in need thereof. Preferably, the bacteria are chosen amongst gram-positive bacteria or gram-negative bacteria, preferably gram-negative bacteria.

The term "patient" means a person or an animal at risk of being infected by bacteria or, a person or an animal being infected by bacteria, preferably by gram-positive and/or by gram-negative bacteria. As used herein, the term "patient" refers to a warm-blooded animal such as a mammal, preferably a human or a human child, who is afflicted with, or has the potential to be afflicted with one or more infections and conditions described herein. The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

The expression "therapeutically effective amount" or "pharmaceutically effective amount" as used herein, refer to an amount of a compound according to the invention, which when administered to a patient in need thereof, is sufficient to effect treatment for disease-states, conditions, or disorders for which the compound has utility. Such an amount would be sufficient to elicit the biological or medical response of a tissue system, or patient that is sought by a researcher or a clinician. The amount of a compound according to the invention which constitutes a "therapeutically effective amount" will vary, notably depending on the compound itself and its biological activity, the composition used for administration, the time of administration, the route of administration, the rate of excretion of the compound, the duration of the treatment, the type of disease-state or disorder being treated and its severity, drugs used in combination with or coincidentally with the compounds of the invention, and the age, body weight, general health, sex and diet of the patient. Such a "therapeutically effective amount" can be determined by one of ordinary skilled in the art having regard to its own knowledge, and this disclosure. Preferably, the compounds according to the invention are administered in an amount comprised between 0.1 to 30g per day.

The compounds according to the invention may be provided in an aqueous physiological buffer solution for parenteral administration.
The compounds of the present invention are also capable of being administered in unit dose forms, wherein the expression "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. Compounds provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via transdermal patches.
The compositions may conveniently be administered in unit dosage form and may be prepared by any of the methods well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.
Preferred formulations include pharmaceutical compositions in which a compound of the present invention is formulated for oral or parenteral administration.
For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolved, modified-release or sustained-release preparations and formulations, and wherein such sustained-release formulations are preferably bi-modal. Preferred tablets contain lactose, cornstarch, magnesium silicate, croscarmellose sodium, povidone, magnesium stearate, or talc in any combination. For oral administration, tablets, pills, powders, capsules, troches and the like can be coated or can comprise a compound or composition enables to neutralize the gastric acid o in order for the compounds according to the invention to pass through the stomach without any degradation.
Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes. Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.
The pharmaceutical composition according to the invention can also comprise any compound or excipient for sustain release of the active compounds.

The present invention also relates to process for the preparation of compounds of formula ((I), (I*), (IA), (IA*), (IB), (IB*), (IB1), (IB1*), (IB2) or (IB2)* as defined above.

### Preparation of the compounds and biological activity:

Abbreviations or symbols used herein include:
   - ACHN:: 1,1'-azobis(cyclohexanecarbonitrile)
   - ACN:: acetonitrile
   - AcOH:: acetic acid
   - Bn:: benzyl
   - Boc:: *tert*-butoxycarbonyl
   - Boc₂O:: *tert*-butoxycarbonyl anhydride
   - BocON:: [2-(*tert-*butoxycarbonyloxyimino)-2-phenylacetonitrile]
   - bs:: broad singlet
   - Burgess reagent:: methyl *N*-triethylammoniosulfonyl)carbamate
   - Cbz:: carboxybenzyl
   - CbzCl:: benzyl chloroformate
   - CFU:: colony-forming units
   - CLSI:: clinical laboratory standards institute
   - d:: doublet
   - DBU:: 1,8-diazabicyclo[5.4.0]undec-7-ene
   - DCM:: dichloromethane
   - DCE:: 1,2-dichloroethane
   - dd:: doublet of doublet
   - ddd :: doublet of doublet of doublet
   - ddt :: doublet of doublet of triplet
   - dq:: doublet of quartet
   - dt :: doublet of triplet
   - DTA:: di-*tert*-butylazodicarboxylate
   - DEAD:: diethyl azodicarboxylate
   - Dess-Martin periodinane:: 1,1,1-tris(acetyloxy)-1,1-dihydro-1,2-benziodoxol-3-(1*H*)-one
   - DIAD:: diisopropyl azodicarboxylate
   - DIPEA:: *N*,*N*-diisopropylethylamine
   - DMAP:: 4-dimethylaminopyridine
   - DMF:: *N*,*N*-dimethylformamide
   - DMSO:: dimethylsulfoxide
   - EtOAc:: ethyl acetate
   - Et₂O:: diethyl ether
   - h:: hours
   - HATU:: 1-[Bis(dimethylamino)methylene]-1*H-*1,2,3-triazolo[4,5-*b*]pyridinium-3-oxid hexafluorophosphate
   - *i*PrOH:: isopropanol
   - m :: multiplet
   - min:: minutes
   - MeOH:: methanol
   - MeONa:: sodium methoxide
   - MIC:: minimum inhibitory concentration
   - MS:: mass spectrometry
   - MsCl:: methanesulfonyl chloride
   - NBS:: *N-*bromosuccinimide
   - NMR:: nuclear magnetic resonance spectroscopy
   - Ns:: nosyl, nitrobenzenesulfonyl
   - OMs:: methanesulfonate
   - OTs:: toluenesulfonate
   - OTf:: trifluoromethanesulfonate
   - Pd(Ph₃)₄:: tetrakis(triphenylphosphine)palladium(0)
   - PG:: protective group
   - PhSH:: thiophenol
   - PMe₃:: trimethylphosphine
   - PPh₃:: triphenylphosphine
   - Ppm:: parts per million
   - q:: quartet
   - rt:: room temperature
   - s:: singlet
   - SEM:: [2-(trimethylsilyl)ethoxy]methyl
   - t:: triplet
   - td:: triplet of doublet
   - TBAF:: tetra-*n*-butylammonium fluoride
   - TBDMSOTf:: trifluoromethanesulfonic acid tert-butyldimethylsilyl ester
   - TBSOTf:: trimethylsilyl trifluoromethanesulfonate
   - tBuOK:: potassium *tert-*butoxide
   - TEA:: triethylamine
   - Tf:: trifluoromethanesulfonate
   - TFA:: trifluoroacetic acid
   - THF:: tetrahydrofuran
   - THP:: tetrahydropyranyl
   - TLC:: thin layer chromatography
   - TMSI:: lodotrimethylsilane
   - Tr:: trityl (triphenylmethyl)

The compounds of the present invention of formula (I), (I*), (IA), (IA*), (IB), (IB*), (IB1), (IB1*), (IB2) or (IB2)* can be prepared respectively by the following reaction schemes 1 to 4.

It should be understood that the processes of schemes 1 to 4 can be adapted for preparing further compounds according to the invention. Further processes for the preparation of compounds according to the invention can be derived from the processes of schemes 1 to 4. 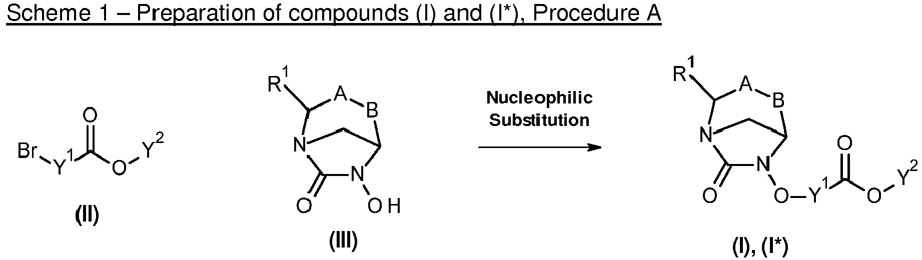 Nucleophilic Substitution could be performed by reaction of the appropriate ester (II) with compounds of formula (III) in a solvent such as DMSO, DMF, THF or ACN, preferably DMSO, in a presence of a base such as DBU, TEA, K₂CO₃ or Cs₂CO₃, preferably DBU and K₂CO₃. Y¹, Y², R¹ and A-B are described as above.

The preparation of compounds of formula (III) can be derived by the skilled person from WO2016156346 when A-B is CH₂-C(=NOR²) and from WO2016156597 and WO2016177862 when A-B is C(R³)=C(R⁴).

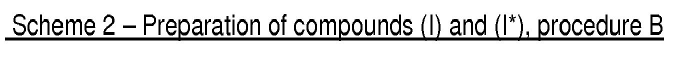

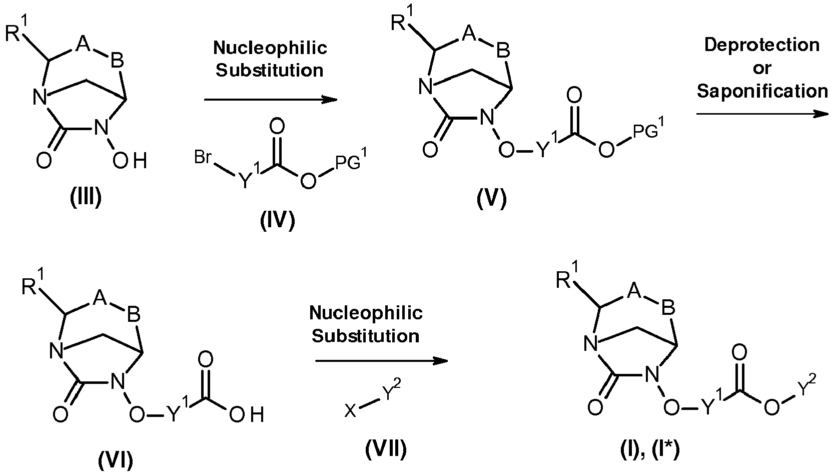

Compounds of formula (V) can be obtained from compounds of formula (III) by Nucleophilic Substitution with the appropriate ester (IV), wherein PG¹ is a protecting group such as ethyl, allyl or benzyl, in a solvent such as DMSO, DMF, THF or ACN, preferably DMSO and DMF, and in a presence of a base such as DBU, TEA, K₂CO₃ or Cs₂CO₃, preferably DBU and K₂CO₃.

Compounds of formula (VI) can be obtained from compounds of formula (V) by hydrogenolysis in a solvent such as THF, MeOH, EtOH, DCM, DMF, preferably THF, in a presence of a catalytic amount of Pd/C and in a presence or not of a base such as DIPEA or TEA, or by saponification in a solvent such as THF, H₂O MeOH, dioxane, preferably THF and H₂O in a presence of a base such as NaOH, LiOH or KOH, preferably LiOH.

Compounds of formula (I) and (I*) can be obtained from compounds of formula (VI) by Nucleophilic substitution with the appropriate compounds of formula (VII), wherein X is a leaving group such as Cl, Br, I, OTf, OMs or OTs, in a solvent such as DMSO, DMF, THF or ACN, preferably DMSO and DMF, and in a presence of a base such as DBU, TEA, K₂CO₃ or Cs₂CO₃, preferably DBU and K₂CO₃.

The preparation of compounds of formula (VI) can be derived by the skilled person from WO2016156346 when A-B is CH₂-C(=NOR²) and from WO2016156597 and WO2016177862 when A-B is C(R³)=C(R⁴).

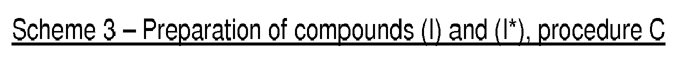

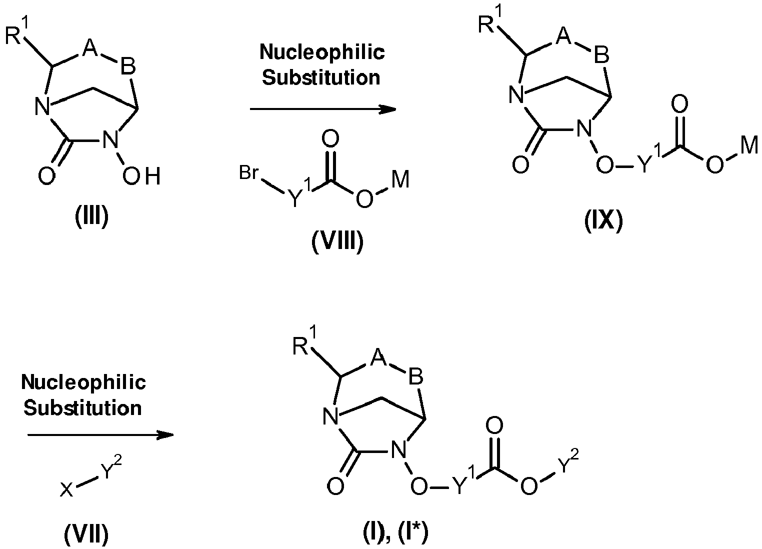

Compounds of formula (IX) can be obtained from compounds of formula (III) by Nucleophilic Substitution with the appropriate ester (VIII), wherein M is H, Li, Na or K, in a solvent such as DMSO, DMF, THF or ACN, preferably DMSO and DMF, and in a presence of a base such as DBU, TEA, K₂CO₃ or Cs₂CO₃, preferably DBU and K₂CO₃.

Compounds of formula (I) and (I*) can be obtained from compounds of formula (IX) by Nucleophilic substitution with compounds of formula (VII), wherein X is a leaving group such as Cl, Br, I, OTf, OMs or OTs, in a solvent such as DMSO, DMF, THF or ACN, preferably DMSO and DMF, and in a presence or not of a base such as DBU, TEA, K₂CO₃ or Cs₂CO₃, preferably DBU and K₂CO₃.

The preparation of compounds of formula (IX) can be derived by the skilled person from WO2016156346 when A-B is CH₂-C(=NOR²) and from WO2016156597 and WO2016177862 when A-B is C(R³)=C(R⁴).

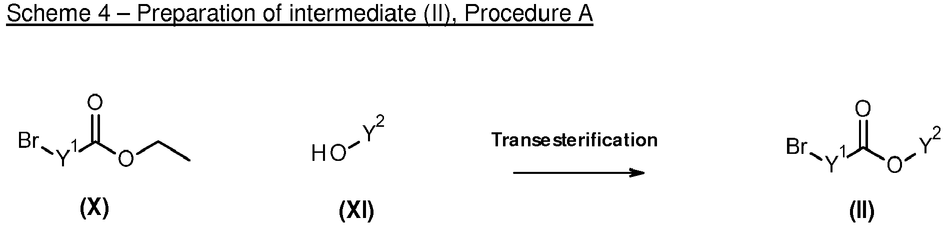

Transesterification could be performed by reaction of the appropriate ester (X) with appropriate alcohol (XI) neat or in a solvent such as Toluene or Dioxane, in a presence or not of a catalytic amount of acid such as MeSO₃H.

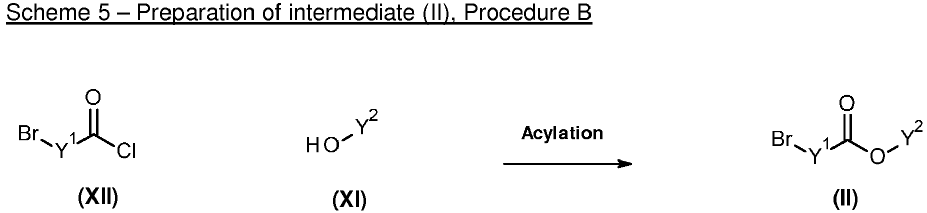

Acylation could be performed by reaction of the appropriate acyl chloride (XII) with appropriate alcohol (XI) in a solvent such as ACN or Et₂O, in a presence of a base such as pyridine or TEA.

### Examples

The following examples are provided for the purpose of illustrating the present invention and by no means should be interpreted to limit the scope of the present invention.

The first part represents the preparation of the compounds (intermediates and final compounds) whereas the second part describes the evaluation of antibacterial activity and biovailability of compounds according to the invention.

### Example 1: synthesis of [2,2-difluoro-2-[(4-isoxazol-4-yl-7-oxo-1,6-diazabicyclo[3.2.1]oct-3-en-6-yl)oxylacetyl]oxymethyl 2,2-dimethylpropanoate

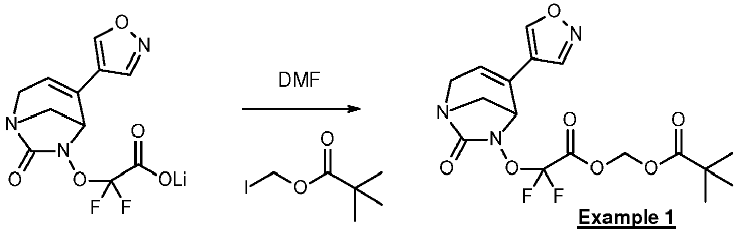

Lithium difluoro-(4-isoxazol-4-yl-7-oxo-1,6-diaza-bicyclo[3.2.1]oct-3-en-6-yloxy)-acetate (prepared according to the procedure described in WO2016177862 Example 6) (20 mg, 0.06 mmol) was solubilised in DMF (1 mL) with iodomethyl 2,2-dimethylpropanoate (16 mg, 0.06 mmol) and stirred for 1 h at rt. The reaction mixture was concentrated and the residue was purified by chromatography on silica gel (Cyclohexane to remove diiode then DCM/Et₂O 9/1) to provide [2,2-difluoro-2-[(4-isoxazol-4-yl-7-oxo-1,6-diazabicyclo[3.2.1]oct-3-en-6-yl)oxy]acetyl]oxymethyl 2,2-dimethylpropanoate (Example 1) (18 mg, 0.04 mmol, 67%) as a colourless oil.
MS *m*/*z*([M+H]⁺) 416
¹H NMR (300 MHz, CDCl₃): *δ*(ppm) 1.21 (s, 9H), 3.27 (dd, J = 11.4, 0.7 Hz, 1 H), 3.68 (dd, *J* = 18.7, 2.0, 1 H), 3.92 (dd, *J* = 18.8, 2.0 Hz, 1 H), 4.04 (dd, *J* = 18.8, 3.4 Hz, 1 H), 4.27-4.29 (m, 1H), 5.86 (d, *J* = 5.4 Hz, 1H), 5.92-5.95 (m, 2H), 8.35 (s, 1H), 8.50 (s, 1H).

### Example 2: synthesis of (5-methyl-2-oxo-1,3-dioxol-4-yl)methyl 2,2-difluoro-2-[(4-isoxazol-4-yl-7-oxo-1,6-diazabicyclo[3.2.1]oct-3-en-6-yl)oxy]acetate

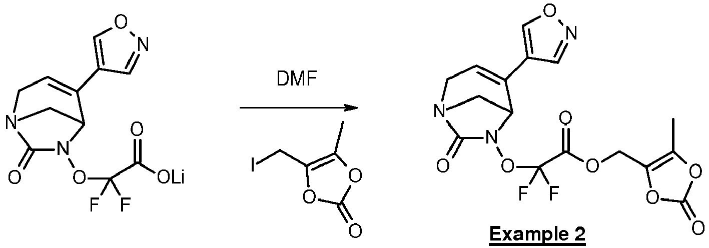

Lithium difluoro-(4-isoxazol-4-yl-7-oxo-1,6-diaza-bicyclo[3.2.1]oct-3-en-6-yloxy)-acetate (prepared according to the procedure described in WO2016177862 Example 6) (20 mg, 0.06 mmol) was dissolved in DMF (0,7 mL) with 4-lodomethyl-5-methyl-[1,3]dioxol-2-one (17 mg, 0.07 mmol) and stirred at rt for 1 h. The reaction mixture was concentrated and the residue was purified by chromatography on silica gel (DCM to DCM/Et₂O: 9/1) to provide (5-methyl-2-oxo-1,3-dioxol-4-yl)methyl 2,2-difluoro-2-[(4-isoxazol-4-yl-7-oxo-1,6-diazabicyclo[3.2.1]oct-3-en-6-yl)oxy]acetate (Example 2) (8.4 mg, 0.02 mmol, 32%) as a beige solid.
MS *m*/*z*([M+H]⁺) 414
¹H NMR (400 MHz, CDCl₃): *δ* (ppm) 2.19 (s, 3H), 3.30 (dd, *J* = 11.4, 0.7 Hz, 1H), 3.69 (dd, *J* = 11.4, 2.8 Hz, 1H), 3.94 (dd, *J* = 18.8, 2.1 Hz, 1H), 4.03 (dd, *J* = 18.8, 3.5 Hz, 1H), 4.27 (d, *J* = 2.8 Hz, 1H), 5.00 (d, *J* = 13.8 Hz, 1H), 5.05 (d, *J* = 13.8 Hz, 1H) 5.93-5.96 (m, 1H), 8.35 (s, 1H), 8.47 (s, 1H).

### Example 3: synthesis of 1-[2,2-difluoro-2-[(4-isoxazol-4-yl-7-oxo-1,6-diazabicyclo[3.2.1]oct-3-en-6-yl)oxylacetyl]oxyethyl 2,2-dimethylpropanoate

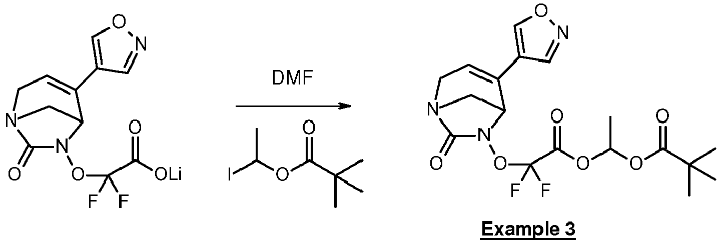

Lithium difluoro-(4-isoxazol-4-yl-7-oxo-1,6-diaza-bicyclo[3.2.1]oct-3-en-6-yloxy)-acetate (prepared according to the procedure described in WO2016177862 Example 6) (30 mg, 0.10 mmol) was solubilised in DMF (1 mL) with 1-iodoethyl 2,2-dimethylpropanoate (27 mg, 0.11 mmol) and stirred at rt for 1 h. The reaction mixture was concentrated and the residue was purified by chromatography on silica gel (DCM to DCM/Et₂O: 9/1) to provide 1-[2,2-difluoro-2-[(4-isoxazol-4-yl-7-oxo-1,6-diazabicyclo[3.2.1]oct-3-en-6-yl)oxy]acetyl]oxyethyl 2,2-dimethylpropanoate (Example 3) as mixture of both diastereoisomers (8.1 mg, 0.02 mmol, 25%).
MS *m*/*z*([M+H]⁺) 430
¹H NMR (400 MHz, CDCl₃): *δ* (ppm) 1.19 (s, 9H), 1.52 and 1.58 (d, *J* = 5.5 Hz, 3H), 3.25 and 3.28 (d, *J* = 11.2 Hz, 1H), 3.66-3.73 (m, 1H), 3.88-4.07 (m, 2H), 4.27 and 4.29 (d, *J* = 2.8 Hz, 1H), 5.89-5.97 (m, 1H), 6.90 and 6.94 (q, *J* = 5.5 Hz, 1H), 8.36 and 8.37 (s, 1H), 8.49 and 8.56 (s, 1H).

### Example 4: synthesis of cyclohexyl 2,2-difluoro-2-[(3-oxazol-2-yl-7-oxo-1,6-diazabicyclo[3.2.1]oct-3-en-6-yl)oxy]acetate

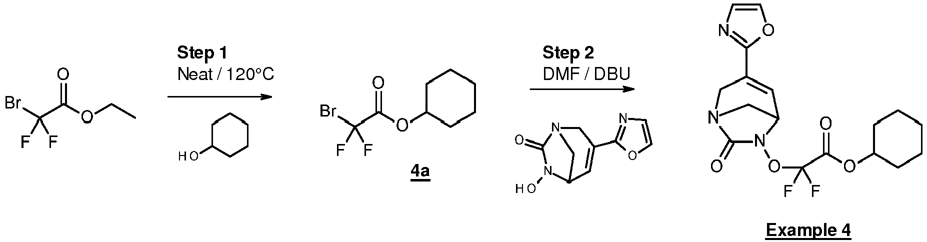

### Step 1: preparation of intermediate cyclohexyl 2-bromo-2,2-difluoro-acetate (4a)

In a sealed vial, a solution of ethyl 2-bromo-2,2-difluoro-acetate (2 mL, 15.6 mmol) and cyclohexanol (1.56 g, 15.6 mmol) was heated at 120°C for 65 h. The reaction mixture was slightly concentrated. The crude was purified by chromatography on silica gel (Heptane/DCM 100/0 to 50/50) to afford cyclohexyl 2-bromo-2,2-difluoro-acetate (4a)
(1.03 g, 5.06 mmol, 32%).
¹H NMR (300 MHz, CDCl₃): *δ* (ppm) 1.30-1.46 (m, 3H), 1.51-1.65 (m, 3H), 1.74-1.82 (m, 2H), 1.88-1.93 (m, 2H), 4.97 (tt, J = 3.8, 8.5 Hz, 1H).

### Step 2: preparation of cyclohexyl 2,2-difluoro-2-[(3-oxazol-2-yl-7-oxo-1,6-diazabicyclo[3.2.1]oct-3-en-6-yl)oxy]acetate, Example 4

To a solution of 6-hydroxy-3-oxazol-2-yl-1,6-diaza-bicyclo[3.2.1]oct-3-en-7-one (prepared according to the procedure described in WO2016177862 compound 37f) (0.08 g, 0.386 mmol) in DMF (4 mL) were added DBU (0.063 mL, 0.430 mmol) and cyclohexyl bromodifluoroacetate (4a) (0.258 g, 1.00 mmol). The mixture was stirred at -20°C for 30 min. The reaction mixture was diluted with diisopropyl Ether and the insolubles were removed by filtration. The filtrate was washed with water, dried over Na₂SO₄ and concentrated *in vacuo.* The residue was purified by chromatography on silica gel (DCM/acetone: 100/0 to 90/10) to provide cyclohexyl difluoro-(3-oxazol-2-yl-7-oxo-1,6-diaza-bicyclo[3.2.1]oct-3-en-6-yloxy)-acetate (Example 4) (0.97 g, 0.253 mmol, 65%).
MS *m*/*z*([M+H]⁺) 384
¹H NMR (400 MHz, CDCl₃) δ (ppm) : 1.24-1.43 (m, 3H), 1.51-1.61 (m, 3H), 1.72-1.81 (m, 2H), 1.87-1.95 (m, 2H), 3.22 (d, *J* = 11.2 Hz, 1H), 3.66 (d, *J* = 11.2 Hz, 1H), 4.17 (dd, *J* = 2.1, 18.0 Hz, 1H), 4.27 (dd, *J* = 2.5, 5.2 Hz, 1H), 4.44 (dd, *J* = 1.3, 18.0 Hz, 1H), 4.90-4.97 (m, 1H), 7.08-7.11 (m, 1H), 7.15 (s, 1H), 7.62 (s, 1H).

### Example 5: synthesis of cyclohexyl cyclohexyl 2,2-difluoro-2-[(7-oxo-3-pyrazol-1-yl-1,6-diazabicyclo[3.2.1]oct-3-en-6-yl)oxy]acetate

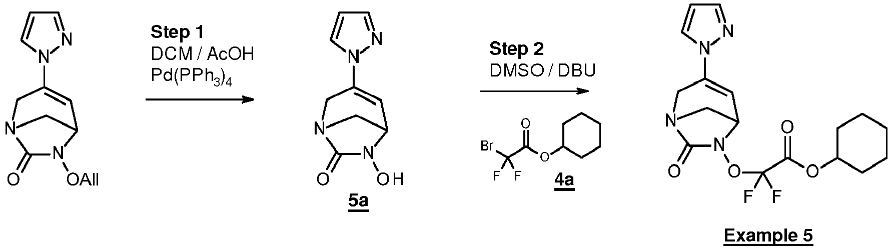

### Step 1: preparation of intermediate 6-hydroxy-3-pyrazol-1-yl-1,6-diaza-bicyclo[3.2.1]oct-3-en-7-one (5a)

A solution of 6-allyloxy-3-pyrazol-1-yl-1,6-diaza-bicyclo[3.2.1]oct-3-en-7-one (prepared according to the procedure described in WO2016177862 compound 47a) (0.100 g, 0.41 mmol) in anhydrous DCM (4 mL) was degassed for 10 min under argon atmosphere. AcOH (0.047 mL, 0.81 mmol) and Pd(PPh₃)₄ (0.237 g, 0.205 mmol) were successively added. After stirring for 30 min at rt, the precipitate was filtered off and washed with DCM to afford 0.05 mg of white solid. The filtrate was concentrated *in vacuo* and purified by preparative TLC on silica gel (DCM/acetone 6/4) to give additional 0.013 g. Both solids were mixed to give 6-hydroxy-3-pyrazol-1-yl-1,6-diaza-bicyclo[3.2.1]oct-3-en-7-one (5a) (0.063 g, 0.31 mmol, 75%).
MS *m*/*z*([M+H]⁺) 207
¹H NMR (400 MHz, DMSO-*d₆*): *δ* (ppm) 3.22 (d, *J* = 10.7 Hz, 1H), 3.36 (dd, J = 2.5, 10.8 Hz, 1H), 4.02 (dd, *J* = 2.5, 5.6 Hz, 1H), 4.18 (s, 2H), 6.45 (dd, J = 2.0, 2.4 Hz, 1 *H*), 6.65 (d*, J* = 5.2 Hz, 1H), 7.64 (d, *J* = 1.5 Hz, 1 H), 8.18 (d, *J* = 2.4 Hz, 1H), 9.65 (s, 1H).

### Step 2: preparation of cyclohexyl 2,2-difluoro-2-[(7-oxo-3-pyrazol-1-yl-1,6-diazabicyclo[3.2.1]oct-3 -en-6-yl)oxy]acetate, Example 5

To a solution of 6-hydroxy-3-pyrazol-1-yl-1,6-diaza-bicyclo[3.2.1]oct-3-en-7-one (5a) (0.40 g, 0.194 mmol) in DMSO (1.9 mL) were added DBU (0.032 mL, 0.213 mmol) and cyclohexyl bromodifluoroacetate (0.130 mL, 0.504 mmol). The mixture was stirred for 30 min at rt then poured in a 2M NaH₂PO₄ solution. The product was extracted with ethyl acetate. The organic layer was filtered on a pad of silica and concentrated *in vacuo.* The residue was purified on silica gel (DCM/acetone: 10/0 to 9/1) to provide cyclohexyl difluoro-(7-oxo-3-pyrazol-1-yl-1,6-diaza-bicyclo[3.2.1]oct-3-en-6-yloxy)-acetate (Example 5) (0.064 g, 0.167 mmol, 86%).
MS *m*/*z*([M+H]⁺) 383
¹H NMR (400 MHz, CDCl₃): *δ* (ppm) 1.25-1.43 (m, 3H), 1.52-1.62 (m, 3H), 1.75-1.82 (m, 2H), 1.87-1.95 (m, 2H), 3.23 (d, *J* = 11.1 Hz, 1H), 3.64 (dd, *J* = 1.7, 11.2 Hz, 1H), 4.25-4.33 (m, 2H), 4.58 (d, *J* = 17.7 Hz, 1H), 4.88-4.99 (m, 1H), 6.38 (dd, J = 2.6, 1.8 Hz, 1H), 6.39-6.43 (m, 1H), 7.60 (d, *J* = 1.6 Hz, 1H), 7.64 (d, *J* = 2.6 Hz, 1H).

### Example 6: synthesis of n-Cetyl 2,2-difluoro-2-[(3-oxazol-2-yl-7-oxo-1,6-diazabicyclo[3.2.1]oct-3-en-6-yl)oxy]-acetate

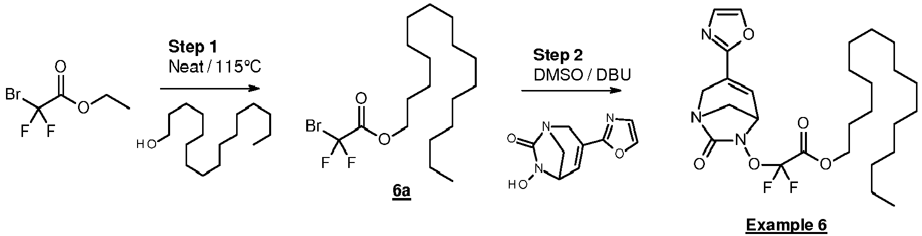

### Step 1: preparation of intermediate n-cetyl 2-bromo-2,2-difluoro-acetate (6a)

In a sealed vial, a solution of ethyl 2-bromo-2,2-difluoro-acetate (300 µL, 2.34 mmol) and n-Cetyl alcohol (200 mg, 0.82 mmol) was heated at 115°C for 2.5 hours. The middle was slightly concentrated. The crude was purified by chromatography on silica gel (Heptane/DCM 100/0 to 70/30) to afford n-cetyl 2-bromo-2,2-difluoro-acetate (6a) (155 mg, 0.388 mmol, 47%).
¹H NMR (300 MHz, CDCl₃): *δ* (ppm) 0.86-0.90 (m, 3H), 1.20-1.41 (m, 26H), 1.70-1.79 (m, 2H), 4.35 (t, *J* = 6.6 Hz, 2H).

### Step 2: preparation of n-Cetyl 2,2-difluoro-2-[(3-oxazol-2-yl-7-oxo-1,6-diazabicyclo[3.2.1]oct-3-en-6-yl)oxyl-acetate, Example 6

At room temperature, a solution of DBU (61 µL, 0.41 mmol) in DMSO (500 µL) was slowly added to a solution of 6-Hydroxy-3-oxazol-2-yl-1,6-diazabicyclo[3.2.1]oct-3-en-7-one (prepared according to the procedure described in WO2016177862 compound 37f) (85 mg, 0.41 mmol) and n-Cetyl 2-bromo-2,2-difluoro-acetate (6a) (245 mg, 0.61 mmol) in a mixture of DMSO (2 mL) and THF (2 mL). The middle was stirred at rt for 2.5 hours and then diluted with AcOEt. The organic layer was washed with brine, dried over sodium sulfate, filtered and concentrated. The residue was purified by chromatography on silica gel (DCM/Acetone 95/5) to provide n-cetyl 2,2-difluoro-2-[(3-oxazol-2-yl-7-oxo-1,6-diazabicyclo[3.2.1]oct-3-en-6-yl)oxy]-acetate (Example 6) (71 mg, 0.135 mmol, 33%).
MS *m*/*z*([M+H]⁺) 526.
¹H NMR (300 MHz, CDCl₃): *δ* (ppm) 0.86-0.90 (m, 3H), 1.20-1.41 (m, 26H), 1.67-1.78 (m, 2H), 3.23 (d, J = 11.1 Hz, 1H), 3.62-3.70 (m, 1H), 4.17 (dd, J = 2.2, 18.1 Hz, 1 H), 4.26-4.33 (m, 3H), 4.45 (dd, J = 1.4, 18.1 Hz, 1H), 7.08-7.11 (m, 1H), 7.15 (d, J = 0.8 Hz, 1H), 7.61 (d, J = 0.8 Hz, 1H).

### Example 7: Synthesis of n-Hexyl 2,2-difluoro-2-[(3-oxazol-2-yl-7-oxo-1,6-diazabicyclo[3.2.1]oct-3-en-6-yl)oxy]-acetate

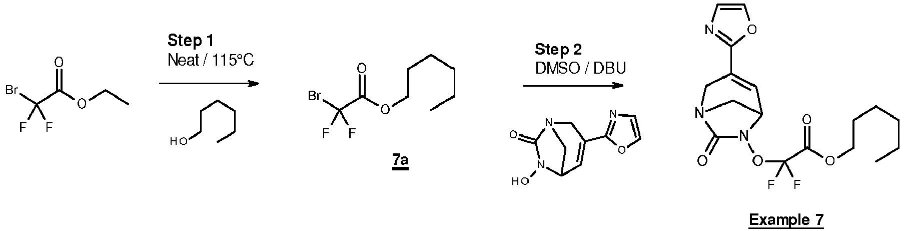

### Step 1: preparation of intermediate n-Hexyl 2-bromo-2,2-difluoro-acetate (7a)

In a sealed vial, a solution of ethyl 2-bromo-2,2-difluoro-acetate (1 mL, 7.8 mmol) and 1-hexanol (980 mg, 7.8 mmol) was heated at 115°C for 2.5 hours. The middle was slightly concentrated. The crude was purified by chromatography on silica gel (Heptane/DCM 100/0 to 70/30) to afford n-Hexyl 2-bromo-2,2-difluoro-acetate (6a) (215 mg, 0.83 mmol, 11%).
¹H NMR (300 MHz, CDCl₃): *δ* (ppm) 0.88-0.92 (m, 3H), 1.29-1.45 (m, 6H), 1.70-1.79 (m, 2H), 4.35 (t, *J* = 6.6 Hz, 2H).

### Step 2: preparation of n-Hexyl 2,2-difluoro-2-[(3-oxazol-2-yl-7-oxo-1,6-diazabicyclo[3.2.1]oct-3-en-6-yl)oxy]-acetate, Example 7

At room temperature, a solution of DBU (72 µL, 0.41 mmol) in DMSO (250 µL) was slowly added to a solution of 6-Hydroxy-3-oxazol-2-yl-1,6-diazabicyclo[3.2.1]oct-3-en-7-one (prepared according to the procedure described in WO2016177862 compound 37f) (100 mg, 0.48 mmol) and n-Hexyl 2-bromo-2,2-difluoro-acetate (7a) (215 mg, 0.83 mmol) in DMSO (2 mL). The middle was stirred at room temperature for 10 minutes and then diluted with AcOEt. The organic layer was washed with brine, dried over sodium sulfate, filtered and concentrated. The residue was purified by chromatography on silica gel (DCM/Acetone 95/5) to provide n-Hexyl 2,2-difluoro-2-[(3-oxazol-2-yl-7-oxo-1,6-diazabicyclo[3.2.1]oct-3-en-6-yl)oxy]-acetate (Example 7) (100 mg, 0.259 mmol, 54%).
MS *m*/*z*([M+H]⁺) 386.
¹H NMR (300 MHz, CDCl₃): *δ* (ppm) 0.86-0.90 (m, 3H), 1.25-1.42 (m, 6H), 1.73 (pent, *J* = 6.9 Hz, 2H), 3.23 (d, *J* = 11.2 Hz, 1H), 3.66-3.71 (m, 1H), 4.17 (dd, *J* = 2.1, 18.1 Hz, 1H), 4.24-4.35 (m, 3H), 4.45 (dd, *J* = 1.4, 18.1 Hz, 1 *H*), 7.08-7.10 (m, 1H), 7.15 (d, *J* = 0.8 Hz, 1 H), 7.62 (d, *J* = 0.8 Hz, 1H).

### Example 8: Synthesis of 2-Adamantyl 2,2-difluoro-2-[(3-oxazol-2-yl-7-oxo-1,6-diazabicyclo[3.2.1]oct-3-en-6-yl)oxyl-acetate

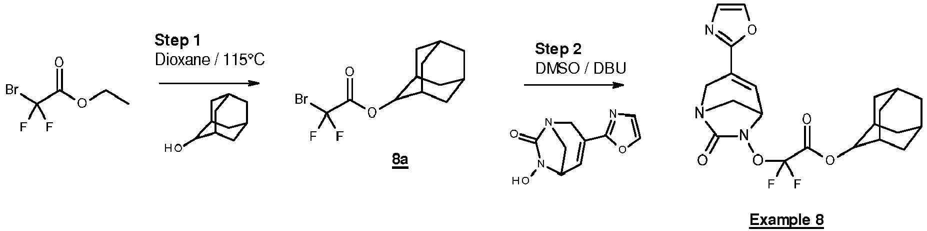

### Step 1: preparation of intermediate 2-Adamantyl 2-bromo-2,2-difluoro-acetate (8a)

In a sealed vial, a solution of ethyl 2-bromo-2,2-difluoro-acetate (1 mL, 7.8 mmol) and 2-Adamantanol (1 g, 6.57 mmol) in Dioxane (12 mL) was heated at 115°C for 16 hours. The middle was slightly concentrated and triturated with Cyclohexane to remove excess of 2-Adamantanol. The crude was purified by chromatography on silica gel (Heptane/DCM 100/0 to 50/50) to afford 2-Adamantyl 2-bromo-2,2-difluoro-acetate (8a) (210 mg, 0.68 mmol, 10%).
¹H NMR (300 MHz, CDCl₃): *δ* (ppm) 1.58-1.67 (m, 2H), 1.73-1.84 (m, 4H), 1.85-1.96 (m, 4H), 2.01-2.17 (m, 4H), 5.10-5.13 (m, 1H).

### Step 2: preparation of 2-Adamantyl 2,2-difluoro-2-[(3-oxazol-2-yl-7-oxo-1,6-diazabicyclo[3.2.1]oct-3-en-6-yl)oxy]-acetate, Example 8

At room temperature, a solution of DBU (72 µL, 0.48 mmol) in DMSO (250 µL) was slowly added to a solution of 6-Hydroxy-3-oxazol-2-yl-1,6-diazabicyclo[3.2.1]oct-3-en-7-one (prepared according to the procedure described in WO2016177862 compound 37f) (100 mg, 0.48 mmol) and 2-Adamantyl 2-bromo-2,2-difluoro-acetate (8a) (210 mg, 0.68 mmol) in DMSO (2 mL). The middle was stirred at room temperature for 35 minutes and then diluted with AcOEt. The organic layer was washed with brine, dried over sodium sulfate, filtered and concentrated. The residue was purified by chromatography on silica gel (DCM/Acetone 95/5) to provide 2-Adamantyl 2,2-difluoro-2-[(3-oxazol-2-yl-7-oxo-1,6-diazabicyclo[3.2.1]oct-3-en-6-yl)oxy]-acetate (Example 8) (116 mg, 0.266 mmol, 55%).
MS *m*/*z*([M+H]⁺) 436.
¹H NMR (300 MHz, CDCl₃): *δ* (ppm) 1.51-1.64 (m, 4H), 1.69-1.94 (m, 6H), 2.00-2.14 (m, 4H), 3.22 (d, *J* = 11.2 Hz, 1H), 3.64-3.69 (m, 1H), 4.17 (dd, *J* = 2.2, 18.1 Hz, 1H), 4.28 (dd, *J* = 2.5, 5.3 Hz, 1H), 4.45 (dd, *J* = 1.4, 18.1 Hz, 1H), 5.08-5.13 (m, 1H), 7.09-7.11 (m, 1H), 7.15 (d, *J* = 0.8 Hz, 1H), 7.62 (d, *J* = 0.8 Hz, 1H).

### Example 9: Synthesis of Benzyl 2,2-difluoro-2-[(3-oxazol-2-yl-7-oxo-1,6-diazabicyclo[3.2.1]oct-3-en-6-yl)oxy]-acetate

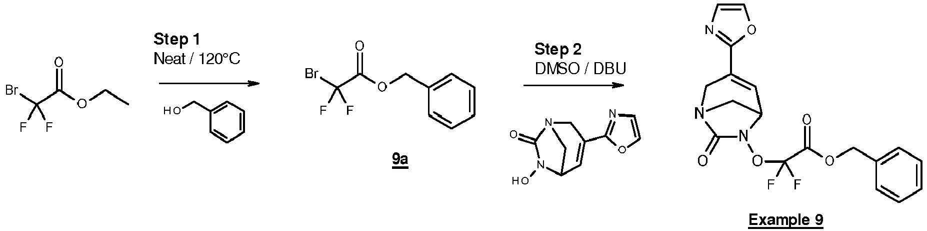

### Step 1: preparation of intermediate Benzyl 2-bromo-2,2-difluoro-acetate (9a)

In a sealed vial, a solution of ethyl 2-bromo-2,2-difluoro-acetate (1 mL, 7.8 mmol) and Benzyl alcohol (800 µL, 7.8 mmol) was heated at 120°C for 20 hours. The middle was slightly concentrated. The crude was purified by chromatography on silica gel (Heptane/DCM 100/0 to 50/50) to afford Benzyl 2-bromo-2,2-difluoro-acetate (9a) (590 mg, 2.22 mmol, 28%).
¹H NMR (300 MHz, CDCl₃): *δ* (ppm) 5.36 (s, 2H), 7.41 (s, 5H).

### Step 2: preparation of Benzyl 2,2-difluoro-2-[(3-oxazol-2-yl-7-oxo-1,6-diazabicyclo[3.2.1]oct-3-en-6-yl)oxy]-acetate, Example 9

At room temperature, a solution of DBU (72 µL, 0.48 mmol) in DMSO (250 µL) was slowly added to a solution of 6-Hydroxy-3-oxazol-2-yl-1,6-diazabicyclo[3.2.1]oct-3-en-7-one (prepared according to the procedure described in WO2016177862 compound 37f) (100 mg, 0.48 mmol) and Benzyl 2-bromo-2,2-difluoro-acetate (9a) (250 mg, 0.94 mmol) in DMSO (2 mL). The middle was stirred at room temperature for 35 minutes and then diluted with AcOEt. The organic layer was washed with brine, dried over sodium sulfate, filtered and concentrated. The residue was purified by chromatography on silica gel (DCM/Acetone 95/5) to provide Benzyl 2,2-difluoro-2-[(3-oxazol-2-yl-7-oxo-1,6-diazabicyclo[3.2.1]oct-3-en-6-yl)oxy]-acetate (Example 9) (142 mg, 0.363 mmol, 76%).
MS *m*/*z*([M+H]⁺) 392.
¹H NMR (300 MHz, CDCl₃): *δ* (ppm) 3.15 (d, J = 11.1 Hz, 1H), 3.48-3.53 (m, 1H), 4.14 (dd, J = 2.4, 12.9 Hz, 1H), 4.17 (d, J = 2.2 Hz, 1H), 4.43 (dd, J = 1.3, 18.0 Hz, 1H), 5.33 (s, 2H), 7.04-7.07 (m, 1H), 7.15 (d, J = 0.8 Hz, 1H), 7.37-7.42 (m, 5H), 7.61 (d, J = 0.8 Hz, 1H).

### Example 10: Synthesis of 4-Heptanyl 2,2-difluoro-2-[(3-oxazol-2-yl-7-oxo-1,6-diazabicyclo[3.2.1]oct-3-en-6-yl)oxy]-acetate

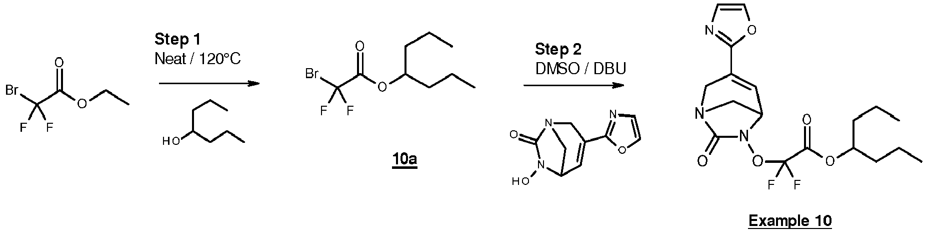

### Step 1: preparation of intermediate 4-Heptanyl 2-bromo-2,2-difluoro-acetate (10a)

In a sealed vial, a solution of ethyl 2-bromo-2,2-difluoro-acetate (1 mL, 7.8 mmol) and 4-Heptanol (906 mg, 7.8 mmol) was heated at 120°C for 60 hours. The middle was slightly concentrated. The crude was purified by chromatography on silica gel (Heptane/DCM 100/0 to 50/50) to afford 4-Heptanyl 2-bromo-2,2-difluoro-acetate (10a) (510 mg, 1.86 mmol, 24%).
¹H NMR (300 MHz, CDCl₃): *δ* (ppm) 0.93 (t, J = 7.3 Hz, 6H), 1.28-1.47 (m, 4H), 1.54-1.75 (m, 4H), 5.07 (tt, J = 4.9, 7.7 Hz, 1H).

### Step 2: preparation of 4-Heptanyl 2,2-difluoro-2-[(3-oxazol-2-yl-7-oxo-1,6-diazabicyclo[3.2.1]oct-3-en-6-yl)oxy]-acetate, Example 10

At room temperature, a solution of DBU (72 µL, 0.48 mmol) in DMSO (250 µL) was slowly added to a solution of 6-Hydroxy-3-oxazol-2-yl-1,6-diazabicyclo[3.2.1]oct-3-en-7-one (prepared according to the procedure described in WO2016177862 compound 37f) (100 mg, 0.48 mmol) and 4-Heptanyl 2-bromo-2,2-difluoro-acetate (10a) (262 mg, 0.96 mmol) in DMSO (2 mL). The middle was stirred at room temperature for 35 minutes and then diluted with AcOEt. The organic layer was washed with brine, dried over sodium sulfate, filtered and concentrated. The residue was purified by chromatography on silica gel (DCM/Acetone 95/5) to provide 4-Heptanyl 2,2-difluoro-2-[(3-oxazol-2-yl-7-oxo-1,6-diazabicyclo[3.2.1]oct-3-en-6-yl)oxy]-acetate (Example 10) (120 mg, 0.30 mmol, 62%).
MS *m*/*z*([M+H]⁺) 400.
¹H NMR (300 MHz, CDCl₃): *δ* (ppm) 0.91 (td, J = 0.9, 7.3 Hz, 6H), 1.24-1.43 (m, 4H), 1.43-1.72 (m, 4H), 3.22 (d, J = 11.3 Hz, 1H), 3.63-3.68 (m, 1H), 4.17 (dd, J = 2.1, 18.1 Hz, 1H), 4.28 (dd, J = 2.5, 5.3 Hz, 1H), 4.45 (dd, J = 1.3, 18.0 Hz, 1H), 5.02-5.10 (m, 1H), 7.08-7.10 (m, 1H), 7.15 (d, J = 0.8 Hz, 1H), 7.62 (d, J = 0.8 Hz, 1H).

### Example 11: Synthesis of cycloheptyl 2,2-difluoro-2-[(3-oxazol-2-yl-7-oxo-1,6-diazabicyclo[3.2.1]oct-3-en-6-yl)oxy]acetate

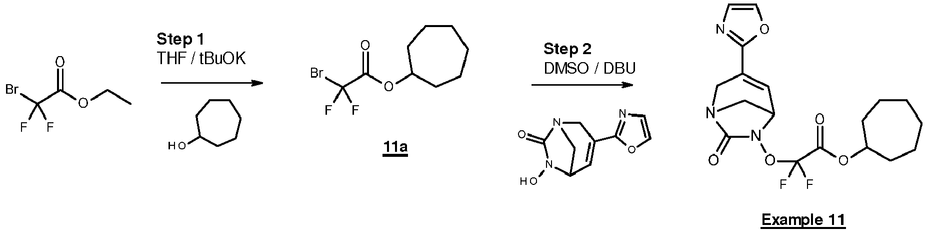

### Step 1: preparation of intermediate cycloheptyl 2-bromo-2,2-difluoro-acetate (11a)

To a solution of ethyl 2-bromo-2,2-difluoro-acetate (1 g, 4.93 mmol) and cycloheptanol (2.8 g, 24.6 mmol) in THF (5 mL) under inert atmosphere at 0°C, was added *t*-BuOK (1M in THF, 2.46 mL). After stirring at room temperature for 16 hours, the mixture was quenched with a 1 N HCl solution, extracted with AcOEt, dried over Na₂SO₄ and filtered. The solvent was removed *in vacuo.* The crude was purified by chromatography on silica gel (Heptane/DCM 10/0 to 7/3) to afford cycloheptyl 2-bromo-2,2-difluoro-acetate (11 a)
(339 mg, 1.25 mmol, 26%).
¹H NMR (400 MHz, CDCl₃): *δ* (ppm) 1.44-1.53 (m, 2H), 1.58-1.61 (m, 4H), 1.67-1.76 (m, 2H), 1.77-1.85 (m, 2H), 1.94-2.02 (m, 2H), 5.09-5.16 (m, 1H).

### Step 2: preparation of cycloheptyl 2,2-difluoro-2-[(3-oxazol-2-yl-7-oxo-1,6-diazabicyclo[3.2.1]oct-3-en-6-yl)oxy]acetate, Example 11

At room temperature, a solution of DBU (61.4 µL, 0.41 mmol) in DMSO (207 µL) was slowly added to a solution of 6-Hydroxy-3-oxazol-2-yl-1,6-diazabicyclo[3.2.1]oct-3-en-7-one (prepared according to the procedure described in WO2016177862 compound 37f) (85 mg, 0.41 mmol) and cycloheptyl 2-bromo-2,2-difluoro-acetate (11a) (133 mg, 0.49 mmol) in DMSO (5 mL). The mixture was stirred at room temperature for 30 minutes then diluted with AcOEt. The organic layer was washed with brine, dried over Na₂SO₄, filtered and concentrated *in vacuo.* The residue was purified by chromatography on silica gel (DCM/Acetone 9/1) to provide cycloheptyl 2,2-difluoro-2-[(3-oxazol-2-yl-7-oxo-1,6-diazabicyclo[3.2.1]oct-3-en-6-yl)oxy]acetate (Example 11) as a white solid (78 mg, 0.20 mmol, 48%).
MS *mlz* ([M+H]⁺) 398.
¹H NMR (400 MHz, acetone-*d₆*): *δ* (ppm) 1.47-1.61 (m, 6H), 1.66-1.84 (m, 4H), 1.95-2.02 (m, 2H), 3.49 (dd, *J*= 11.4, 0.8 Hz, 1H), 3.60-3.64 (m, 1H), 4.25 (d, *J*= 1.8 Hz, 2H), 4.42 (dd, *J*= 5.2, 2.5 Hz, 1H), 5.09-5.16 (m, 1H), 7.10-7.12 (m, 1H), 7.24 (d, *J* = 0.8 Hz, 1H), 7.97 (d, *J* = 0.8 Hz, 1H).

### Example 12: Synthesis of indan-2-yl 2,2-difluoro-2-[(3-oxazol-2-yl-7-oxo-1,6-diazabicyclo[3.2.1]oct-3-en-6-yl)oxy]acetate

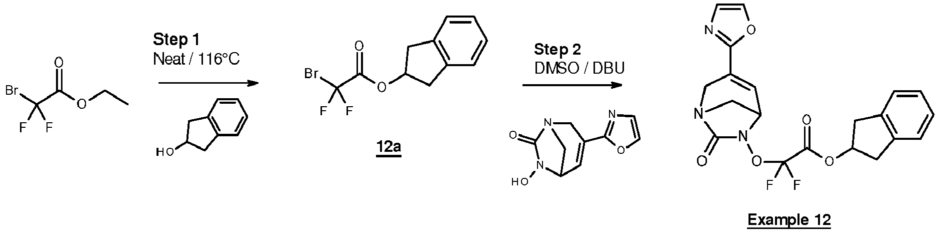

### Step 1: preparation of intermediate indan-2-yl 2-bromo-2,2-difluoro-acetate (12a)

In a sealed vial, a solution of ethyl 2-bromo-2,2-difluoro-acetate (1.5 g, 7.39 mmol) and 2-Indanol (992 mg, 7.39 mmol) was heated at 110°C for 16 hours. The mixture was concentrated *in vacuo.* The crude was purified by chromatography on silica gel (Heptane/DCM 100/0 to 50/50) to afford indan-2-yl 2-bromo-2,2-difluoro-acetate (12a)
(318 mg, 1.09 mmol, 15%).
¹H NMR (400 MHz, acetone-d₆): *δ* (ppm) 3.14 (dd, *J* = 17.5, 2.2 Hz, 2H), 3.47 (dd, *J*= 17.5, 6.1 Hz, 2H), 5.78-5.83 (m, 1H), 7.19-7.23 (m, 2H), 7.27-7.32 (m, 2H).

### Step 2: preparation of indan-2-yl 2,2-difluoro-2-[(3-oxazol-2-yl-7-oxo-1,6-diazabicyclo[3.2.1]oct-3-en-6-yl)oxy]acetate, Example 12

At room temperature, a solution of DBU (79.4 µL, 0.53 mmol) in DMSO (268 µL) was slowly added to a solution of 6-Hydroxy-3-oxazol-2-yl-1,6-diazabicyclo[3.2.1]oct-3-en-7-one (prepared according to the procedure described in WO2016177862 compound 37f) (110 mg, 0.53 mmol) and indan-2-yl 2-bromo-2,2-difluoro-acetate (12a) (186 mg, 0.64 mmol) in DMSO (5.8 mL). The mixture was stirred at room temperature for 30 minutes and then diluted with AcOEt. The organic layer was washed with brine, dried over sodium sulfate, filtered and concentrated. The residue was purified by chromatography on silica gel (DCM/AcOEt 8/2) to provide indan-2-yl 2,2-difluoro-2-[(3-oxazol-2-yl-7-oxo-1,6-diazabicyclo[3.2.1]oct-3-en-6-yl)oxy]acetate (Example 12) (108 mg, 0.26 mmol, 13%).
MS *m*/*z* ([M+H]⁺ 418).
¹H NMR (300 MHz, acetone-*d₆*): *δ* (ppm) 3.13 (dd, *J*= 17.4, 2.3 Hz, 2H), 3.39-3.49 (m, 3H), 3.55 (ddd, *J* = 11.5, 2.6, 1.3 Hz, 1H), 4.22-4.25 (m, 3H), 5.72-5.79 (m, 1 *H*), 6.99-7.03 (m, 1H), 7.18-7.22 (m, 2H), 7.23 (d, *J* = 0.8 Hz, 1H), 7.28-7.31 (m, 2H), 7.96 (d, *J*= 0.8 Hz, 1H).

### Example 13: Synthesis of (2,2,6,6-tetramethyltetrahydropyran-4-yl) 2,2-difluoro-2-[(3-oxazol-2-yl-7-oxo-1,6-diazabicyclo[3.2.1]oct-3-en-6-yl)oxy]acetate

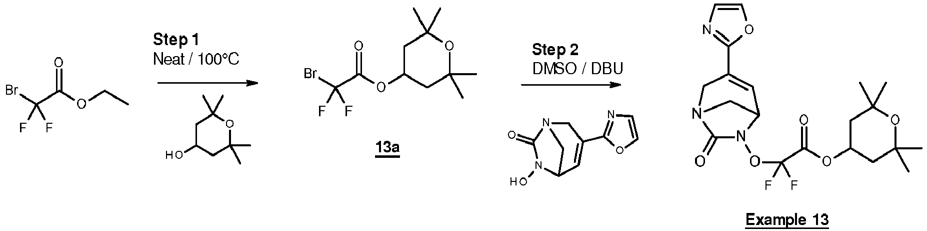

### Step 1: preparation of intermediate (2,2,6,6-tetramethyltetrahydropyran-4-yl) 2-bromo-2,2-difluoro-acetate (13a)

In a sealed vial, a solution of ethyl 2-bromo-2,2-difluoro-acetate (1.5 g, 7.39 mmol), methane sulfonic acid (10 µL) and 2,2,6,6-tetramethyltetrahydropyran-4-ol (1 g, 9.43 mmol) was heated at 100°C for 16 hours. The mixture was concentrated *in vacuo.* The crude was purified by chromatography on silica gel (Heptane/DCM 100/0 to 50/50) to afford (2,2,6,6-tetramethyltetrahydropyran-4-yl) 2-bromo-2,2-difluoro-acetate (13a) (900 mg, 2.85 mmol, 30%).
¹H NMR (300 MHz, CDCl₃): *δ* (ppm) 1.28 (s, 6H), 1.32 (s, 6H), 1.50-1.58 (m, 2H), 1.99-2.04 (m, 2H), 5.33-5.43 (m, 1 H).

### Step 2: preparation of (2,2,6,6-tetramethyltetrahydropyran-4-yl) 2,2-difluoro-2-[(3-oxazol-2-yl-7-oxo-1,6-diazabicyclo[3.2.1]oct-3-en-6-yl)oxy]acetate, Example 13

At room temperature, DBU (76 µL, 0.5 mmol) was slowly added to a solution of 6-Hydroxy-3-oxazol-2-yl-1,6-diazabicyclo[3.2.1]oct-3-en-7-one (prepared according to the procedure described in WO2016177862 compound 37f) (100 mg, 0.48 mmol) and (2,2,6,6-tetramethyltetrahydropyran-4-yl) 2-bromo-2,2-difluoro-acetate (13a) (228 mg, 0.72 mmol) in DMSO (1 mL). The mixture was stirred at room temperature for 10 minutes and then diluted with AcOEt. The organic layer was washed with brine, dried over sodium sulfate, filtered and concentrated. The residue was purified by chromatography on silica gel (DCM/Acetone 10/0 to 4/6) to provide (2,2,6,6-tetramethyltetrahydropyran-4-yl) 2,2-difluoro-2-[(3-oxazol-2-yl-7-oxo-1,6-diazabicyclo[3.2.1]oct-3-en-6-yl)oxy]acetate (Example 13) (95 mg, 0.21 mmol, 45%).
MS *m*/*z* ([M+H]⁺ 442)*.*
¹H NMR (300 MHz, CDCl₃): *δ* (ppm) 1.26 (s, 6H), 1.30 (s, 6H), 1.47-1.60 (m, 2H), 1.97-2.02 (m, 2H), 3.23 (d, J = 11.2 Hz, 1H), 3.66-3.71 (m, 1H), 4.17 (dd, J = 2.1/18.1 Hz, 1H), 4.28 (dd, J = 2.5/5.3 Hz, 1H), 4.45 (dd, J = 1.4/18.1 Hz, 1H), 5.30-5.41 (m, 1H), 7.08-7.11 (m, 1H), 7.16 (d, J = 0.8 Hz, 1H), 7.63 (d, J = 0.8 Hz, 1H).
¹⁹F NMR (300 MHz, CDCl₃): *δ* (ppm) -83.70 (d, J = 139.8 Hz, 1F), -83.04 (d, J = 139.8 Hz, 1F).

### Biological Activity

Compound AF1, described as example 6 in WO2016177862, is the active form of prodrug compounds of formula (I) as Examples 1 to 3.

Compound AF2, described as example 37 in WO2016177862, is the active form of prodrug compounds of formula (I) as Example 4 and 6 to 13.

Compound AF3, which can be prepared by following the general procedure described in WO2016177862, is the active form of prodrug of formula (I) compound as Example 5.

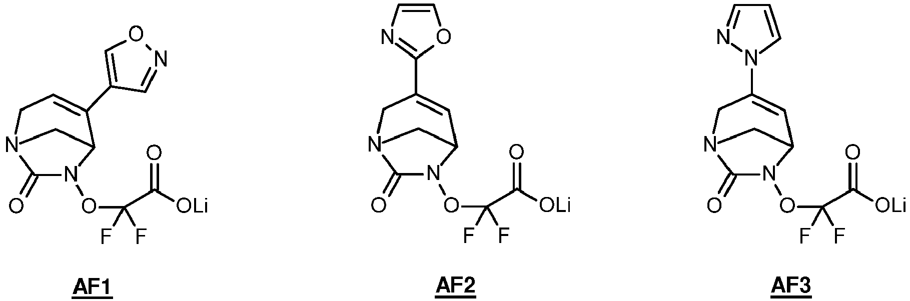

### Method 1: β-lactamase inhibitory activity, determination of IC₅₀ (Table 1)

Enzyme activity was monitored by spectrophotometric measurement of nitrocefin (NCF - TOKU-E, N005) hydrolysis at 485nm, at room temperature and in assay buffer A: 100mM Phosphate pH7, 2% glycerol and 0.1mg/mL Bovine serum albumin (Sigma, B4287). Buffer A was supplemented with 100mM NaHCO3 for several OXA-type enzymes (OXA-1, OXA-11, OXA-15 and OXA-163). Enzymes were cloned in *E. coli* expression vector, expressed and purified in house using classical procedures. To a transparent polystyrene plate (Corning, 3628) were added in each well 5µL DMSO or inhibitor dilutions in DMSO and 80µL enzyme in buffer A. Plates were immediately read at 485nm in a microplate spectrophotometer (BioTek, Powerwave HT) to enable background subtraction. After 30min of pre-incubation at room temperature, 15µL of NCF (100µM final) were finally added in each well. Final enzyme concentrations were 0.1 nM (TEM-1), 0.075nM (SHV-1), 1.5nM (SHV-12), 0.4nM (CTX-M-15), 1nM (KPC-2), 5nM (PC1 S. *aureus*), 0.2nM (P99 AmpC), 0.2nM (CMY-37), 0.8nM (DHA-1), 0.4nM (AmpC *P. aeruginosa*), 0.2nM (OXA-1), 1.2nM (OXA-11), 0.4nM (OXA-15), 0.2nM (OXA-23), 0.4nM (OXA-40), 0.3nM (OXA-48), 75nM (OXA-51), 0.5nM (OXA-58) and 0.15nM (OXA-163). After 20 min incubation at room temperature, plates were once again read at 485nm. Enzyme activity was obtained by subtracting the background from the final signal, and was converted to enzyme inhibition using non inhibited wells. IC₅₀ curves were fitted to a classical Langmuir equilibrium model with Hill slope using XLFIT (IDBS).

**Table 1: IC₅₀ of compounds AF1, AF2, AF3 against bacterial beta-lactamases**

| | IC50 (µM) | | |
|---|---|---|---|
| beta-lactamase | AF1 | AF2 | AF3 |
| TEM-1 | 0.010 | 0.0026 | 0.00081 |
| SHV-1 | 0.020 | 0.012 | 0.0060 |
| SHV-12 | 0.0038 | 0.0041 | 0.0043 |
| CTX-M-15 | 0.0024 | 0.0015 | 0.00066 |
| KPC-2 | 0.51 | 0.069 | 0.077 |
| SAU PC1 | 0.56 | 0.22 | 0.12 |
| P99 ampC | 2.2 | 0.73 | 0.27 |
| CMY-37 | 2.0 | 0.45 | 0.15 |
| DHA-1 | 11 | 0.37 | 0.21 |
| PAE ampC | 4.5 | 0.28 | 0.098 |
| OXA-1 | 1.6 | 2.1 | 1.1 |
| OXA-11 | 0.25 | 0.084 | 0.040 |
| OXA-15 | 0.024 | 0.14 | 0.12 |
| OXA-23 | 0.11 | 7.4 | 9.6 |
| OXA-40 | 0.12 | 8.3 | 8.9 |
| OXA-48 | 0.0030 | 0.0024 | 0.0051 |
| OXA-51 | 0.086 | 0.45 | 0.52 |
| OXA-58 | 0.015 | 0.58 | 1.0 |
| OXA-163 | 0.0072 | 0.011 | 0.0059 |

### Method 2: MIC of compounds alone and combined with antibacterials against bacterial isolates.

Compounds of the present invention were assessed against genotyped bacterial strains (Table 3, 4) alone or in combination with an antibacterial (Table 2). In the assays, MICs of said compounds or combination of antibiotics with fixed concentrations of said compounds (4 or 8 µg/mL) were determined by the broth microdilution method according to the Clinical Laboratory Standards Institute (CLSI - M7-A7). Briefly, compounds alone according to the invention were prepared in DMSO and spotted (2µL each) on sterile polystyrene plates (Corning, 3788). Combinations of compounds and antibiotics dilutions were prepared in DMSO and spotted (1µL each) on sterile polystyrene plates (Corning, 3788). Log phase bacterial suspensions were adjusted to a final density of 5.10⁵ CFU/mL in cation-adjusted Mueller-Hinton broth (ca-MHB; Becton-Dickinson and Company) and added to each well (98µL). Microplates were incubated for 16-20 h at 35 °C in ambient air. The MIC of the compounds was defined as the lowest concentration of said compounds that prevented bacterial growth as read by visual inspection. The MIC of ATB at each compound concentration was defined as the lowest concentration of ATB that prevented bacterial growth as read by visual inspection.

Results are presented in Tables 4, 5 and 6. They show the advantage of combining antibiotics including Cefixime with the active forms AF1, AF2 or AF3 of the prodrugs herein described to combat resistant isolates.

**Table 2 : Antibacterials or beta-lactamase inhibitors used in MIC and combination studies**

| Abbreviations - Antibacterials | |
|---|---|
| ATB | Antibiotic |
| AMX | Amoxicillin |
| CAZ | Ceftazidime |
| CDR | Cefdinir |
| FIX | Cefixime |
| FUR | Cefuroxime |
| POD | Cefpodoxime |
| CLA | Clavulanic acid |

**Table 3 : Bacterial species used in MIC determination**

| Abbreviations -Strains | |
|---|---|
| ECO | *Escherichia coli* |
| KPN | *Klebsiella pneumoniae* |
| ECL | *Enterobacter cloacae* |
| EAE | *Enterobacter aerogenes* |
| CFR | *Citrobacter freundii* |
| CKR | *Citrobacter koseri* |
| CMU | *Citrobacter murliniae* |
| MMO | *Morganella morpanii* |
| PMI | *Proteus mirabilis* |
| PRE | *Providencia rettgeri* |
| PST | *Providencia stuartii* |
| KOX | *Klebsiella oxytoca* |
| SMA | *Serratia marcescens* |
| STY | *Salmonella typhimurium* |

**Table 4: List of the basterial isolates, their resistance genotype, and the MIC of reference antibiotics.**

| | | MIC (µg/mL) | | | | | |
|---|---|---|---|---|---|---|---|
| | | ATB | | | | | |
| Strains ID | Resistance genotype | CAZ | FIX | AMX | FUR | POD | CDR |
| ECO UFR86 | ompC-, ompF- | 2 | 2 | 16 | 64 | 4 | 2 |
| ECO 260304 | CTX-M-15 | 16 | 32 | >256 | >256 | >256 | 256 |
| ECO 260096 | CTX-M-132 | 128 | >128 | >256 | >256 | >256 | >256 |
| KPN 270077 | TEM-1, SHV-1, CTX-M-15 | 128 | >128 | >256 | >256 | >256 | >256 |
| ECL 260508 | TEM-1, CTX-M-15 | 64 | >128 | >256 | >256 | >256 | >256 |
| ECO 190549 | CTX-M-1 | 4 | 16 | >256 | >256 | >256 | 256 |
| ECO 190314 | CTX-M-1 | 8 | 16 | >256 | >256 | >256 | >256 |
| ECO 180070 | TEM-1, CTX-M-15 | 64 | 128 | >256 | >256 | >256 | >256 |
| ECO 200159 | TEM-1, CTX-M-14 | 2 | 8 | >256 | >256 | >256 | 256 |
| ECO 200259 | CTX-M-14 | 2 | 8 | >256 | >256 | >256 | 256 |
| ECO 200344 | CTX-M-1 | 8 | 32 | >256 | >256 | >256 | >256 |
| KPN 700603 | SHV-18, OXA-2 | 64 | 16 | >256 | 32 | 16 | 4 |
| ECL UFR60 | TEM-1, CTX-M-15, KPC-2 | >128 | >128 | >256 | >256 | >256 | >256 |
| ECO UFR61O | TEM-1, KPC-2 | >128 | 32 | >256 | >256 | >256 | >256 |
| ECO UFR62 | TEM-1, CTX-M-9, KPC-2 | 8 | 128 | >256 | >256 | >256 | >256 |
| KPN UFR65 | TEM-1, SHV-11, KPC-2 | 128 | >128 | >256 | >256 | >256 | >256 |
| KPN UFR66 | TEM-1, SHV-11, CTX-M-15, KPC-2 | >128 | 512 | >256 | >256 | >256 | >256 |
| KPN 260251 | TEM-1, SHV-11, SHV-12, CTX-M-15, KPC-2 | >128 | >128 | >256 | >256 | >256 | >256 |
| KPN BAA-1898 | TEM-1, SHV-11, SHV-12, KPC-2 | 256 | >128 | >1024 | >512 | >512 | >256 |
| KPN 160143 | TEM-1, SHV-1, CTX-M-15, KPC-2, | 64 | >128 | >256 | >256 | >256 | >256 |
| | OXA-1 | | | | | | |
| KPN UFR67 | TEM-1, SHV-11, KPC-3 | >128 | >128 | >256 | >256 | >256 | >256 |
| KPN UFR68 | TEM-1, SHV-11, CTX-M-15, KPC-3 | 512 | >128 | >1024 | >256 | >256 | >256 |
| KPN 140513 | TEM-1, SHV-11, CTX-M-15, KPC-3 | >256 | >128 | >256 | >256 | >256 | >256 |
| KPN 260252 | TEM-1, SHV-11, KPC-3 | >128 | >128 | >256 | >256 | >256 | >256 |
| ECL 260253 | TEM-1, KPC-3 | >128 | >128 | >256 | >256 | >256 | >256 |
| ECL P99 | AmpC | 128 | >128 | >1024 | >512 | >512 | >256 |
| ECL 190310 | AmpC | 256 | >128 | >256 | >256 | >256 | >256 |
| ECL 200138 | AmpC | >256 | >128 | >256 | >256 | >256 | >256 |
| ECL 260323 | AmpC | >256 | >128 | >256 | >256 | >256 | 256 |
| ECL 260033 | AmpC | 512 | >128 | >256 | >256 | >256 | >256 |
| ECL NEM146383 | AmpC | 128 | >128 | >256 | >256 | >256 | >256 |
| EAE 200261 | TEM-x, AmpC | 128 | >128 | >256 | >256 | >256 | >256 |
| EAE 49469 | AmpC | 128 | >128 | >1024 | >128 | >128 | >128 |
| CFR UFR83 | TEM-3, AmpC | >128 | >128 | >256 | >256 | >256 | >256 |
| ECL UFR84 | TEM-1, AmpC, OXA-1 | >128 | >128 | >256 | >256 | >256 | >256 |
| ECL UFR85 | TEM-1, CTX-M-15, AmpC | 128 | >128 | >256 | >256 | >256 | >256 |
| KPN UFR76 | TEM-155, SHV-11, ACT-1, OXA-2 | >128 | >128 | >256 | >256 | >256 | >256 |
| ECL UFR70 | TEM-1, CTX-M-15, CMY-2, OXA-1, Porin loss | >128 | >128 | >256 | >256 | >256 | >256 |
| KPN UFR77 | CMY-2 | 32 | 128 | >256 | 64 | 64 | 64 |
| PMI UFR82 | CMY-2 | 4 | 8 | 256 | 16 | 64 | 16 |
| ECO UFR74 | SHV-1, DHA-1 | 64 | >128 | >256 | >256 | >256 | >256 |
| KPN UFR79 | DHA-1, OXA-1 | 16 | >128 | >256 | >256 | 32 | 256 |
| KPN UFR80 | SHV-11, DHA-1, OXA-1 | 0.5 | <=0.25 | >256 | 32 | 2 | 1 |
| KPN UFR78 | TEM-1, SHV-1, CTX-M-15, CMY-2, OXA-1, OXA-48 | >256 | >128 | >256 | >256 | >256 | >256 |
| KPN UFR81 | TEM-1, SHV-1, DHA-1, OXA-48 | 128 | >128 | >256 | >256 | >256 | >256 |
| ECL UFR14 | TEM-1, SHV-12, CTX-M-15, DHA-1, OXA-1, OXA-48 | >256 | >128 | >256 | >256 | >256 | >256 |
| ECO UFR17 | TEM-1, CTX-M-15, CMY-2, OXA-1, OXA-181 | >128 | >128 | >256 | >256 | >256 | >256 |
| ECO UFR19 | CTX-M-15, CMY-2, OXA-1, OXA-204 | 128 | >128 | >256 | >256 | >256 | >256 |
| KPN 110376 | TEM-1, SHV-1, CTX-M-15, OXA-1, OXA-48 | 128 | >128 | >256 | >256 | >256 | >256 |
| CFR UFR10 | OXA-48 | 128 | >128 | >256 | >256 | >256 | >256 |
| CFR UFR11 | TEM-1, OXA-1, OXA-48 | 8 | 32 | >256 | >256 | >256 | >256 |
| ECL UFR12 | CTX-M-9, OXA-48 | 2 | 16 | >256 | >256 | 128 | >256 |
| ECL UFR13 | TEM-1, SHV-12, CTX-M-9, OXA-48 | >256 | >128 | >256 | >256 | >256 | >256 |
| ECO UFR15 | TEM-1, OXA-48 | 0.5 | 1 | >256 | 16 | 2 | >256 |
| ECO UFR16 | TEM-1, CTX-M-15, OXA-1, OXA-48 | 64 | >128 | >256 | >256 | >256 | >256 |
| ECO UFR18 | CTX-M-15, OXA-204 | 128 | >128 | >256 | >256 | >256 | >256 |
| ECO 131119 | TEM-1, OXA-48 | 0.5 | <=0.25 | >1024 | 8 | 1 | 256 |
| ECO UFR20 | SHV-1, CTX-M-15, OXA-1, OXA-232 | 128 | 512 | >256 | >256 | >256 | >256 |
| KOX UFR21 | TEM-1, CTX-M-15, OXA-48 | 128 | >128 | >256 | >256 | >256 | >256 |
| KPN UFR22 O | TEM-1, SHV-1, OXA-48 | 2 | <=0.25 | >256 | 32 | 1 | >256 |
| KPN UFR23 | TEM-1, SHV-1, OXA-48 | 0.5 | <=0.25 | >256 | 8 | 0.5 | >256 |
| KPN UFR24 | TEM-1, SHV-2, SHV-11, OXA-1, OXA-48, OXA-47 | >128 | >128 | >256 | 128 | 256 | >256 |
| KPN UFR25 | TEM-1, SHV-11, CTX-M-15, OXA-162 | 128 | >128 | >256 | >256 | >256 | >256 |
| KPN UFR27 | TEM-1, SHV-28, CTX-M-15, OXA-204 | >128 | >128 | >256 | >256 | >256 | >256 |
| KPN UFR28 | TEM-1, SHV-1, CTX-M-15, OXA-1, OXA-232 | 64 | 256 | >256 | >256 | >256 | >256 |
| SMA UFR30 | OXA-405 | 8 | 1 | >256 | >256 | 32 | >256 |
| CKO ROU | TEM-1, SHV-12, CTX-M-15, OXA-1, OXA-48 | 1 | 1 | >256 | 64 | 4 | >256 |
| KPN LIB | SHV-11, OXA-48 | 0.25 | <=0.25 | >256 | 16 | 1 | >256 |
| ECL 2185D | OXA-163 | >128 | >128 | >256 | >256 | >256 | >256 |
| KPN ARA | TEM-1, SHV-11, CTX-M-15, OXA-1, OXA-48 | 128 | >128 | >256 | >256 | >256 | >256 |
| KPN 6299 | TEM-1, SHV-11, OXA-163 | 256 | 8 | >1024 | >512 | 64 | 256 |
| KPN 131119 | TEM-1, SHV-11, CTX-M-15, OXA-1, OXA-48 | >128 | >128 | >256 | >256 | >256 | >256 |
| ECO RGN238 | OXA-1 | 0.5 | <=0.25 | >1024 | 16 | 2 | 0.5 |
| STY S3371 | OXA-1 | 0.5 | <=0.25 | >256 | 32 | 4 | 0.5 |
| ECO 5302 | TEM-1, OXA-1 | 0.5 | 0.5 | >256 | 32 | 4 | 1 |
| ECO 4133 | TEM-30, OXA-1 | 0.5 | 0.5 | >256 | 16 | 2 | 0.5 |
| ECO 190457 | CTX-M-15, OXA-1 | 16 | 128 | >256 | >256 | >256 | >256 |
| ECO 260508 | TEM-1, CTX-M-15, OXA-1 | 128 | >128 | >256 | >256 | >256 | >256 |
| KPN 190128 | TEM-1, SHV-32, CTX-M-15, OXA-1 | >128 | >128 | >256 | >256 | >256 | >256 |
| KPN 190270 | TEM-1, SHV-76, CTX-M-15, OXA-1 | 128 | >128 | >256 | >256 | >256 | >256 |
| KPN 200047 | TEM-1, SHV-32, CTX-M-15, OXA-1 | 128 | >128 | >256 | >256 | >256 | >256 |
| KPN 190551 | TEM-1, SHV-1, CTX-M-15, OXA-1 | 64 | >128 | >256 | >256 | >256 | >256 |
| KPN 190425 | TEM-1, SHV-1, CTX-M-15, OXA-1 | 128 | >128 | >256 | >256 | >256 | >256 |
| KPN 200327 | TEM-1, SHV-1, CTX-M-15, OXA-1 | 32 | 64 | >256 | >256 | >256 | >256 |
| ECO 190317 | TEM-1, SHV-12, CTX-M-15, OXA-1 | 128 | >128 | >1024 | >512 | >512 | >256 |
| ECL 190408 | TEM-1, CTX-M-15, OXA-1 | 128 | 512 | >256 | >256 | >256 | >256 |
| ECL 200322 | TEM-1, CTX-M-15, OXA-1 | >128 | >128 | >256 | >256 | >256 | >256 |
| MMO 200321 | TEM-1, CTX-M-15, OXA-1 | 16 | >128 | >256 | >256 | >256 | 256 |
| KPN 260376 | SHV-1, SHV-49, OXA-1 | 128 | >128 | >256 | >256 | >256 | >256 |
| ECO UFR32 | TEM-1, VEB-1, OXA-10 | >128 | 128 | >256 | | | |
| KPN UFR33 | TEM-2, SHV-12, CTX-M-15, OXA-1, OXA-10 | >128 | >128 | >256 | | | |
| ECL HAN | OXA-35 | 256 | >128 | >256 | | | |
| CFR UFR37 | TEM-1, CTX-M-15, NDM-1 | >128 | >128 | >256 | | | |
| ECL UFR38 | CTX-M-15, NDM-1 | >256 | >128 | >256 | | | |
| ECO UFR39 | CTX-M-15, NDM-1 | >256 | >128 | >1024 | >256 | >256 | |
| ECO UFR41 | TEM-1, CTX-M-15, CMY-2, OXA-1, NDM-4 | >128 | >128 | >256 | | | |
| KPN UFR42 | SHV-2, CTX-M-15, OXA-1, OXA-181, NDM-1 | >128 | >128 | >256 | >256 | >256 | |
| KPN UFR43 | SHV-11, CTX-M-15, CMY-2, OXA-1, NDM-1 | >128 | >128 | >256 | | | |
| KPN 121206 | SHV-1, NDM-1 | >256 | >128 | >256 | >256 | >256 | |
| CMU 210102 | VIM-4 | >128 | >128 | >256 | | | |
| ECO UFR45 | TEM-1, CMY-4, OXA-1, OXA-48, VIM-1 | 2 | >128 | >256 | | | |
| KPN UFR46 | TEM-1, SHV-12, CTX-M-15, OXA-9, VIM-1 | >128 | >128 | >256 | | | |
| ECL UFR51 | SHV-12, IMP-8 | >256 | >128 | >256 | | | |
| ECO UFR52 | TEM-1, SHV-12, IMP-8 | >128 | >128 | >256 | | | |
| KPN UFR53 | TEM-1, IMP-1 | >128 | >128 | >256 | | | |
| PST UFR94 | CTX-M-14 | 1 | 0.5 | >128 | >256 | 32 | 64 |
| PST UFR95 | TEM-24 | 64 | 4 | >128 | 128 | 16 | 32 |
| PMI UFR120 | TEM-1, SHV-11, CTX-M-14 | <=0.25 | 0.5 | >128 | >256 | >256 | 64 |
| PMI UFR121 | TEM-1, TEM-52 | 16 | 128 | >128 | >256 | >256 | >256 |
| PMI UFR122 | TEM-1, CTX-M-15 | 1 | 1 | >128 | >256 | 64 | 16 |
| PMI UFR123 | CTX-M-1 | 2 | 128 | >128 | >256 | >256 | >256 |
| PMI UFR124 | CTX-M-2 | 2 | >128 | >128 | >256 | >256 | >256 |
| PMI UFR125 | CTX-M-71 | 2 | 0.5 | >128 | >256 | >256 | 256 |
| PMI UFR126 | TEM-2, PER-1 | >128 | 1024 | >128 | >256 | >256 | >256 |
| PMI UFR127 | VEB-1 | >128 | >128 | >128 | >256 | 128 | >256 |
| PMI UFR129 | TEM-1, VEB-6 | >128 | >128 | >128 | >256 | >256 | >256 |
| SMA UFR134 | TEM-1, BES-1 | 8 | >128 | >128 | >256 | >256 | 256 |
| EAE UFR201 | TEM-1, SHV-12, CTX-M-15 | 128 | >128 | >128 | >256 | >256 | >256 |
| EAE UFR202 | TEM-24 | >256 | >128 | >128 | >256 | >256 | 256 |
| ECO UFR207 | CTX-M-15 | 64 | >128 | >128 | >256 | >256 | >256 |
| ECO UFR208 | SHV-12 | 128 | >128 | >128 | >256 | >256 | >256 |
| ECO UFR209 | TEM-1, CTX-M-15 | 128 | 1024 | >128 | >256 | >256 | >256 |
| ECO UFR210 | SHV-12 | 32 | 32 | >128 | >256 | >256 | >256 |
| ECO UFR211 | TEM-24 | >128 | >128 | >128 | 64 | 32 | 32 |
| EAE UFR213 | TEM-24 | >256 | >128 | >128 | >256 | 256 | 256 |
| KPN UFR215 | SHV-27, CTX-M-15 | >128 | >128 | >128 | >256 | >256 | >256 |
| KPN UFR216 | SHV-28, CTX-M-15 | 128 | >128 | >128 | >256 | >256 | >256 |
| KPN UFR217 | TEM-1, SHV-1, CTX-M-15 | 128 | >128 | >128 | >256 | >256 | >256 |
| ECO UFR218 | TEM-1, SHV-1, CTX-M-15 | 64 | >128 | >128 | >256 | >256 | >256 |
| KPN UFR219 | SHV-12, CTX-M-15 | 256 | >128 | >128 | >256 | >256 | >256 |
| KPN UFR2270 | TEM-x, SHV-x, CTX-M-x | >128 | >128 | >128 | >256 | >256 | >256 |
| MMO UFR144 | TEM-1, CTX-M-15 | 8 | >128 | >128 | >256 | >256 | 128 |
| KOX UFR173 | OXY2-2 | 8 | 16 | >128 | >256 | >256 | >256 |
| PST UFR235 | VEB-1 | >128 | 512 | >128 | 256 | 128 | 256 |
| PMI UFR237 | VEB-6 | >128 | >128 | >128 | >256 | >256 | >256 |
| MMO UFR240 | CTX-M-9 | 0.5 | 1 | >128 | >256 | 256 | 64 |
| MMO UFR241 | TEM-1, CTX-M-15 | 8 | >128 | >128 | >256 | >256 | 128 |
| MMO UFR242 | TEM-52 | 32 | 1024 | >128 | >256 | >256 | >256 |
| CFR UFR248 | CTX-M-15 | 128 | >128 | >128 | >256 | >256 | >256 |
| CFR UFR249 | TEM-1, CTX-M-15 | 64 | >128 | >128 | >256 | >256 | >256 |
| CFR UFR250 | TEM-1, SHV-28, CTX-M-15 | 128 | >128 | >128 | >256 | >256 | >256 |
| ECO UFR174 | TEM-1, KPC-2, OXA-1 | 8 | 8 | >128 | >256 | >256 | >256 |
| ECO UFR175 | TEM-1, KPC-2, OXA-9 | 32 | 64 | >128 | >256 | >256 | >256 |
| ECO UFR176 | KPC-3, OXA-9* | 256 | 64 | >128 | >256 | >256 | >256 |
| SMA UFR135 | TEM-1, KPC-2 | 32 | 64 | >128 | >256 | >256 | >256 |
| SMA UFR136 | TEM-1, SHV-12, KPC-2 | >256 | >128 | >128 | >256 | >256 | >256 |
| CFR UFR146 | TEM-1, KPC-2 | 32 | 64 | >128 | >256 | >256 | 256 |
| EAE UFR199 | TEM-1b, SHV-12, KPC-2, OXA-9 | >256 | >1024 | >128 | >256 | >256 | >256 |
| ECL UFR200 | TEM-1, SHV-12, KPC-2 | >256 | >128 | >128 | >256 | >256 | >256 |
| SMA UFR137 | SME-1 | 0.5 | 0.5 | >128 | 256 | 1 | 4 |
| SMA UFR138 | SME-1 | <=0.25 | 0.5 | >128 | 256 | 2 | 8 |
| SMA UFR139 | SME-2 | <=0.25 | 1 | >128 | >256 | 8 | 64 |
| PMI UFR130 | CMY-2 | 4 | 8 | >128 | 8 | 128 | 16 |
| ECO UFR212 | CMY-2 | 128 | >128 | >128 | >256 | >256 | >256 |
| KPN UFR220 | TEM-1, SHV-12, DHA-1 | >128 | >128 | >128 | >256 | >256 | >256 |
| KPN UFR221 | TEM-1, SHV-11, CTX-M-14, DHA-1 | 16 | 64 | >128 | >256 | 256 | 128 |
| KPN UFR222 | DHA-2 | >256 | >128 | >128 | >256 | >256 | >256 |
| SMA UFR239 | ESAC | 32 | 2 | >128 | 256 | 16 | 128 |
| MMO UFR243 | DHA-1 | 1 | 8 | >128 | 128 | 64 | 64 |
| MMO UFR244 | DHA-1 | 0.5 | 4 | >128 | 64 | 16 | 32 |
| MMO UFR245 | DHA-1 | 8 | 32 | >128 | 128 | 64 | 64 |
| MMO UFR246 | DHA-1 | 4 | 32 | >128 | 128 | 64 | 64 |
| MMO UFR247 | DHA-1 | 0.5 | 16 | >128 | >256 | 64 | 128 |
| PMI UFR128 | VEB-1, OXA-10 | >128 | >128 | >128 | | | |
| PMI UFR133 | OXA-23 | <=0.25 | <=0.25 | >128 | | | |
| PRE UFR99 | OXA-1, OXA-181 | >256 | >128 | >128 | >256 | >256 | >256 |
| KOX UFR223 | SHV-11, OXA-48 | 0.5 | <=0.125 | >128 | 8 | 0.5 | >256 |
| KOX UFR224 | CTX-M-15, OXA-48 | 64 | >128 | >128 | >256 | >256 | >256 |
| SMA UFR141 | OXA-48 | 1 | 2 | >128 | >256 | 8 | >256 |
| SMA UFR142 | OXA-48 | 0.5 | 2 | >128 | >256 | 8 | >256 |
| SMA UFR143 | CTX-M-15, OXA-1, OXA-48 | 64 | 512 | >128 | >256 | >256 | >256 |
| CKO UFR149 | OXA-48 | >128 | 0.5 | >128 | >256 | >256 | >256 |
| CKO UFR150 | TEM-1, OXA-48 | 4 | 2 | >128 | 64 | 16 | >256 |
| ECO UFR184 | CTX-M-15, CMY-4, OXA-1, OXA-204 | 128 | >128 | >128 | >256 | >256 | >256 |
| ECO UFR185 | OXA-48 | >256 | >128 | >128 | >256 | >256 | >256 |
| ECO UFR186 | TEM-1, CTX-M-14, OXA-48 | 8 | 32 | >128 | >256 | >256 | >256 |
| ECO UFR187 | CTX-M-15, OXA-48 | 8 | 32 | >128 | >256 | >256 | >256 |
| ECO UFR189 | TEM-1, CTX-M-15, OXA-48 | 128 | >128 | >128 | >256 | >256 | >256 |
| ECO UFR190 | CTX-M-24, OXA-48 | 2 | 64 | >128 | >256 | >256 | >256 |
| ECO UFR191 | TEM-1, CTX-M-24, OXA-48 | 4 | >128 | >128 | >256 | >256 | >256 |
| ECL UFR194 | OXA-48 | 1 | 4 | >128 | 32 | 16 | >256 |
| ECL UFR195 | TEM-1, CTX-M-15, OXA-1, OXA-48 | 128 | >128 | >128 | >256 | >256 | >256 |
| ECL UFR196 | TEM-1, CTX-M-15, OXA-1, OXA-48 | >256 | >128 | >128 | >256 | >256 | >256 |
| ECL UFR197 | TEM-1, CTX-M-15, OXA-1, OXA-48 | 128 | >128 | >128 | >256 | >256 | >256 |
| ECL UFR198 | TEM-1, SHV-12, CTX-M-15, DHA-1, OXA-1, OXA-48 | >256 | >128 | >128 | >256 | >256 | >256 |
| PRE UFR236 | TEM-1, OXA-48 | 32 | 32 | >128 | 64 | 64 | >256 |
| CFR UFR253 | TEM-1, SHV-12, OXA-48 | >128 | >128 | >128 | 32 | 32 | >256 |
| CFR UFR254 | VEB-1b, OXA-48, qnrA | 128 | 32 | >128 | 32 | 32 | 256 |
| SMA UFR238 | OXA-48 | 0.5 | 1 | >128 | >256 | 8 | >256 |
| PRE UFR96 | CTX-M-15, NDM-1 | >128 | >128 | >128 | | | |
| PRE UFR97 | TEM-1, NDM-1 | >128 | >128 | >128 | | | |
| PST UFR98 | TEM-1, CMY-6, OXA-1, NDM-1, RmtC | >256 | >128 | >128 | | | |
| PMI UFR131 | CMY-16, OXA-1, OXA-10, NDM-1, ArmA | >128 | >128 | >128 | | | |
| PMI UFR132 | VEB-6, DHA-1, NDM-1, AphA6, AacA4 | >128 | >128 | >128 | | | |
| SMA UFR140 | IMP-1 | >128 | >128 | >128 | | | |
| MMO UFR145 | CTX-M-15, NDM-1 | >128 | >128 | >128 | | | |
| CFR UFR147 | TEM-1, CTX-M-15, OXA-1, OXA-181, OXA-10, OXA-9, NDM-1, ArmA, dfrA12, aadA2 | >128 | >128 | >128 | | | |
| CFR UFR148 | TEM-1, TEM-2, CTX-M-15, NDM-1 | >128 | >128 | >128 | | | |
| KPN UFR162 | SHV-28, TEM-1, CTX-M-15, OXA-181, OXA-181, NDM-1 | >256 | >128 | >128 | | | |
| KPN UFR163 | TEM-1, SHV-1, CTX-M-15, OXA-232, OXA-9, NDM-1, qnrB1, qepA, RmtB | >256 | >128 | >128 | | | |
| KPN UFR164 | SHV-11, CTX-M-15, OXA-1, OXA-181, NDM-1 | >256 | >128 | >128 | | | |
| KPN UFR165 | TEM-1, SHV-11, CTX-M-15, OXA-1, OXA-181, NDM-1 | >256 | >128 | >128 | | | |
| KPN UFR166 | TEM-1, TEM-1, CTX-M-15, OXA-181, OXA-9, NDM-1 | >256 | >128 | >128 | | | |
| KPN UFR167 | TEM-1, SHV-12, CTX-M-15, OXA-9, NDM-1 | >256 | >128 | >128 | | | |
| KPN UFR168 | SHV-2, CTX-M-15, OXA-1, OXA-181, NDM-1, ArmA | >256 | >128 | >128 | | | |
| KPN UFR169 | TEM-1, SHV-28, CTX-M-15, CMY-4, OXA-1, NDM-1 | >256 | >128 | >128 | | | |
| KPN UFR170 | TEM-1, SHV-28, CTX-M-15, CMY-6, OXA-1, OXA-9, NDM-1 | >256 | >128 | >128 | | | |
| KPN UFR171 | TEM-1, SHV-28, CTX-M-15, OXA-1, OXA-10, NDM-1, RmtA | >256 | >128 | >128 | | | |
| KPN UFR172 | SHV-38, CMY-16, OXA-10, NDM-1 | >128 | >128 | >128 | | | |
| ECO UFR177 | TEM-1, CTX-M-15, OXA-1, OXA-2, NDM-1, RmtC | >256 | >128 | >128 | | | |
| ECO UFR178 | TEM-1, CTX-M-15, OXA-9, NDM-1 | >256 | >128 | >128 | | | |
| ECO UFR179 | TEM-1, SHV-12, CTX-M-15, NDM-1 | >256 | >128 | >128 | | | |
| ECO UFR180 | TEM-1, CMY-30, OXA-1, NDM-1 | >256 | >128 | >128 | | | |
| ECO UFR181 | TEM-1, CTX-M-15, NDM-5 | >256 | >128 | >128 | | | |
| ECO UFR182 | CTX-M-15, OXA-1, NDM-6 | >256 | >128 | >128 | | | |
| ECO UFR183 | CTX-M-15, OXA-1, NDM-7 | >256 | >128 | >128 | | | |
| ECL UFR192 | TEM-1, NDM-1 | >128 | >128 | >128 | | | |
| ECL UFR193 | TEM-1, CTX-M-15, OXA-1, NDM-1, RmtC | >128 | >128 | >128 | | | |
| ECO UFR255 | NDM-1 | >128 | >128 | >128 | | | |
| KPN 140347 | CTX-M-15, NDM-1 | >128 | >128 | >128 | | | |
| ECL UFR203 | TEM-1, SHV-12, VIM-1 | >256 | >128 | >128 | | | |
| ECO UFR204 | TEM-1, CMY-4, OXA-48, VIM-1 | >128 | >128 | >128 | | | |
| ECO UFR205 | CTX-M-15, VIM-4 | >128 | >128 | >128 | | | |
| ECO UFR206 | TEM-1, CTX-M-15, OXA-1, VIM-4 | >128 | >128 | >128 | | | |
| ECL UFR214 | TEM-1, SHV-31, CTX-M-15, VIM-4 | >128 | >128 | >128 | | | |
| KPN UFR2290 | SHV-12, VIM-1 | >128 | >128 | >128 | | | |
| CFR UFR251 | SHV-11, VIM-1 | >256 | >128 | >128 | | | |
| CFR UFR252 | VIM-2 | 64 | >128 | >128 | | | |
| ECO UFR252GO | VIM-2 | >256 | >128 | >128 | | | |
| ECL UFR252PT | VIM-2 | >256 | >128 | >128 | | | |

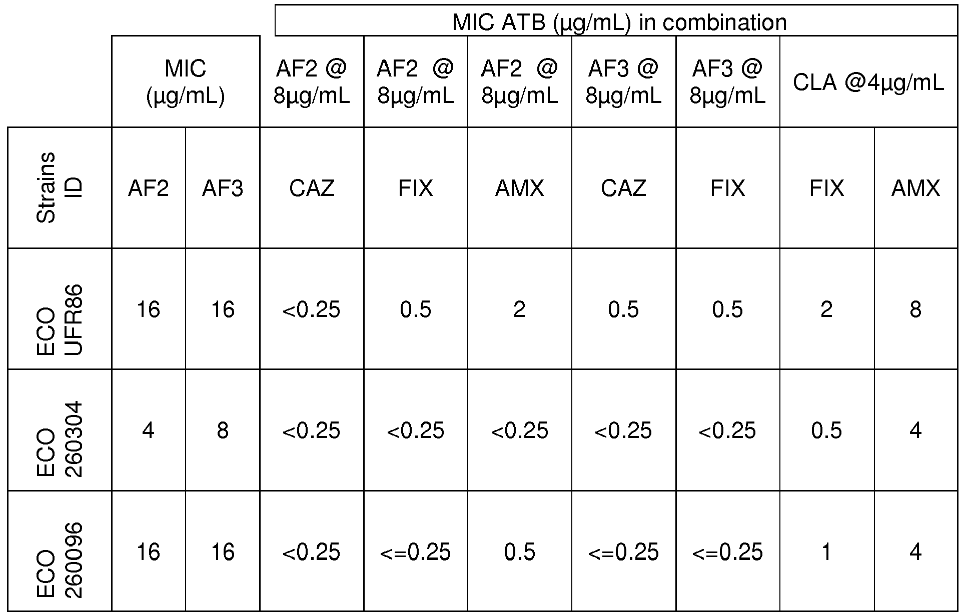

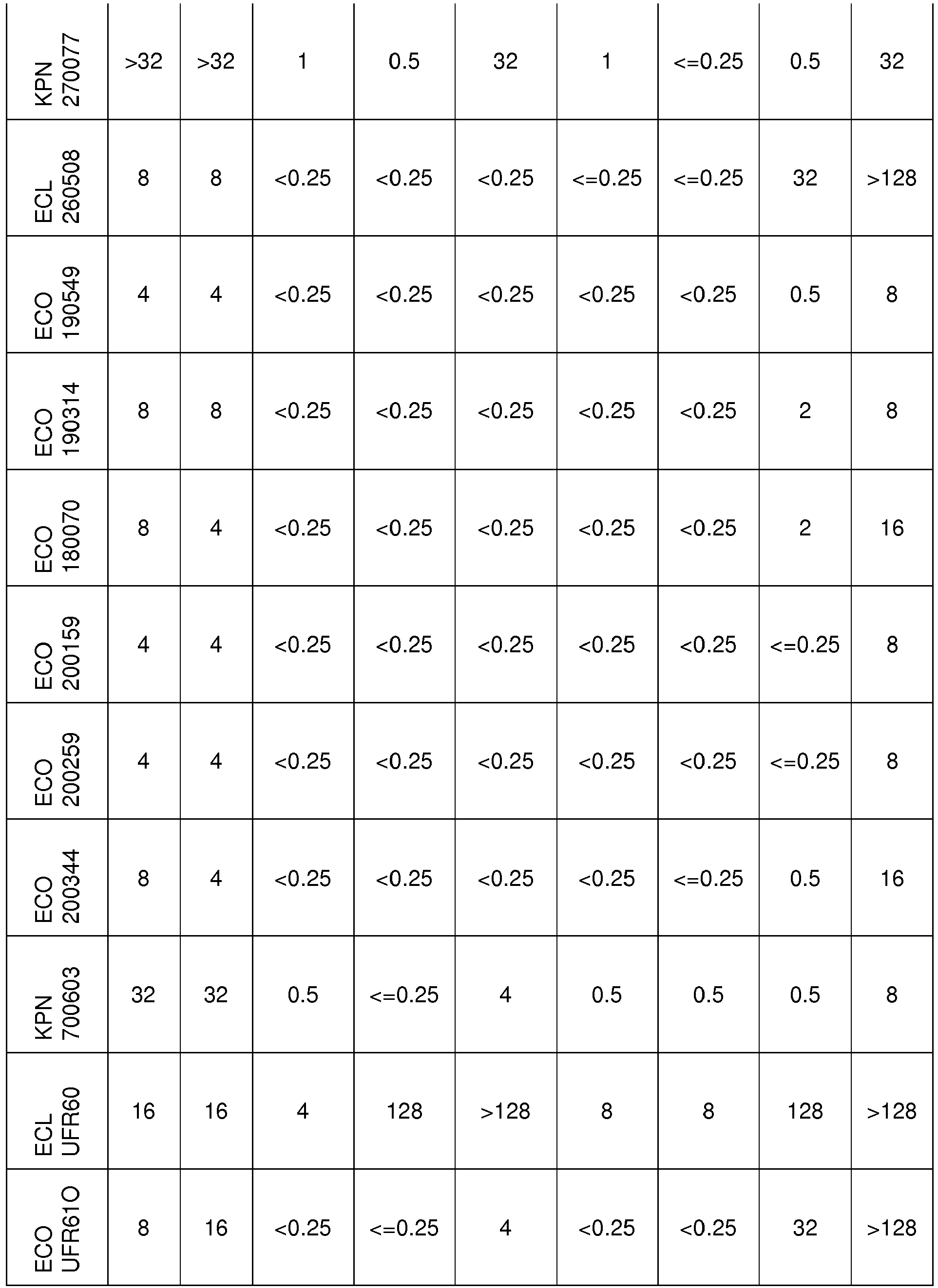

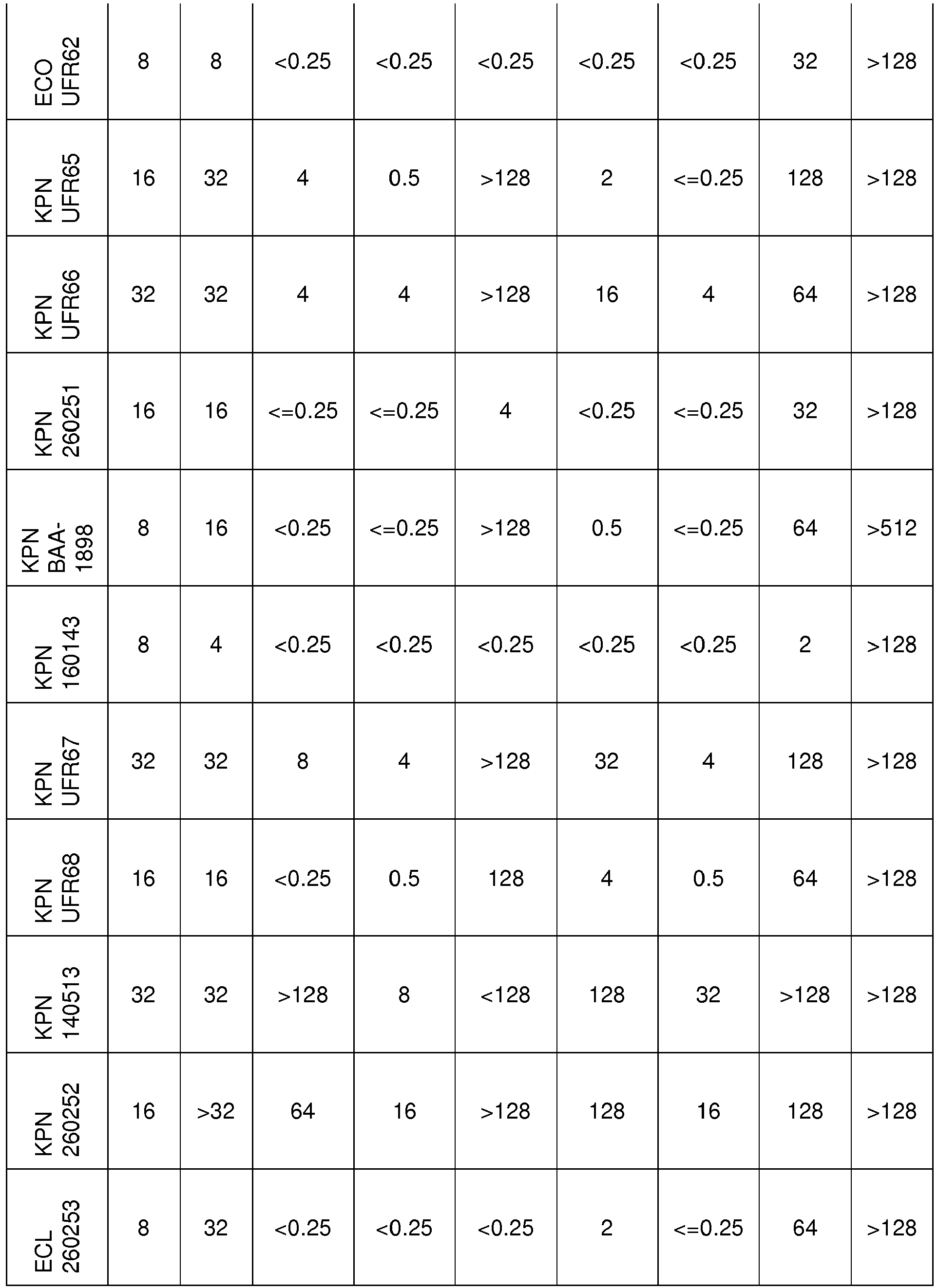

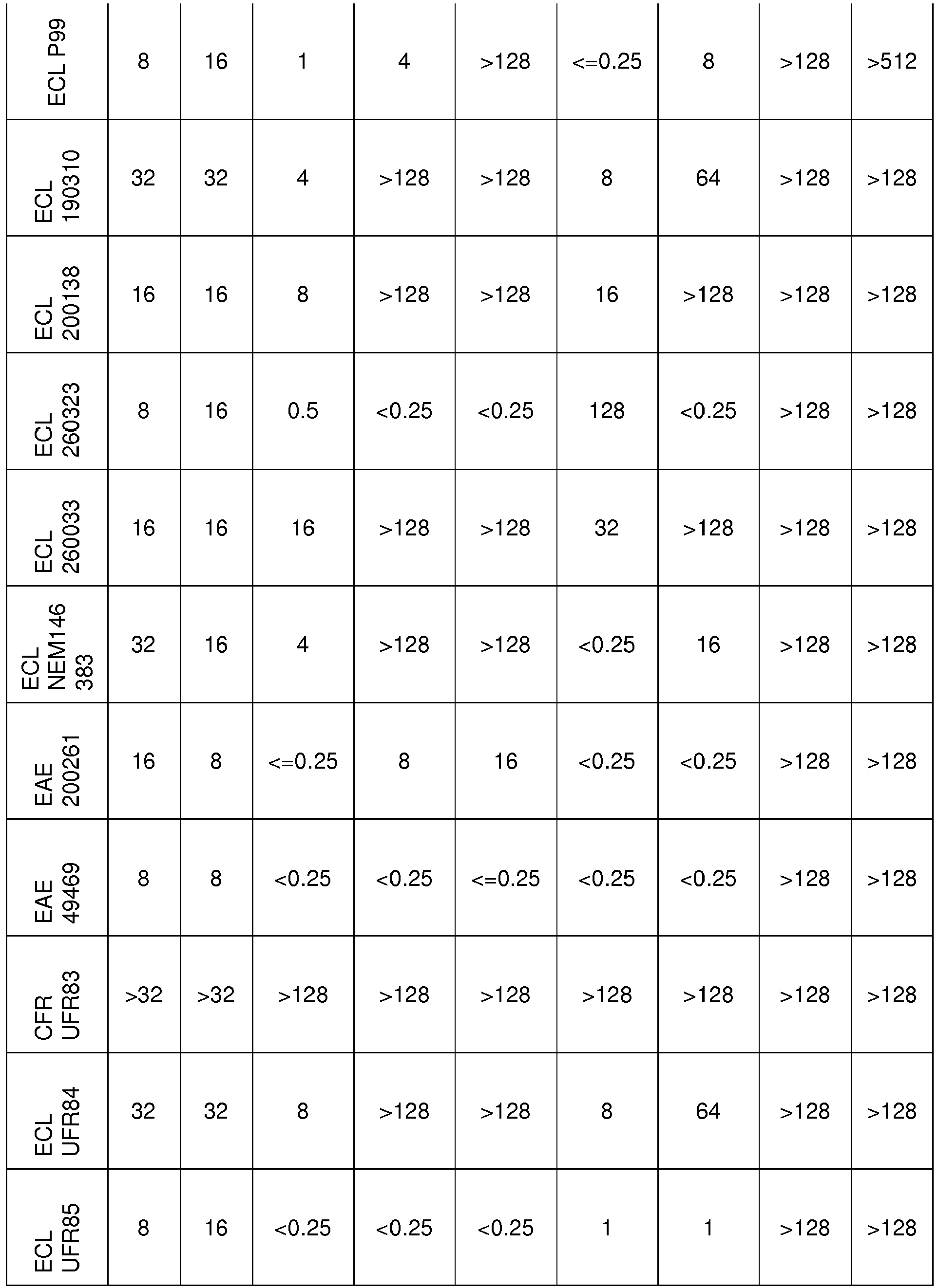

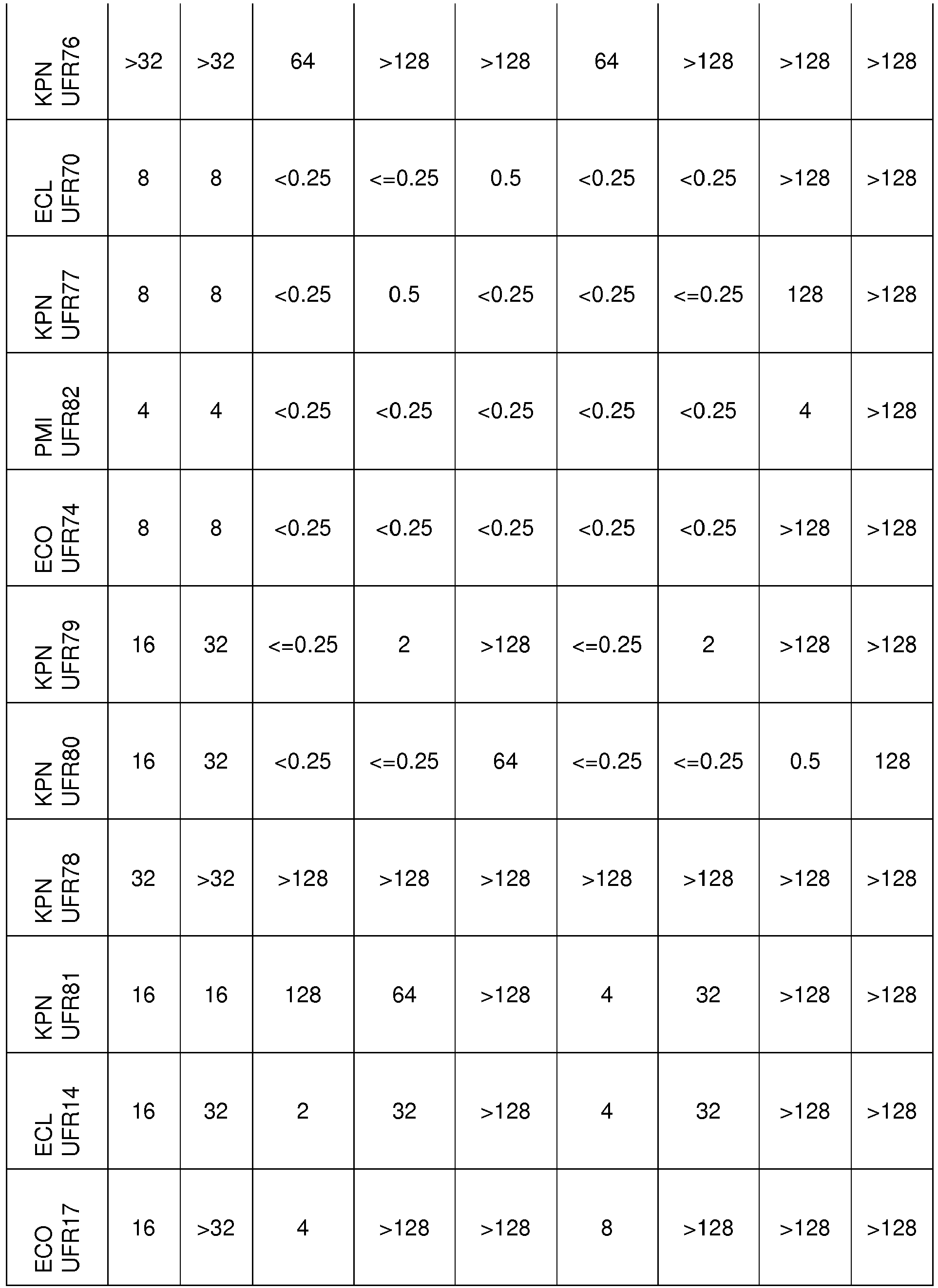

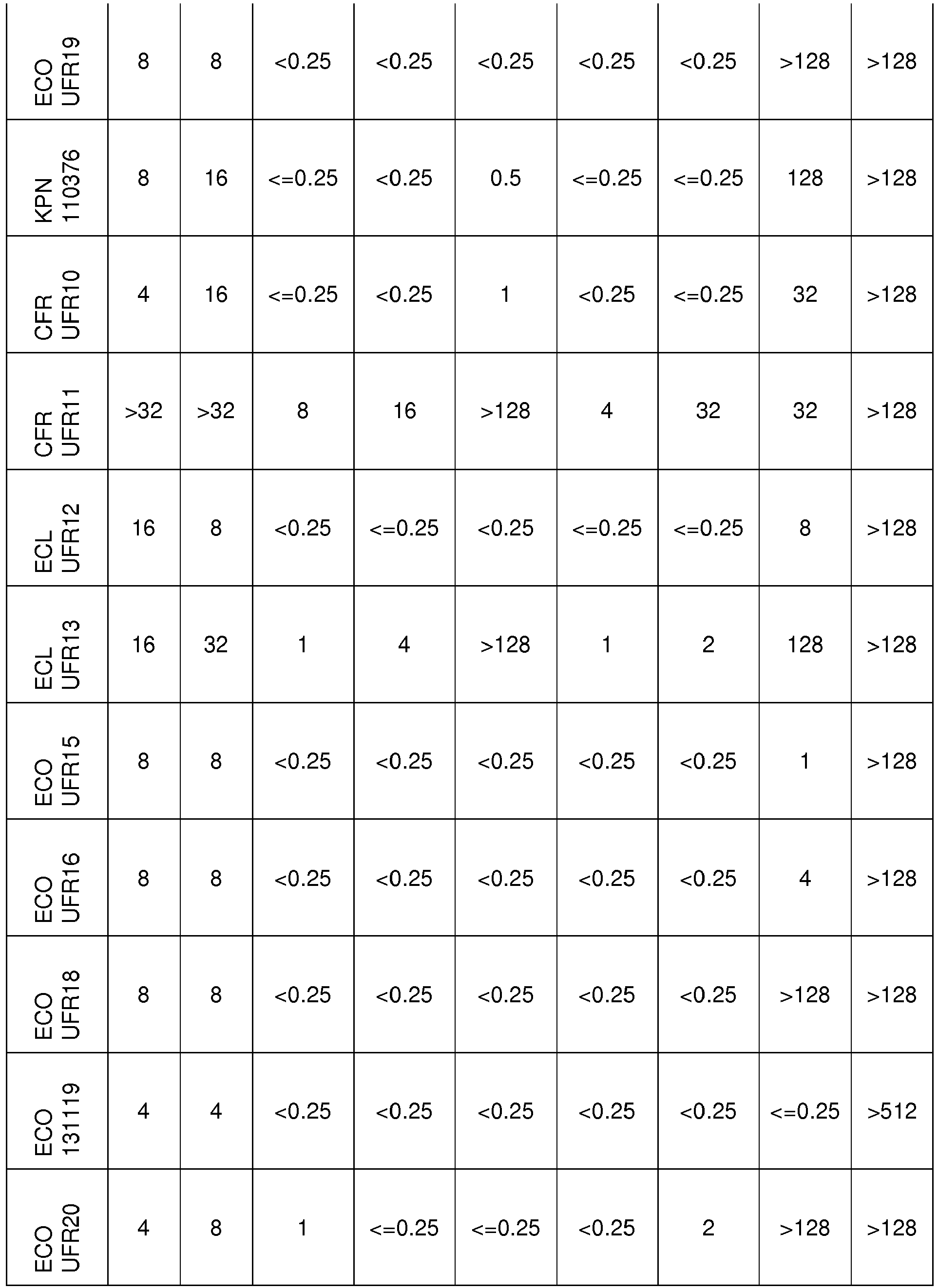

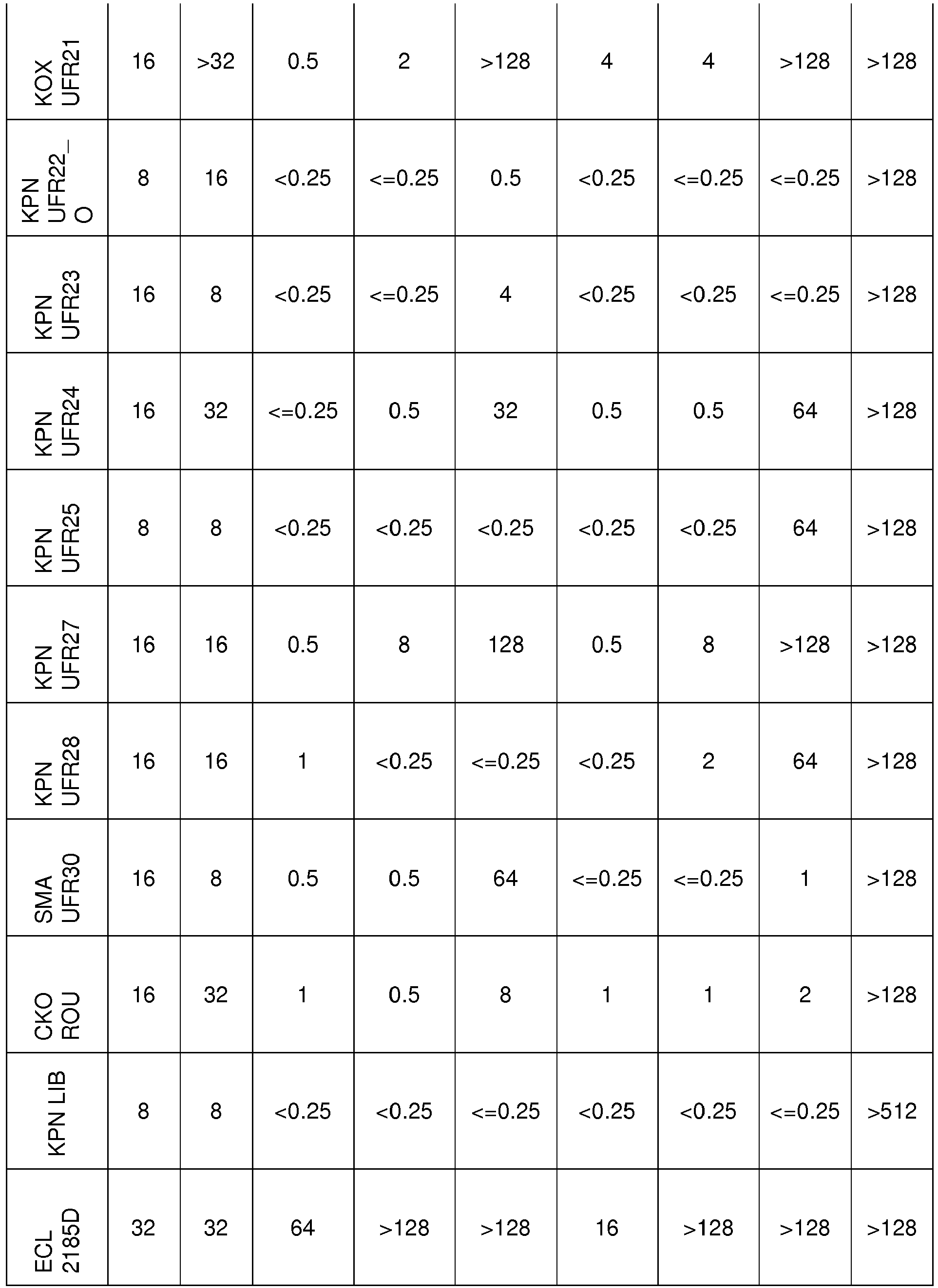

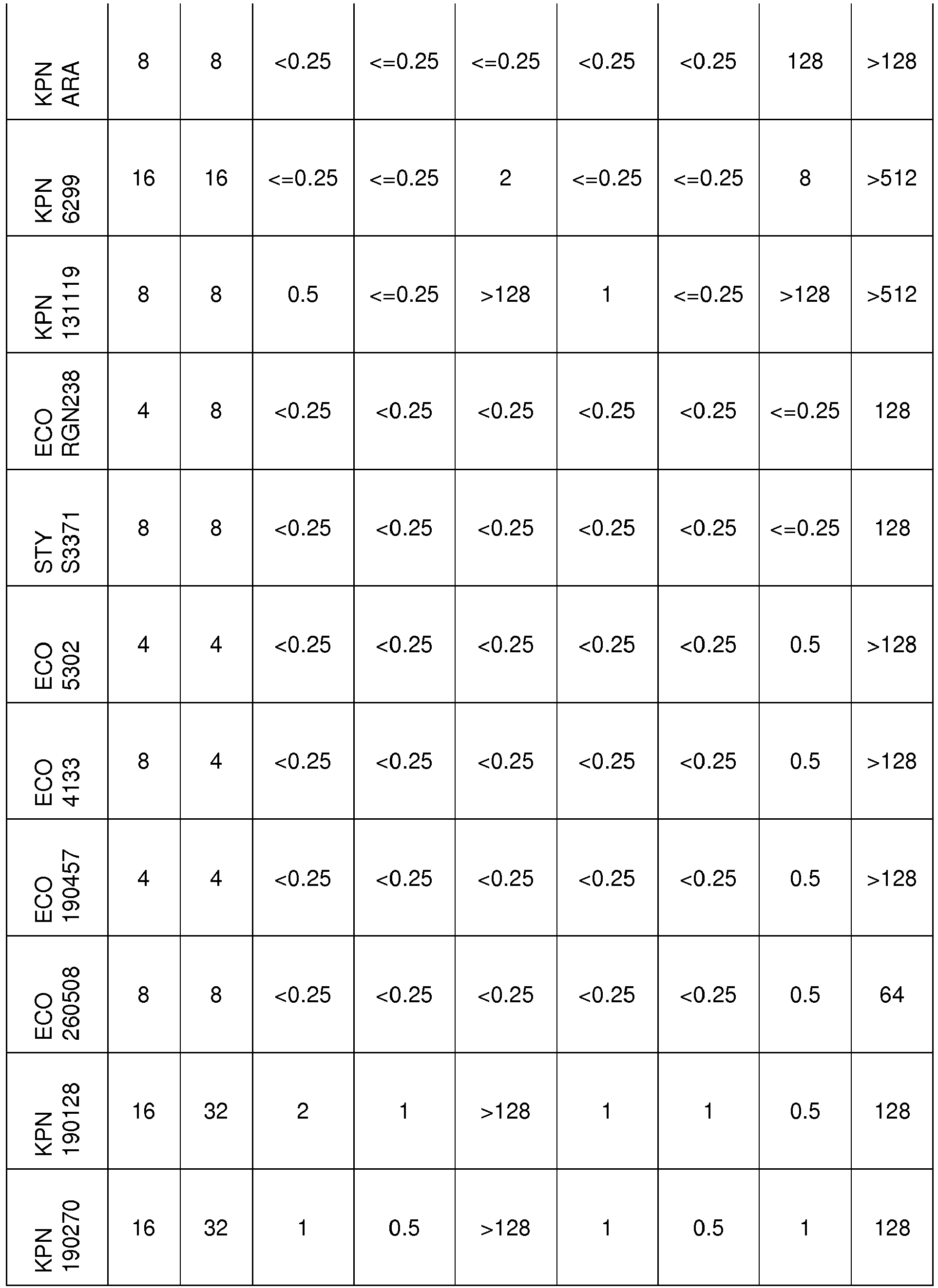

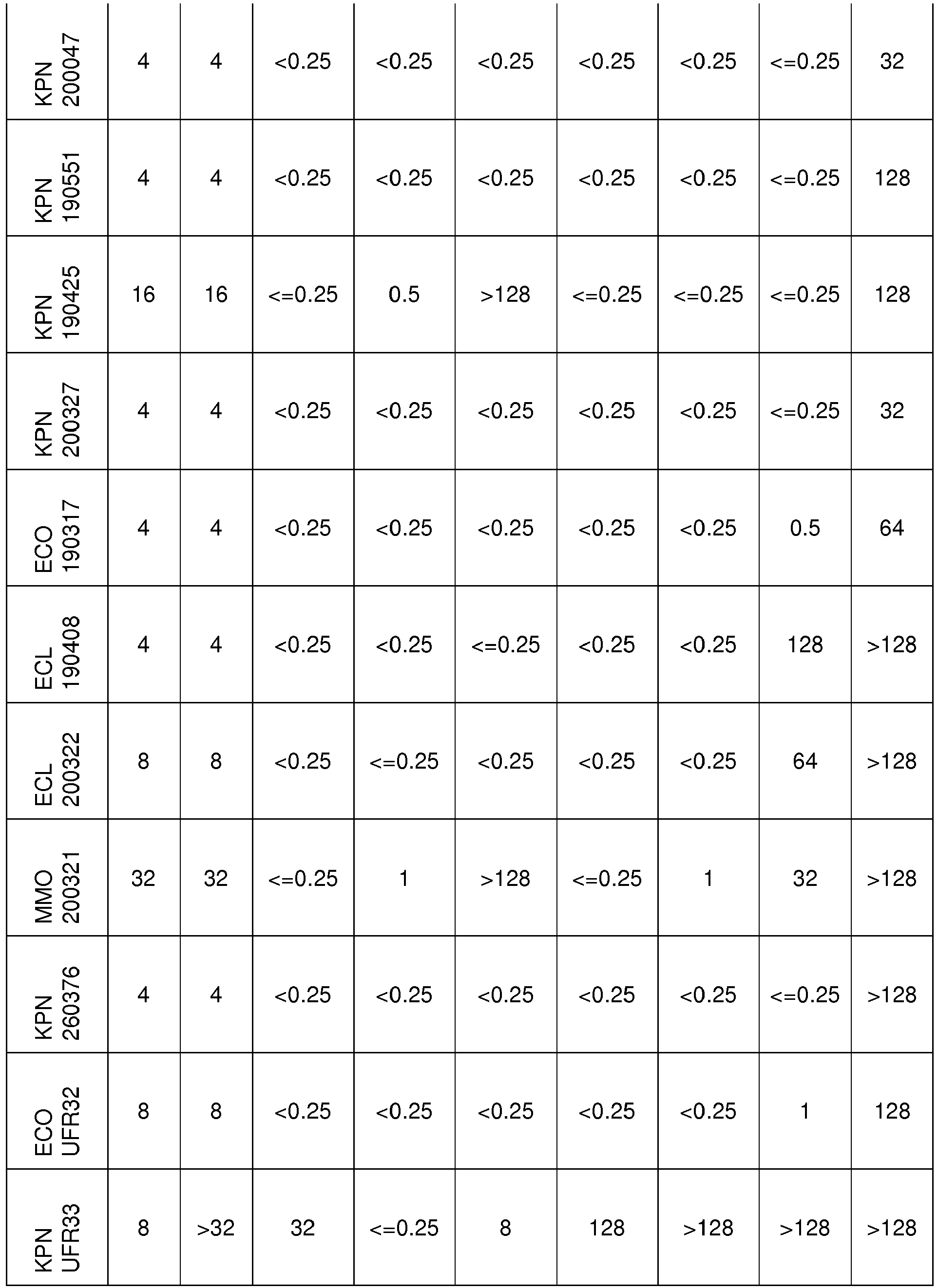

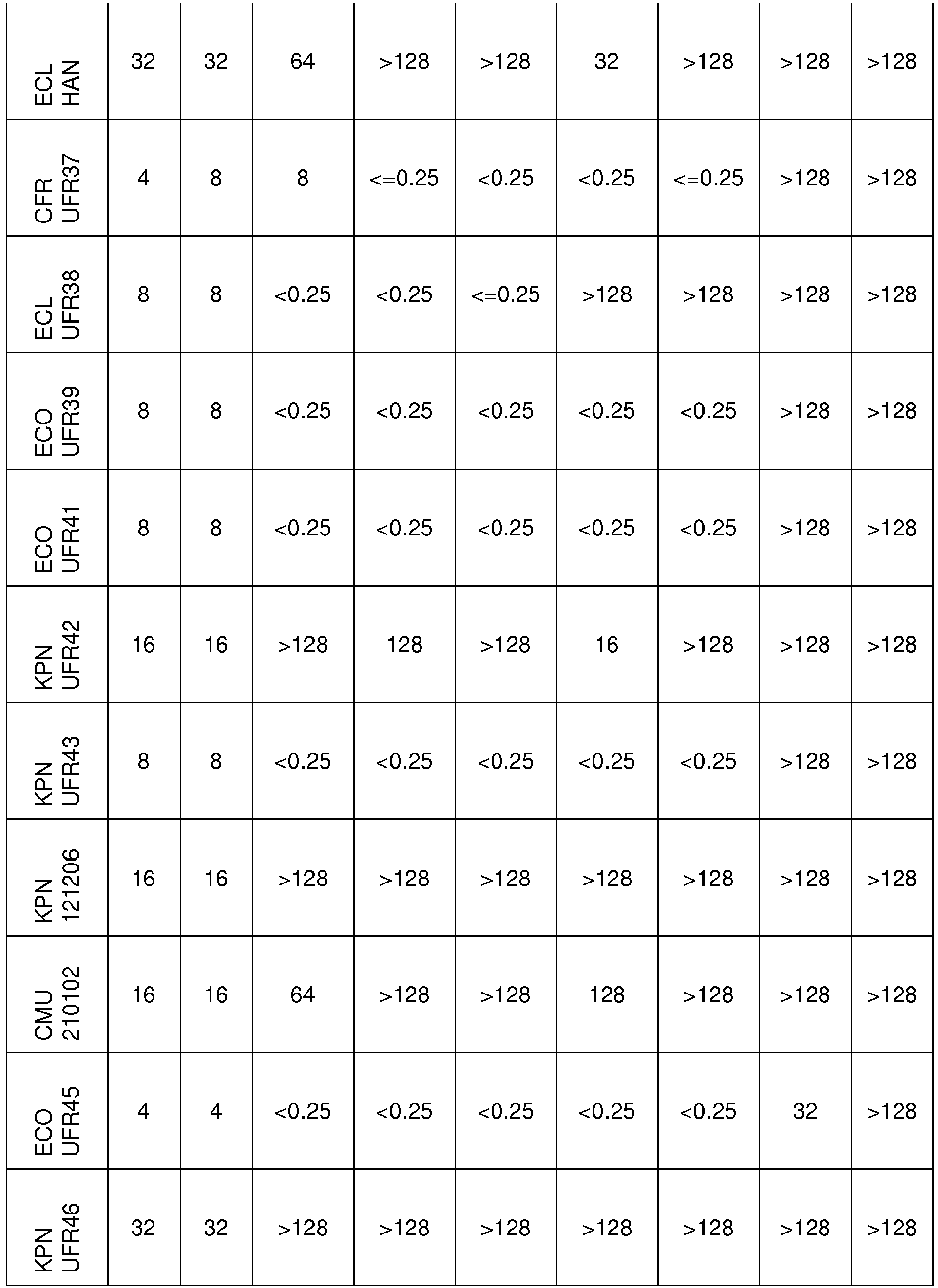

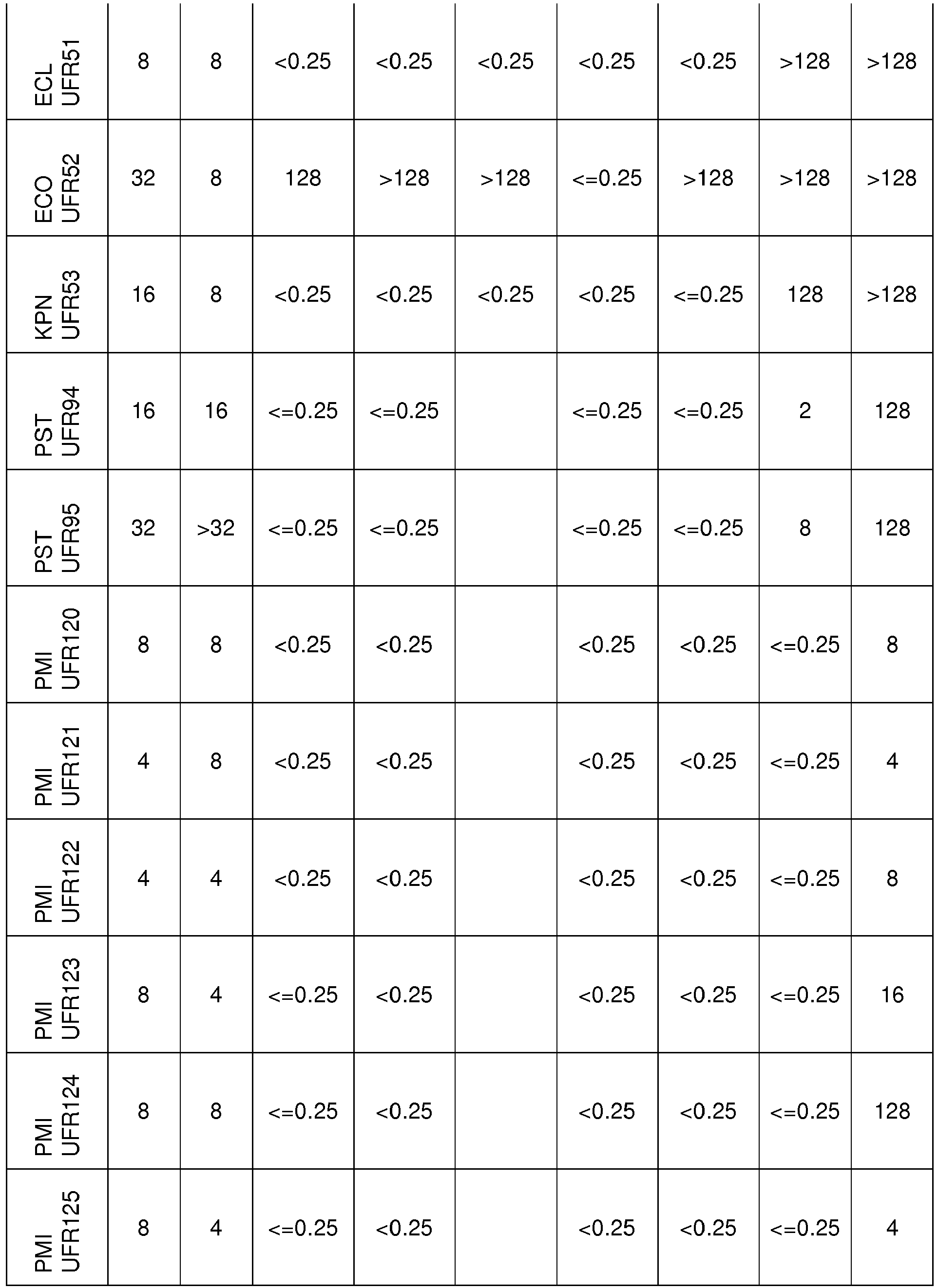

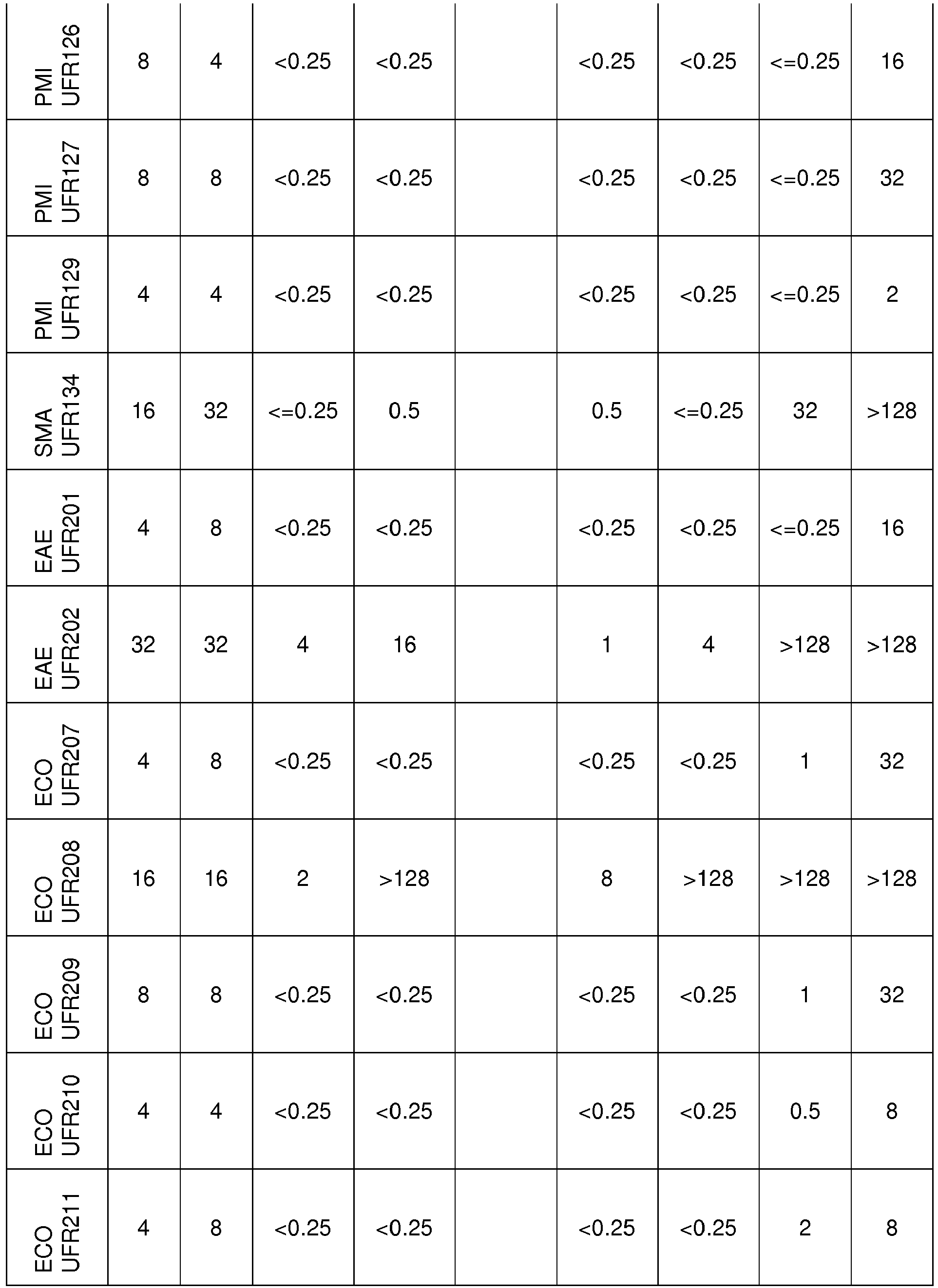

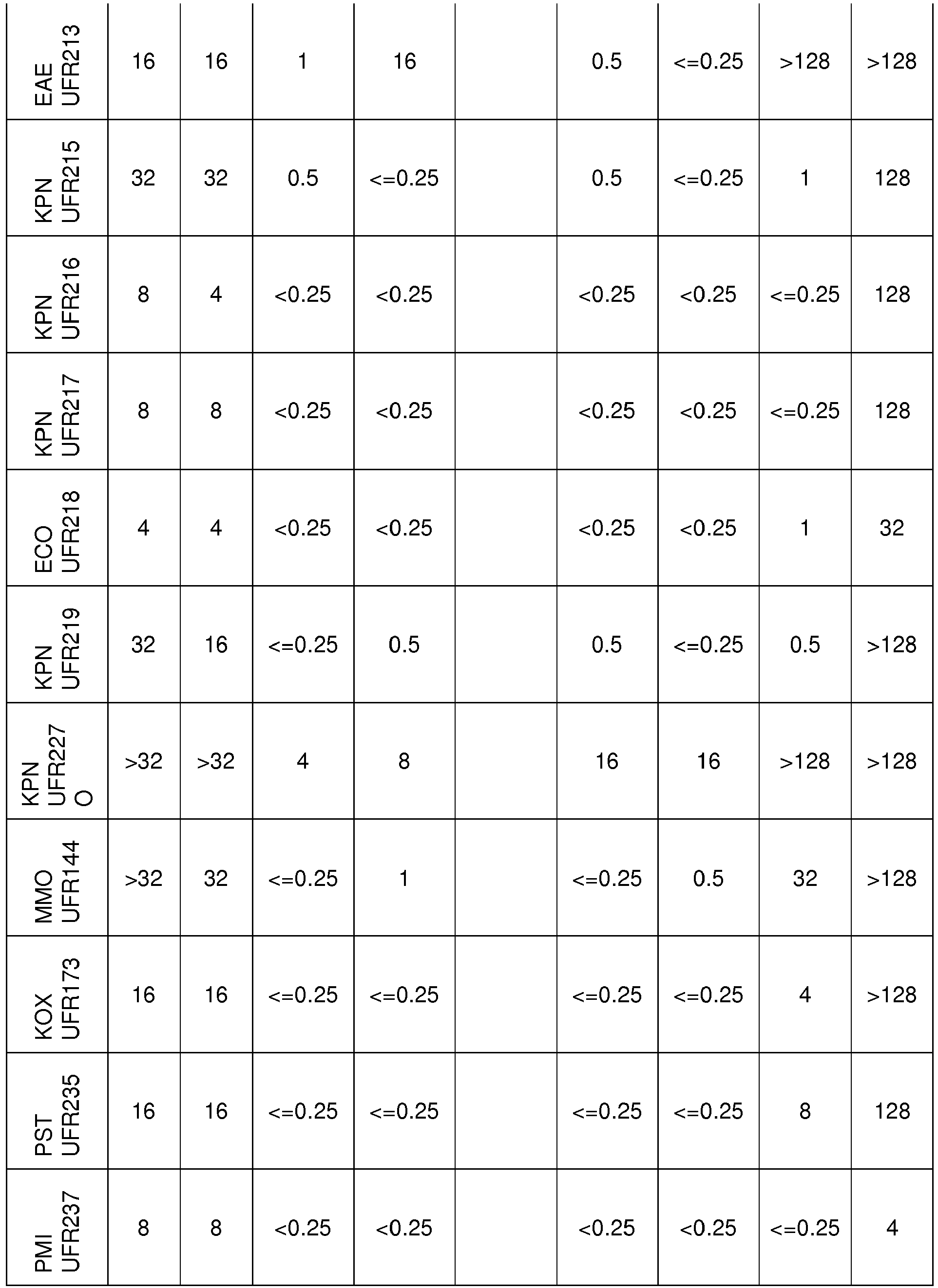

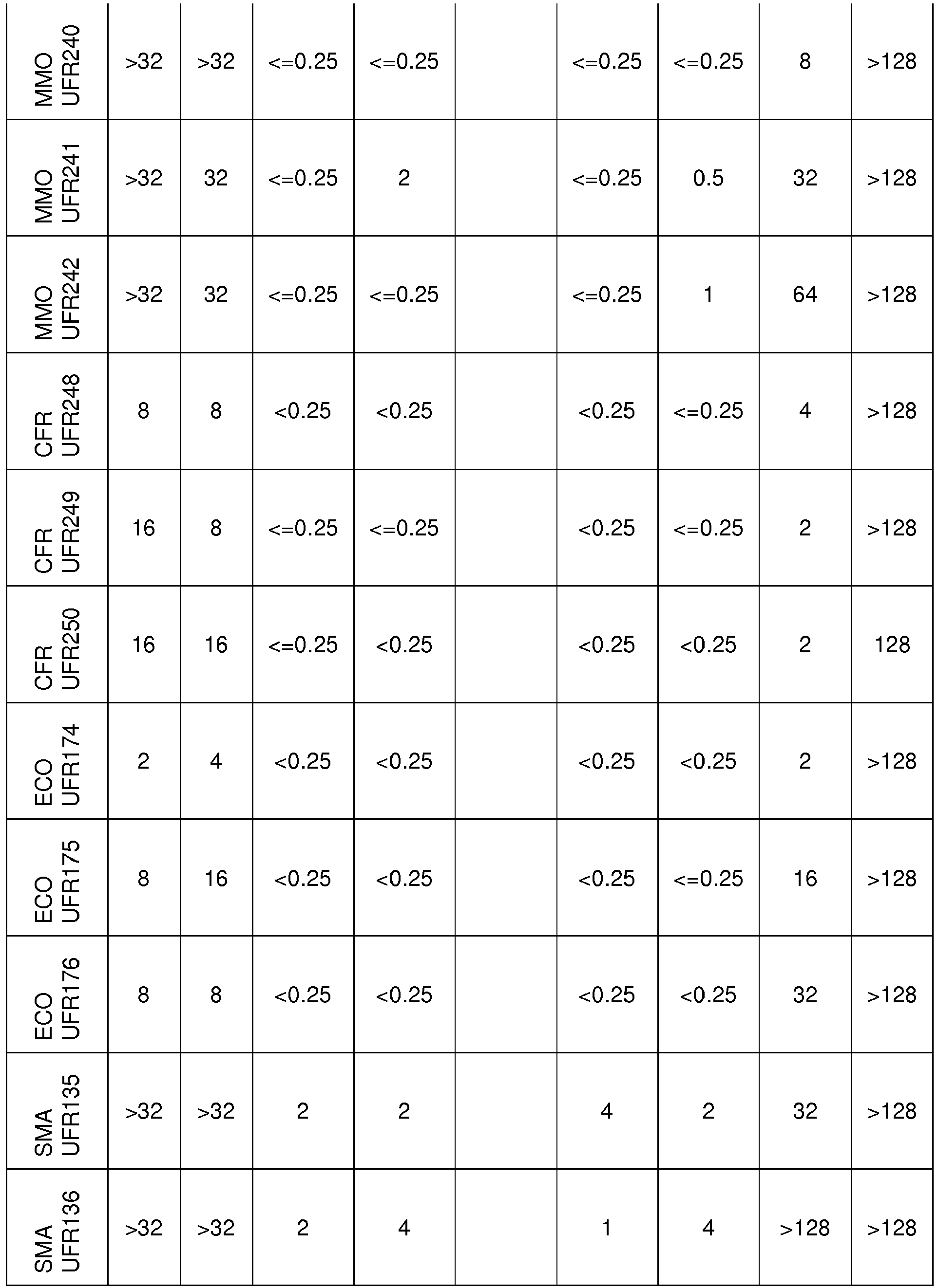

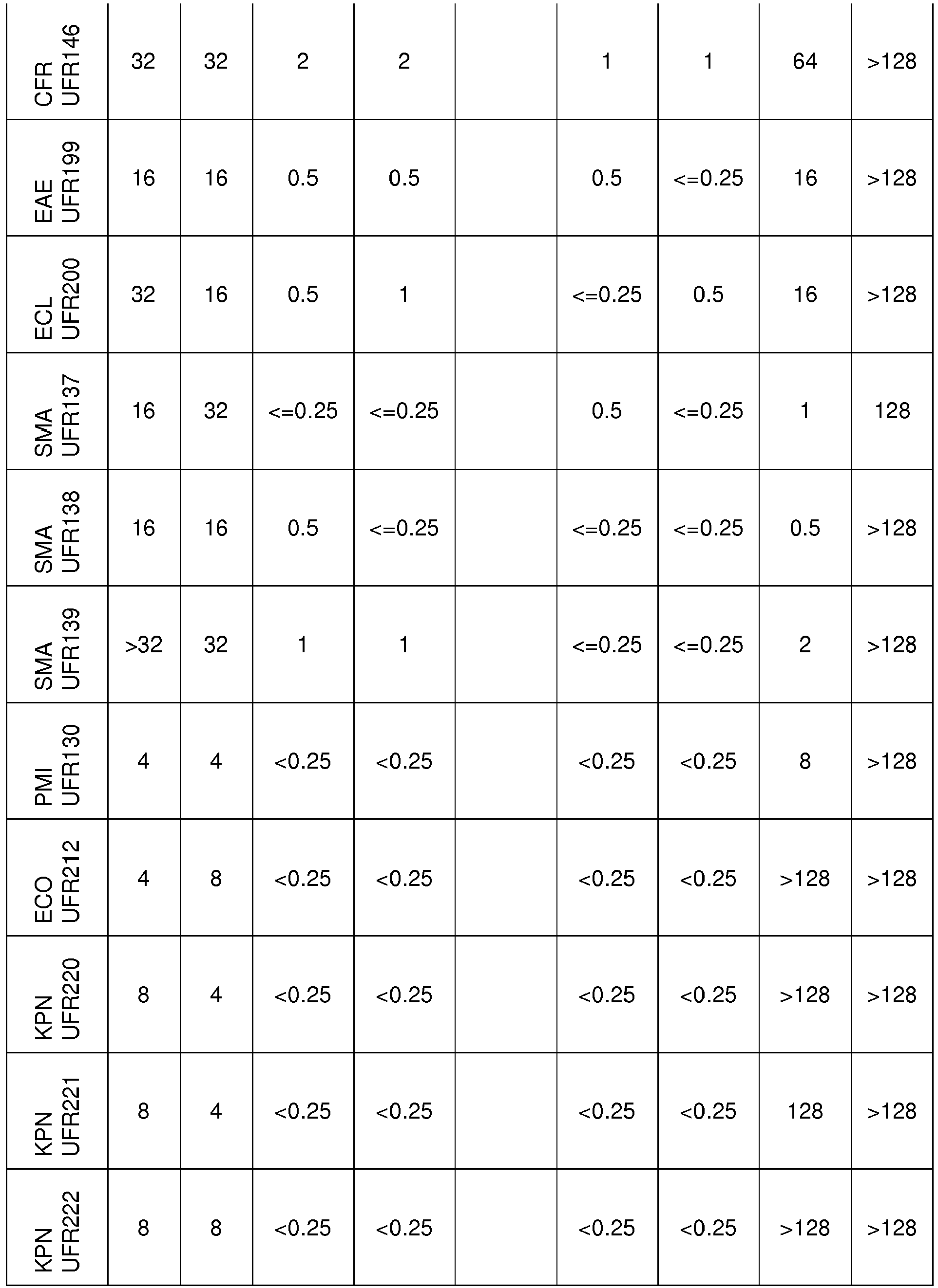

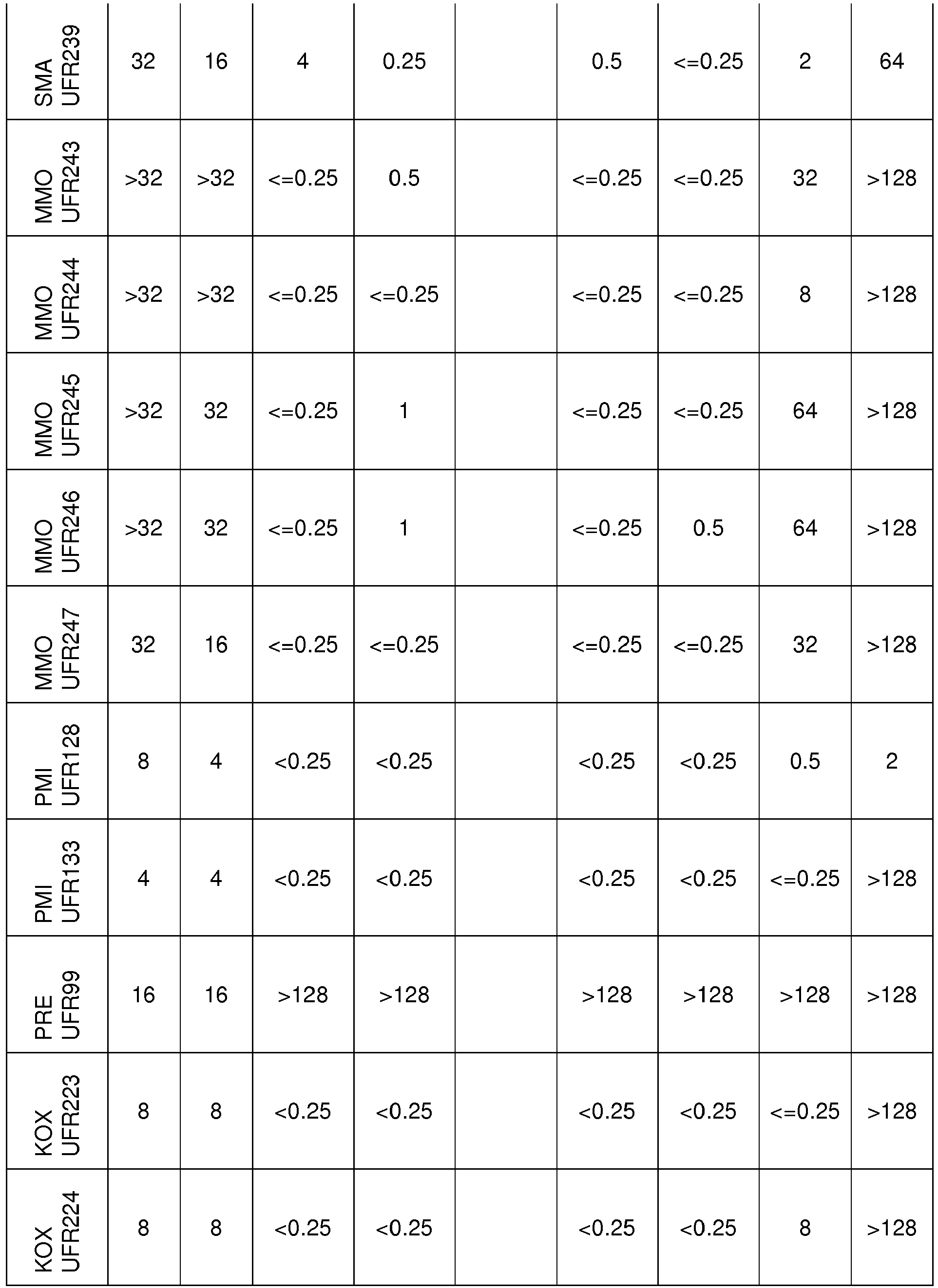

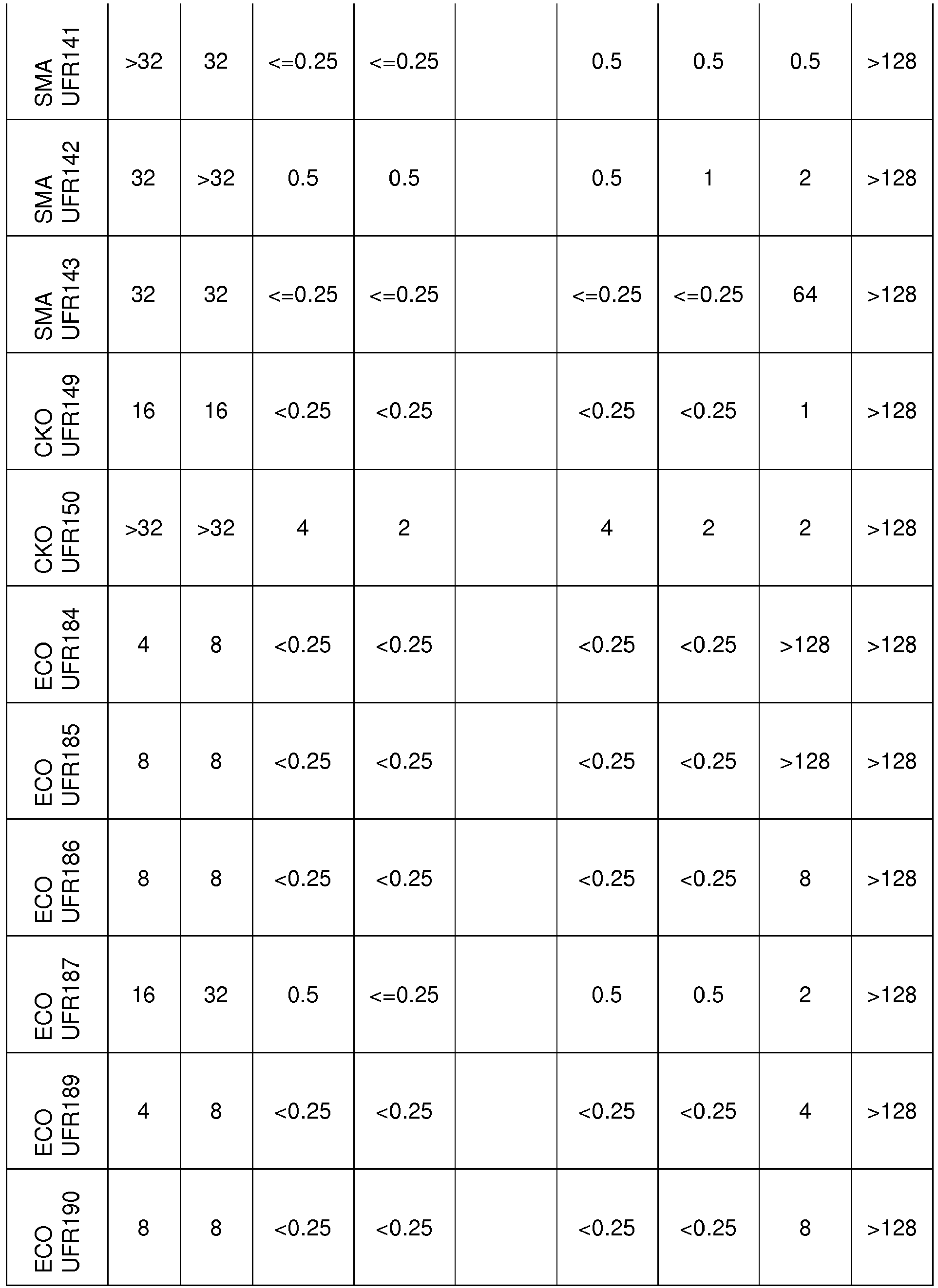

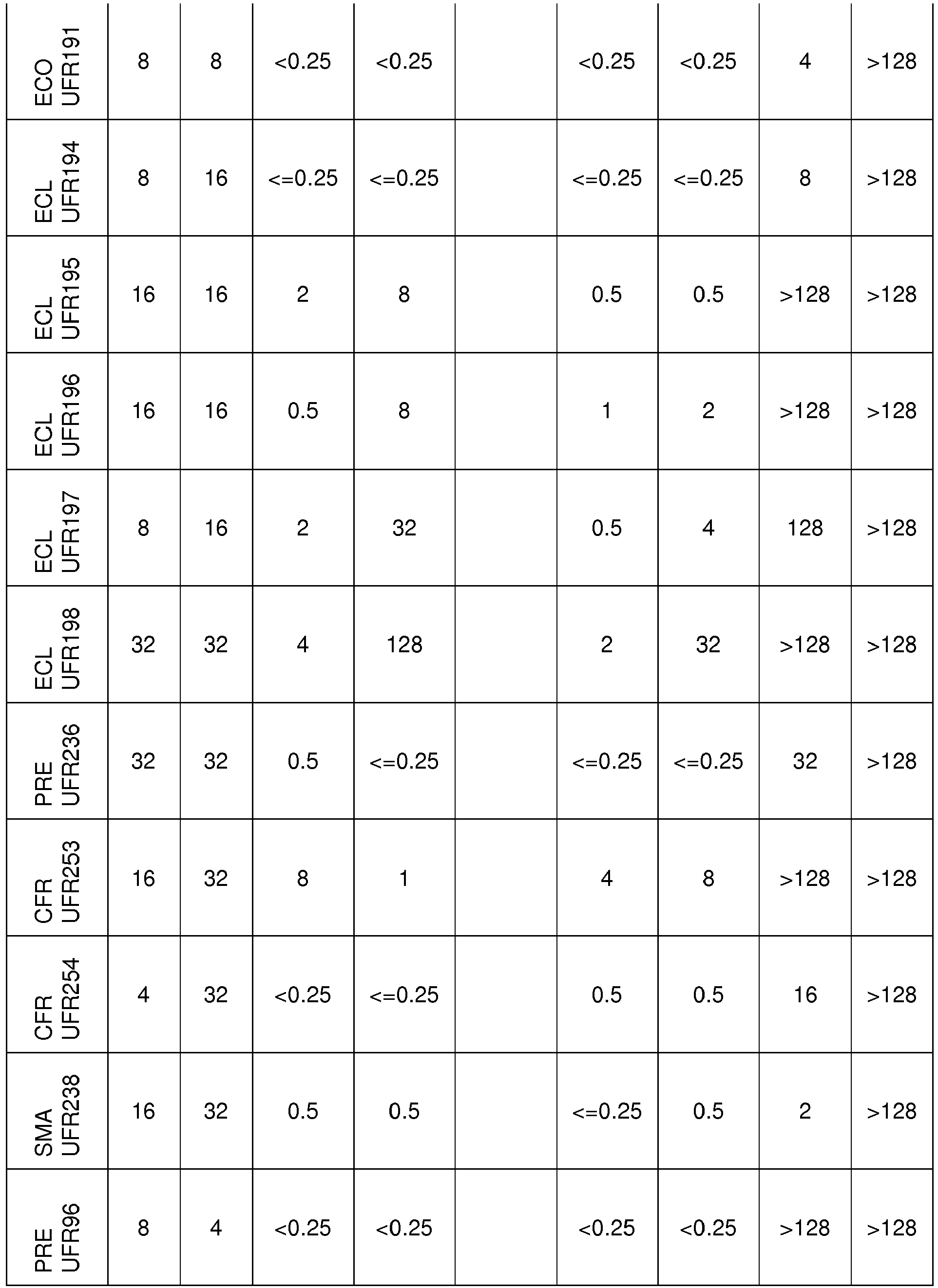

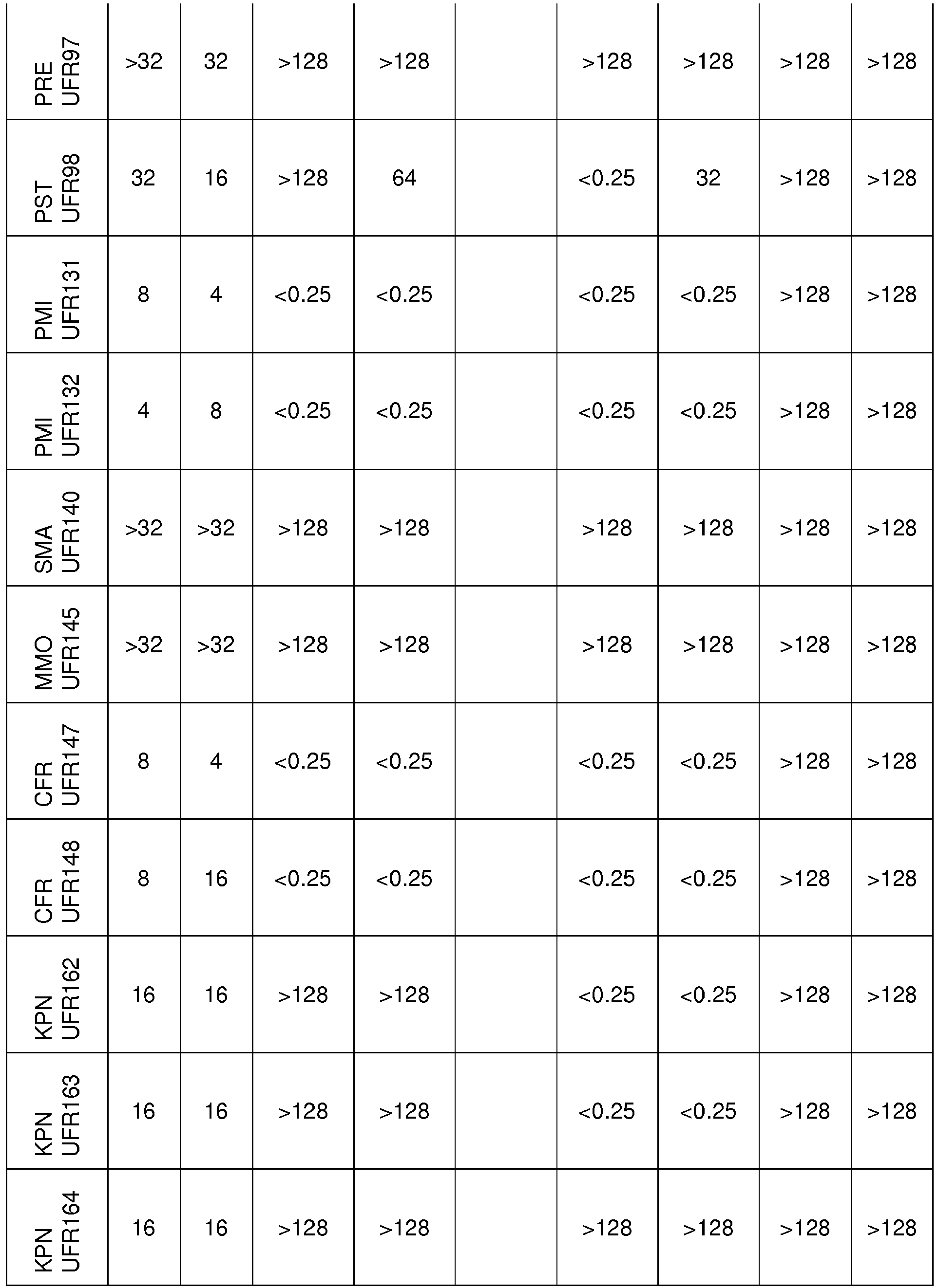

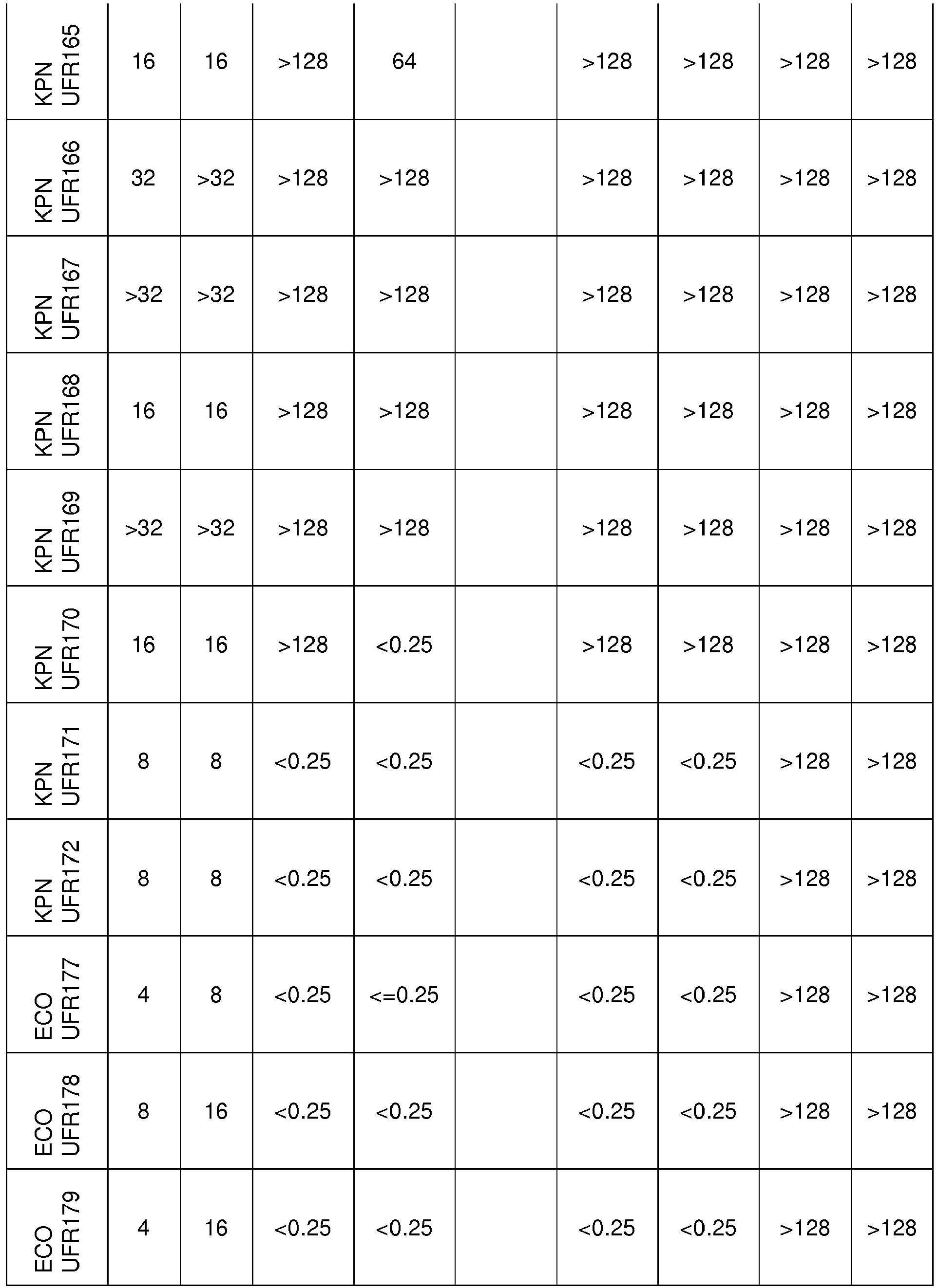

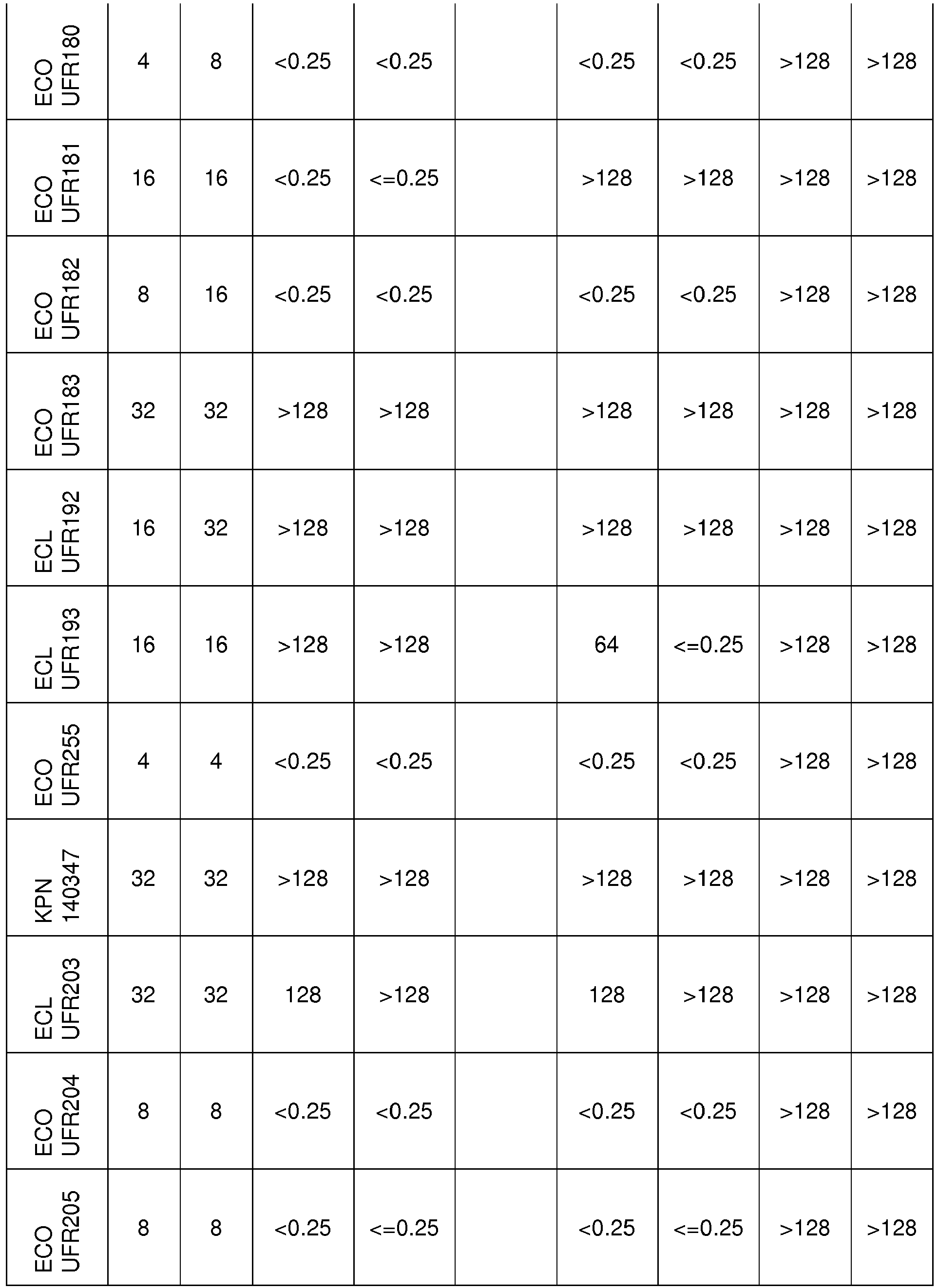

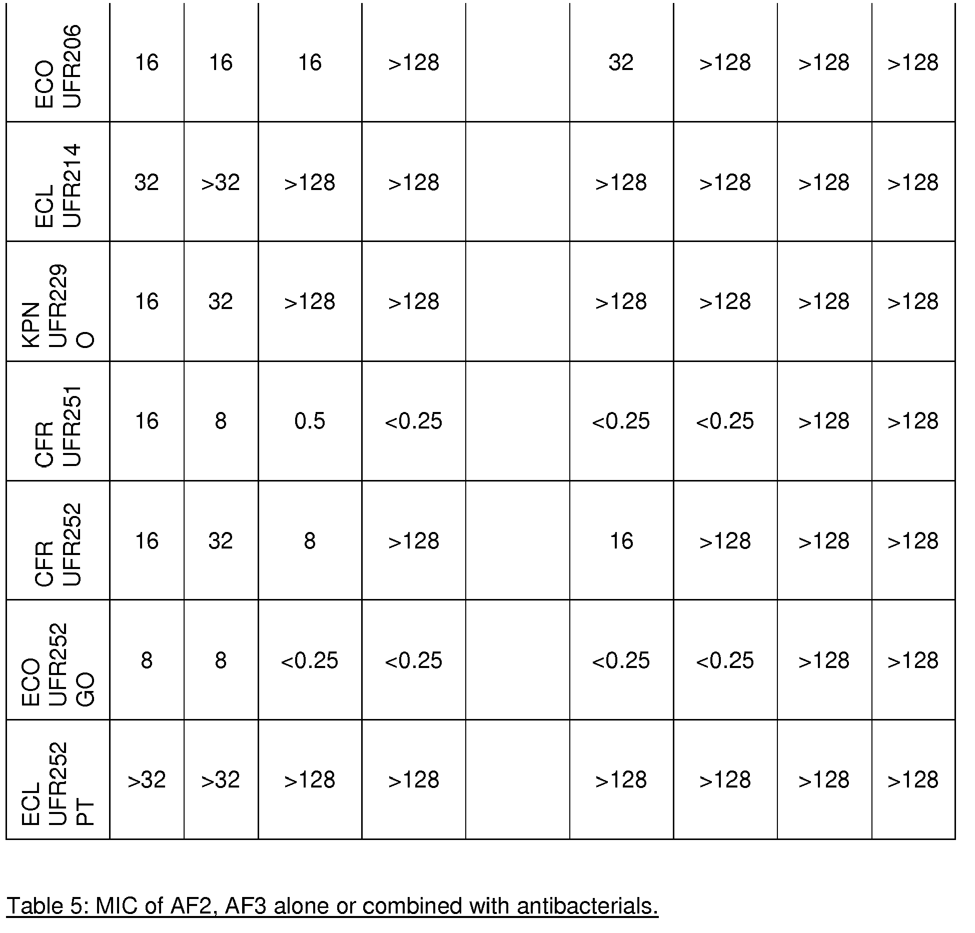

**Table 6: MIC of AF1 alone or combined with Cefixime.**

| | MIC (µg/mL) | | |
|---|---|---|---|
| | FIX | AF1 | FIX |
| | | | + AF1 @8 µg/mL |
| ECO 190317 | >128 | 2 | <0.25 |
| ECO 190457 | 128 | 4 | <0.25 |
| ECO UFR16 | >128 | 8 | <0.25 |
| ECO UFR20 | 512 | 16 | 1 |
| ECO UFR610 | 32 | 8 | <=0.25 |
| ECO UFR209 | 1024 | 8 | <=0.25 |
| EAE UFR199 | >1024 | 32 | 0.5 |
| PMI UFR126 | 1024 | 8 | <=0.25 |
| PMI UFR127 | >128 | 4 | <=0.25 |
| SMA UFR143 | 512 | 32 | <=0.25 |
| PST UFR235 | 512 | >32 | <=0.25 |
| CFR UFR250 | >128 | >32 | <=0.25 |
| KPN 110376 | >128 | >32 | <=0.25 |
| KPN 131119 | >128 | >32 | <=0.25 |
| KPN 190270 | >128 | >32 | 1 |
| KPN UFR25 | >128 | >32 | <=0.25 |
| KPN UFR66 | 512 | >32 | 2 |
| KPN UFR68 | >128 | >32 | <=0.25 |

### Method 3: Rat intraduodenal bioavailability determination (Table 7)

Intravenous (jugular) or intraduodenal catheterized Male Sprague-Dawley (SD) rats (250-270g) were obtained from Janvier Labs (Le Genest-Saint-Isle, France). All rats were housed in a -temperature (20 ± 2 °C) and -humidity (55% ±10%) controlled room with 12h light/dark cycle, and were acclimatized for at least 4 days before experimentation. Water and food were available ad libitum throughout the study. All rats were handled in accordance with the institutional and national guidelines for the care and use of laboratory animals.

Rats were allocated to two groups based on the administration route: intravenous or intraduodenal administration (n = 3/group).

In the intravenous administration study, drugs (10mg/kg in phosphate buffer 10mM, pH7.4) were administered under isoflurane anesthesia via the catheter placed in the jugular vein.

In the intraduodenal administration study, drugs (20mg/kg in phosphate buffer 10mM, pH5.0, 30-35% hydroxyl-propyl-beta-cyclodextrin, DMSO 0-10%) were administered under isoflurane anesthesia via the catheter placed in the duodenum.

For all groups, blood samples (100µL) were withdrawn from the tail vein at 5, 10, 20, 30, 45, 60, 120 and 240min after drug administration using Heparin-Lithium Microvette (Sarstedt, France) and immediately placed on ice. The collected blood was centrifuged at 2000xg and 4°C for 5 min to obtain plasma. Plasma samples were stored at -80°C until bioanalysis.

### Method 4: Plasma samples bioanalysis and data analysis

The plasma samples (20 µl) were thawed at 0°C. The samples were protein precipitated using 3-25 fold volume of acetonitrile, shaken and centrifuged for 20 min at 15 000 × g, diluted with a varying volume of deionized water, and pipetted to 96-well plates to wait for the LC-MS/MS analysis. Standard samples were prepared by spiking the blank plasma into concentrations 10 - 5 000 ng/ml and otherwise treated as the samples. Chromatographic separation was achieved with columns (T3 or C18 Cortex of Waters) and mobile phases according to the polarity of the drugs. Mass spectrometric detection involved electrospray ionization in the negative mode followed by multiple reaction monitoring of the drugs and internal standard transitions. Actual drug concentrations were deduced from interpolation of the standard curve. The pharmacokinetic parameters were calculated using XLfit (IDBS) and Excel (Microsoft) software, using standard non-compartmental methods. The intraduodenal bioavailability was calculated by dividing the AUC obtained from the intraduodenal administration by the AUC obtained from the intravenous administration.

**Table 7: Rat intraduodenal bioavailability of Examples 4, 6, 8, 10, 11, 13**

| Animal | Rat | | | | | | | |
|---|---|---|---|---|---|---|---|---|
| Compound administered | AF2 | | Example 4 | Example 6 | Example 8 | Example 10 | Example 11 | Example 13 |
| Route of administration | Intravenous | | Intraduodenal | | | | | |
| Dose (mg/kg) | 10 | | 20 | | | | | |
| Compound titrated in plasma | AF2 | | AF2 | | | | | |
| AUC 0-∞ (h*ng/mL) | 11022 | 10789 | 4620 | 435 | 10641 | 12431 | 6056 | 13152 |
| Bioavailability (%) | - | | 21 | 2 | 49 | 57 | 28 | 60 |

As shown in Table 7, the intraduodenal administration to rats of the prodrug Examples 4, 6, 8, 10, 11, 13 leads to the effective detection in plasma of their hydrolyzed form AF2, with intraduodenal bioavailabilities generally higher than 20% and culminating at 60% with Example 13. The best prodrug examples are therefore effectively absorbed in the gastrointestinal tract of the rats, and then effectively hydrolyzed into the active form AF2.

## Claims

1. Compound of formula (I) 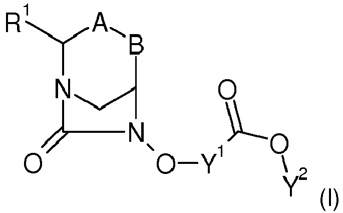 wherein
Y¹ represents CHF or CF₂;
Y² represents linear or branched (C3-C16)-alkyl, (C3-C11)-cycloalkyl, (C5-C11)-cycloalkenyl, (C4-C10)-heterocycloalkyl comprising from 1 to 2 heteroatoms chosen among N, O or S, (C5-C10)-heteroaryl comprising from 1 to 4 heteroatom chosen among N, O or S, (C6-C10)-aryl, (C7-C16)-aralkyl, (C7-C16)-heteroaralkyl comprising from 1 to 4 heteroatom chosen among N, O or S, a (C1-C6)alkyl-heterocycle wherein the heterocycle comprises from 4 to 5 carbon atoms and 1 to 2 heteroatoms chosen among N, O or S, preferably N and O, a polyethylene glycol group (PEG), a cetal group or an acetal group, wherein the alkyl, cycloalkyl, cycloalkenyl, heterocycloalkyl, heteroaryl, aryl, aralkyl, heterocycle and heteroaralkyl is optionally substituted ;
R¹ represents H, CN, CH₂OQ¹, C(=O)OQ¹, C(=O)NQ¹Q², C(=O)NQ¹OQ², C(=O)NQ¹NQ¹Q² or C(=O)ONQ¹Q²;
Q¹ and Q², identical or different represents H, linear or branched (C1-C6)-alkyl, (C3-C6)-cycloalkyl, (4-,5-,6-membered)-heterocycle aromatic, saturated or partially unsaturated with at least 1 N and optionally 1 or 2 other heteroatom chosen among N, O or S, linear or branched C(=O)(C1-C6)-alkyl, C(=O)(C1-C6)-cycloalkyl, C(=O)(4-,5-,6-membered)-heterocycle aromatic, saturated or partially unsaturated with at least 1 N and optionally 1 or 2 other heteroatom chosen among N, O or S or Q¹ and Q² form together a saturated or partially unsaturated (4-,5-,6-membered)-heterocycle comprising 1 to 4 heteroatoms chosen among N, O or S; the alkyl, cycloalkyl and heterocycle is optionally substituted;
A-B represents CH₂-C(=NOR²), C(R³)=C(R⁴);
R² represents H, linear or branched (C1-C6)-alkyl, (C1-C6)-alkyl-C(=O)NH₂, (C3-C6)-cycloalkyl; (4-,5-,6-membered)-heterocycle aromatic, saturated or partially unsaturated with at least 1 N, the alkyl, cycloalkyl and heterocycle is optionally substituted;
R³ and R⁴, different, represents H, (4 to 10-membered)-heterocycle, aromatic, saturated or partially or totally unsaturated, optionally substituted, or R³ and R⁴ form together with the carbon atoms to which they are linked a non-aromatic cycle of formula (II) 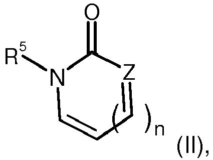
n represents 0 or 1 and Z represents S, N(R⁶) or C(R⁶) with the condition that if Z is S then n=0;
R⁵ different represents a linear or branched (C1-C6)-alkyl, a linear or branched (C1-C6)alkyl-OH, a linear or branched (C1-C6)-alkyl-NH₂, optionally substituted or a (C3-C6)-cycloalkyl optionally substituted;
R⁶ represents H, a linear or branched (C1-C6)-alkyl optionally substituted or a (C3-C6)-cycloalkyl optionally substituted;
• any carbon atom present within a group selected from alkyl ; cycloalkyl; cycloalkenyl; heterocycle can be oxidized to form a C(O) group;
• any sulphur atom present within an heterocycle can be oxidized to form a S(O) group or a S(O)₂ group ;
• any nitrogen atom present within a group wherein it is trisubstituted (thus forming a tertiary amine) or within an heterocycle can be further quaternized by a methyl group;
with the exception that one of R³ and R⁴ is H and at most one of R³ and R⁴ is H;
and a pharmaceutically acceptable salt, a zwitterion, an optical isomer, a racemate, a diastereoisomer, an enantiomer, a geometric isomer or a tautomer thereof.

2. Compound according to claim 1, wherein:
- the alkyl, cycloalkyl, cycloalkenyl, heterocycloalkyl, heteroaryl, aryl, aralkyl and heteroaralkyl representing Y² is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴, and
- Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S, the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
- the alkyl, cycloalkyl and heterocycle representing Q¹, Q² and R² is optionally substituted by one or more T¹ chosen among F, =O, CN, OT³, OC(=O)NT³T⁴, NT³C(=O)T⁴, NT³S(=O)₂T⁴, NT³S(=O)₂NT³T⁴, NT³C(=O)OT⁴, NT³C(=O)NT³T⁴, NT³T⁴, NT³C(=NT³)NT³T⁴, NT³CH(=NT⁴), C(=O)NT³T⁴, C(=O)NT³OT⁴, C(=O)NT³NT³T⁴, C(=NT³)NT³T⁴, linear or branched (C1-C6)-alkyl, (C3-C6)-cycloalkyl, S(=O)NT³T⁴, S(=O)₂NT³T⁴, (4-,5-,6-membered)-heterocycle aromatic, saturated or partially unsaturated with at least 1 N; the alkyl, cycloalkyl, and Heterocycle is optionally substituted by one or more T²; and
- the heterocycle representing R³ and/or R⁴ is optionally substituted by one or more T¹;
- the alkyl, cycloalkyl and heterocycle representing T¹ is optionally substituted by one or more T²;
- T², identical or different, is chosen among F, CN, NT³T⁴, NT³C(=NT³)NT³T⁴, NT³CH(=NT⁴), OT³, NT³C(=O)T⁴ and C(=O)NT³T⁴,
- T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂, and
- the alkyl or cycloalkyl representing R⁵ and R⁶ is optionally substituted by one or more T².

3. Compound according to claim 1 or 2 of formula (IA): 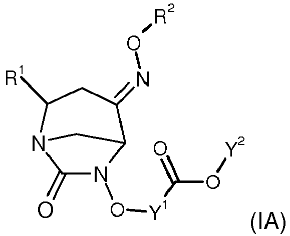 wherein
Y¹ represents CHF or CF₂;
Y² represents linear or branched (C3-C16)-alkyl, (C3-C11)-cycloalkyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, (C1-C6)alkyl-heterocycle wherein the heterocycle comprises from 4 to 5 carbon atoms and 1 to 2 heteroatoms chosen among N, O or S, preferably N and O, (C5-C11)-cycloalkenyl, or a group of formula 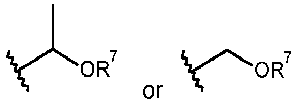
wherein R⁷ represents a linear or branched (C1-C6)-alkyl or C(=O)(C1-C6)-alkyl, a polyethylene glycol group (PEG), wherein the alkyl, cycloalkyl, cycloalkenyl, heterocycle, heterocycloalkyl, aralkyl and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CH₂OQ¹, C(=O)OQ¹, C(=O)NQ¹Q², C(=O)NQ¹OQ² or C(=O)NQ¹NQ¹Q²_{;}
Q¹ and Q², identical or different represents H, linear or branched (C1-C6)-alkyl, (5-,6-membered)-heterocycle aromatic, saturated or partially unsaturated with at least 1 N and optionally 1 or 2 other heteroatom chosen among N, O or S, C(=O)(4-,5-,6-membered)-heterocycle aromatic, saturated or partially unsaturated with at least 1 N and optionally 1 or 2 other heteroatom chosen among N, O or S; the alkyl and heterocycle is optionally substituted by one or more T¹;
R² represents H, linear or branched (C1-C6)-alkyl, (C1-C6)-alkyl-C(=O)NH₂, the alkyl is optionally substituted by one or more T¹;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S;
T¹, identical or different, represents OT³, NT³T⁴, C(=O)NT³T⁴, linear or branched (C1-C6)-alkyl, (5-,6-membered)-heterocycle aromatic, saturated or partially unsaturated with at least 1 N; the alkyl and Heterocycle is optionally substituted by one or more T²; and
T² identical or different, is chosen among CN, NT³T⁴, OT³ and C(=O)NT³T⁴,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

4. Compound according to claim 1 or 2 of formula (IB): 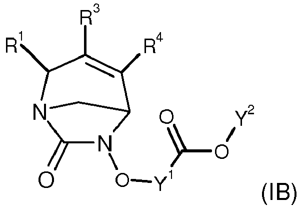 wherein
Y¹ represents CHF or CF₂;
Y² represents linear or branched (C3-C16)-alkyl, (C3-C11)-cycloalkyl, (C4-C10)-heterocycloalkyl comprising from 1 to 2 heteroatoms chosen among N, O or S, (C7-C16)-aralkyl, (C7-C16)-heteroaralkyl comprising from 1 to 4 heteroatom chosen among N, O or S, a polyethylene glycol group (PEG), (C1-C6)alkyl-heterocycle wherein the heterocycle comprises from 4 to 5 carbon atoms and 1 to 2 heteroatoms chosen among N, O or S,
preferably N and O, (C5-C11)-cycloalkenyl, or a group of formula 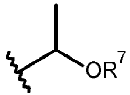 or 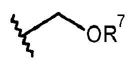
wherein R⁷ represents a linear or branched (C1-C6)-alkyl or C(=O)(C1-C6)-alkyl, wherein the alkyl, cycloalkenyl, heterocycle, cycloalkyl, heterocycloalkyl, aralkyl and heteroaralkyl is optionally substituted by one or more group chosen among : halogen, =O, Y³, OY³, OC(=O)Y³, SY³, NY³Y⁴, NY³C(=O)Y⁴, NY³S(=O)₂Y⁴, C(=O)Y³, C(=O)OY³, C(=O)NY³Y⁴, S(=O)Y³, S(=O)₂Y³ or S(=O)₂NY³Y⁴;
R¹ represents H, CN, CH₂OQ¹, C(=O)OQ¹, C(=O)NQ¹Q², C(=O)NQ¹OQ² or C(=O)NQ¹NQ¹Q²;
Q¹ and Q², identical or different represents H, linear or branched (C1-C6)-alkyl, (5-,6-membered)-heterocycle aromatic, saturated or partially unsaturated with at least 1 N and optionally 1 or 2 other heteroatom chosen among N, O or S, C(=O)(4-,5-,6-membered)-heterocycle aromatic, saturated or partially unsaturated with at least 1 N and optionally 1 or 2 other heteroatom chosen among N, O or S; the alkyl and heterocycle is optionally substituted by one or more T¹;
R³ and R⁴, different, represents H, (5-,6-membered)-heterocycle aromatic optionally substituted by one or more T¹, or R³ and R⁴ form together with the carbon atoms to which the following cycle: 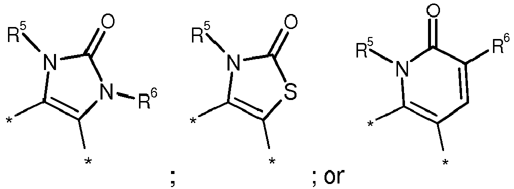
R⁵ different represents a linear or branched (C1-C6)-alkyl optionally substituted by one or more T², a linear or branched (C1-C6)-alkyl-OH, a linear or branched (C1-C6)-alkyl-NH₂,or a (C3-C6)-cycloalkyl optionally substituted by one or more T²;
R⁶ represents H, a linear or branched (C1-C6)-alkyl optionally substituted by one or more T² or a (C3-C6)-cycloalkyl optionally substituted by one or more T²;
Y³ and Y⁴, identical or different, represent H, linear or branched (C1-C6)-alkyl, (C3-C11)-cycloalkyl, (C6-C10)-aryl, (C4-C10)-heterocycloalkyl comprising from 1 to 2 heteroatoms chosen among N, O or S, (C5-C10)-heteroaryl comprising from 1 to 4 heteroatom chosen among N, O or S, or form together with the nitrogen atom to which they are linked a (C4-C10)-heterocycloalkyl comprising from 1 to 2 heteroatoms chosen among N, O or S; the alkyl, cycloalkyl, aryl, heterocycloalkyl and heteroaryl is optionally substituted by one or more linear or branched (C1-C10)-alkyl, OH, O(C1-C6)-alkyl, NH₂, NH(C1-C6)-alkyl, N[(C1-C6)-alkyl]₂, C(=O)NH₂, C(=O)NH(C1-C6)-alkyl or C(=O)N[(C1-C6)-alkyl]₂;
T¹, identical or different, represents F, OT³, NT³C(=O)T⁴, NT³T⁴, CN, C(=O)NT³T⁴, C(=O)NT³OT⁴, C(=O)NT³NT³T⁴, linear or branched (C1-C6)-alkyl, (5-,6-membered)-heterocycle aromatic, saturated or partially unsaturated with at least 1 N; the alkyl, and Heterocycle is optionally substituted by one or more T²; and
T² identical or different, is chosen among CN, NT³T⁴, OT³ and C(=O)NT³T⁴,
T³ and T⁴, identical or different, represent H, linear or branched (C1-C6)-alkyl, (C3-C10)-cycloalkyl, the alkyl and cycloalkyl is optionally substituted by one or more OH, NH₂ or CONH₂.

5. Compound according to anyone of claims 1 to 4 of formula (I*), (IA*), or (IB*) 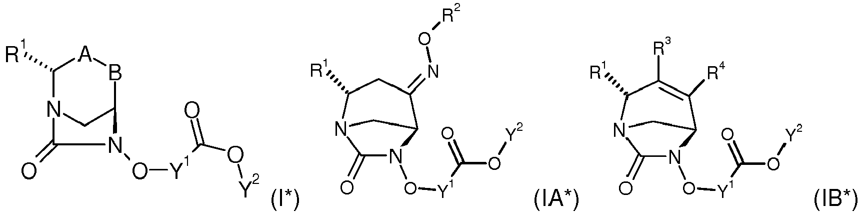 wherein R¹, R², R³, R⁴, Y¹ and Y² are as defined in claims 1 to 4.

6. Compound according to anyone of claims 1 to 5 for use as a pro-drug of a compound of formula (I') 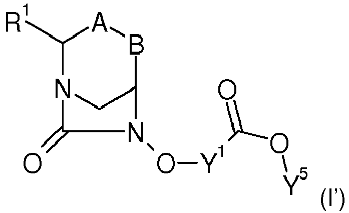 wherein R¹, Y¹, A and B are as defined in anyone of claim 1 to 5 and Y⁵ represents H or a base addition salts for example chosen among ammonium salts such as tromethamine, meglumine, epolamine; metal salts such as sodium, lithium, potassium, calcium, zinc, aluminium or magnesium; salts with organic bases such as methylamine, propylamine, trimethylamine, diethylamine, triethylamine, N,N-dimethylethanolamine, tris(hydroymethyl)aminomethane, ethanolamine, pyridine, picoline, dicyclohexylamine, morpholine, benzylamine, procaine, N-methyl-D-glucamine; salts with amino acids such as arginine, lysine, ornithine and so forth; phosphonium salts such as alkylphosphonium, arylphosphonium, alkylarylphosphonium and alkenylarylphosphonium; and salts with quaternary ammonium such as tetra-n-butylammonium.

7. A pharmaceutical composition comprising at least a compound of formula (I) according to anyone of claims 1 to 5, and optionally a pharmaceutically acceptable excipient.

8. A pharmaceutical composition according to claim 7 further comprising at least one compound selected from an antibacterial compound, preferably a β-lactam compound.

9. A pharmaceutical composition according to one of claims 7 and 8 comprising
• a single compound according to one of claims 1 to 5 ; or
• a compound according to one of claims 1 to 5 and one or more antibacterial compound ; or
• a compound according to one of claims 1 to 5 and one or more β-lactam compound ; or
• a compound according to one of claims 1 to 5, one or more antibacterial compound and one or more β-lactam compound.

10. A pharmaceutical composition according to one claims 8 and 9 wherein
• the antibacterial compound is selected from aminoglycosides, β-lactams, glycylcyclines, tetracyclines, quinolones, fluoroquinolones, glycopeptides, lipopeptides, macrolides, ketolides, lincosamides, streptogramins, oxazolidinones, polymyxins and mixtures thereof; or
• the β-lactam compound is selected from β-lactams, and mixtures thereof, preferably penicillin, cephalosporins, penems, carbapenems and monobactam.

11. Composition according to anyone of claims 7 to 10, wherein
• the antibacterial compound is selected from orally bioavailable aminoglycosides, β-lactams, glycylcyclines, tetracyclines, quinolones, fluoroquinolones, glycopeptides, lipopeptides, macrolides, ketolides, lincosamides, streptogramins, oxazolidinones, polymyxins and mixtures thereof; or
• the β-lactam compound is selected from orally available β-lactams, or prodrugs of β-lactams, and mixtures thereof, preferably penicillin, cephalosporins, penems, carbapenems and monobactams.

12. Composition according to anyone of claims 7 to 11, wherein the β-lactam is chosen among amoxicillin, amoxicillin-clavulanate, sultamicillin, cefuroxime, cefazolin, cefaclor, cefdinir, cefpodoxime, cefprozil, cephalexin, loracarbef, cefetamet, ceftibuten, tebipenem pivoxil, cefixime, preferably cefixime.

13. A kit comprising a pharmaceutical composition according to one of claims 7 to 12 and at least one second composition according to one of claims 7 to 12.

14. A compound or a composition according to one of claims 1 to 5 and 7 to 12 for its use as a medicine or for its use for treating or preventing a bacterial infection.

15. A compound or a composition according to claim 14 for its use for treating or preventing a bacterial infection caused by bacteria producing one or more β-lactamase.

16. A compound or a composition according to one of claims 14 and 15 for its use for treating or preventing a bacterial infection caused by a gram-positive bacteria or by gram-negative bacteria, preferably a bacterial infection caused by gram-negative bacteria.

17. A kit according to claim 13 for the treatment or prevention of bacterial infections by its simultaneous, separate or sequential administration to a patient in need thereof.
